# EUROPEAN PATENT SPECIFICATION

(11) **EP 2 464 646 B1**
(45) Date of publication and mention of the grant of the patent: **07.05.2014**
(21) Application number: 10744808.6
(22) Date of filing: 11.08.2010
(51) Int. Cl.: C07D 471/04

(54) **PROCESS FOR THE PREPARATION OF CATHEPSIN S INHIBITORS**
VERFAHREN ZUR HERSTELLUNG VON CATHEPSIN-S-HEMMERN
PROCÉDÉ POUR LA PRÉPARATION D'INHIBITEURS DE LA CATHEPSINE S

(30) Priority: 12.08.2009 US 233231 P
(43) Date of publication of application: 20.06.2012
(73) Proprietor: Janssen Pharmaceutica, N.V., 2340 Beerse (BE)
(72) Inventor: LIANG, Jimmy, T., San Diego CA 92128 (US); MANI, Neelakandha, S., San Diego CA 92129 (US)
(74) Representative: Warner, James Alexander
(86) International application number: PCT/US2010/045204
(87) International publication number: WO 2011/019842

(56) References cited:
- US-A1- 2003 078 419
- WEI ET AL: "Pyrazole-based cathepsin S inhibitors with improved cellular potency" 14 September 2007 (2007-09-14), BIOORGANIC & MEDICINAL CHEMISTRY LETTERS, PERGAMON, ELSEVIER SCIENCE, GB LNKD- DOI:10.1016/J.BMCL.2007.08.038, PAGE(S) 5525 - 5528 , XP022249659 ISSN: 0960-894X page 5526, scheme 1

## Description

### CROSS REFERENCE TO RELATED APPLICATION

This application claims the benefit of US provisional patent application serial number 61/233,231, filed August 12, 2009.

### FIELD OF THE INVENTION

The present invention is directed to processes for the preparation of compounds, such as inhibitors of Cathepsin S enzyme, and to some of such compounds. Some of these compounds have been proposed as candidates for the treatment of for example, autoimmune diseases such as lupus, rheumatoid arthritis and asthma and further candidates for the prevention, inhibition and / or treatment of tissue transplant rejection.

### BACKGROUND OF THE INVENTION

Cathepsin S (EC 3.4.22.27) is a cysteine protease of the papain family found primarily in lysosomes (Bromme, D.; McGrath, M. E. High Level Expression and Crystallization of Recombinant Human Cathepsin S. Protein Science 1996, 5, 789-791).

The role of cathepsin S in the immune response is anticipated by its tissue distribution: cathepsin S is found primarily in lymphatic tissues, lymph nodes, the spleen, B lymphocytes, and macrophages (Kirschke, H. Chapter 211. Cathepsin S. In Handbook of Proteolytic Enzymes. Barrett, A. J.; Rawlings, N. D.; Woessner, J. F., Eds. San Diego: Academic Press, 1998. pp. 621-624.). Cathepsin S inhibitors have been shown in animal models to modulate antigen presentation and are effective in an animal model of asthma (Riese, R. J.; Mitchell, R. N.; Villadangos, J. A.; Shi, G.-P.; Palmer, J. T.; Karp, E. R.; De Sanctis, G. T.; Ploegh, H. L.; Chapman, H. A. Cathepsin S Activity Regulates Antigen Presentation and Immunity. J. Clin. Invest. 1998, 101, 2351-2363 and Shi, G.-P.; Villadangos, J. A.; Dranoff, G.; Small, C.; Gu, L.; Haley, K. J.; Riese, R.; Ploegh, H. L.; Chapman, H. A. Cathepsin S Required for Normal MHC Class II Peptide Loading and Germinal Center Development. Immunity 1999, 10, 197-206.).

Mice in which the gene encoding cathepsin S has been knocked out are less susceptible to collagen-induced arthritis and their immune systems have an impaired ability to respond to antigens (Nakagawa, T. Y.; Brissette, W. H.; Lira, P. D.; Griffiths, R. J.; Petrushova, N.; Stock, J.; McNeish, J. D.; Eastman, S. E.; Howard, E. D.; Clarke, S. R. M.; Rosloniec, E. F.; Elliott, E. A.; Rudensky, A. Y. Impaired Invariant Chain Degradation and Antigen Presentation and Diminished Collagen-Induced Arthritis in Cathepsin S Null Mice. Immunity 1999, 10, 207-217).

These data support that compounds that inhibit the proteolytic activity of human cathepsin S be proposed as candidates in the treatment of chronic autoimmune diseases including, but not limited to, lupus, rheumatoid arthritis, and asthma; and in modulating the immune response to tissue transplantation.

Wei et al., Bioorg. Med. Chem. Lett. 17 (2007) 5525-5528 discloses the preparation of pyrazole-based cathepsin S inhibitors via pyrazoles containing an epoxy group.

Butler et al., in U.S. Patent 6,953,793, granted October 11, 2005 disclose substituted pyrazoles, pharmaceutical compositions comprising said substituted pyrazoles and methods of treating disorders or conditions mediated by the cathepsin S enzyme, such as autoimmune diseases such as lupus, rheumatoid arthritis, and asthma, and for the prevention, inhibition, or treatment of tissue transplant rejection. U.S. Patent 6,953,793 further discloses a process for the preparation of said substituted pyrazoles. However, the process disclosed is not suitable for large scale manufacture. Therefore, there remains a need for a process for the preparation of substituted pyrazoles.

### SUMMARY OF THE INVENTION

The present invention is directed to a process for the preparation of compounds of formula (I) 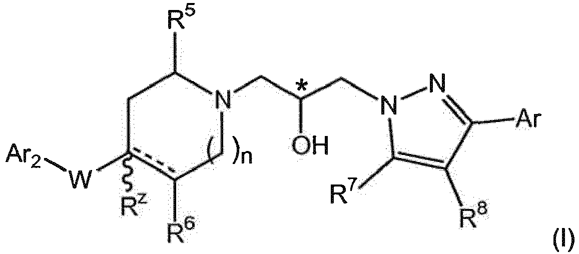 wherein
Ar₂ is a monocyclic or bicyclic ring system, unsaturated, saturated or aromatic, optionally fused, optionally including between 1 and 5 heteroatom ring moieties independently selected from the group consisting of O, S, N, SO₂ and C=O; wherein said Ar₂ ring system is optionally substituted with between 1 and 4 substituents;
W represents O, S, NR²⁷, C=O, (C=O)NH, NH(C=O),CHR²⁸, or a covalent bond;
R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂ or R³⁰R³¹ NSO₂; or alternatively, R²⁷ and part of Ar₂ can be taken together to form an optionally substituted 5- to 6-membered heterocyclic ring with optionally 1 to 3 additional heteroatom moieties in the ring selected from O, NR⁹, NR¹⁰, N, SO₂, C=O and S; which ring may be saturated, unsaturated or aromatic; wherein R⁹ and R¹⁰ are independently selected from the group cosisting of H, C₁₋₃alkyl, and -CH₂CO₂(C₁₋₄alkyl);
R²⁸ is hydrogen, C₁₋₅alkyl, C₃₋₅alkenyl, hydroxy, phenyl, benzyl, C₁₅heterocyclyl, R²⁹O, R³⁰R³¹NC=O, R²⁹S, R²⁹SO, R²⁹SO₂ or R³⁰R³¹NSO₂;
R²⁹ is C₁₋₅alkyl, C₃₋₅akenyl, phenyl, benzyl or C₁₋₅heterocyclyl;
R³⁰ and R³¹ are each independently selected from the group consisting of hydrogen, C₁₋₅alkyl, C₃₋₅alkenyl, phenyl, benzyl, phenethyl, naphthyl, and C₁₅heteroaryl; alternatively R³⁰ and R³¹ can be taken together to form an optionally substituted 4- to 7-membered ring carbocyclic or heterocyclic ring, which ring may be saturated, unsaturated or aromatic;
R^{z} is H or OH and the dashed line is absent; or R^{z} is absent where the dashed line is an sp² bond;
R⁵ and R⁶ are each independently selected from the group consisting of hydrogen and C₁₋₅alkyl;
n is an integer selected from 0, 1 or 2;
R⁷ and R⁸ are each independently hydrogen, C₁₋₅alkyl, C₂₋₅alkenyl, C₁₅alkoxy, C₁₋₅alkylthio, halogen, or a 4-7 membered carbocyclyl or heterocyclyl;
alternatively, R⁷ and R⁸ can be taken together to form an optionally substituted 5- to 7-membered carbocyclyc or heterocyclic ring, which ring may be unsaturated or aromatic, and may be optionally substituted with between one and three substituents independently selected from the group consisting of halo, cyano, amino, hydroxy, nitro, R⁴, R⁴O-, R⁴S-, R⁴O(C₁₋₅alkylene)-, R⁴O(C=O₎-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, an NHR⁴⁴(C=O)-;
R⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
R⁴⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁-₅alkylene, phenylene, or divalent C₁₋₅heterocyclyl;
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O, C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl, C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C1-5heterocyclyl; alternatively, R²² and R²³ can be taken together to form an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; comprising 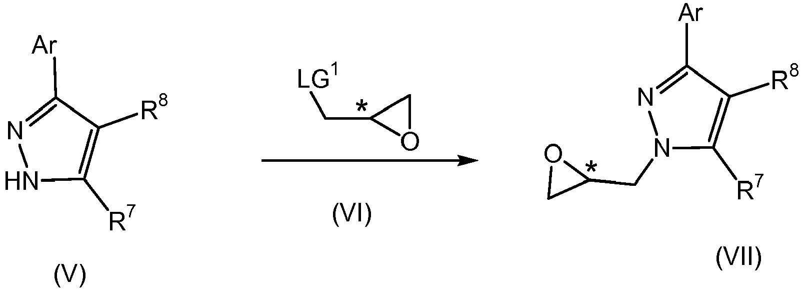 reacting a compound of formula (V) with a compound of formula (VI), wherein LG¹ is a leaving group; in the presence of an organic or inorganic base; in an organic solvent; to yield the corresponding compound of formula (VII); 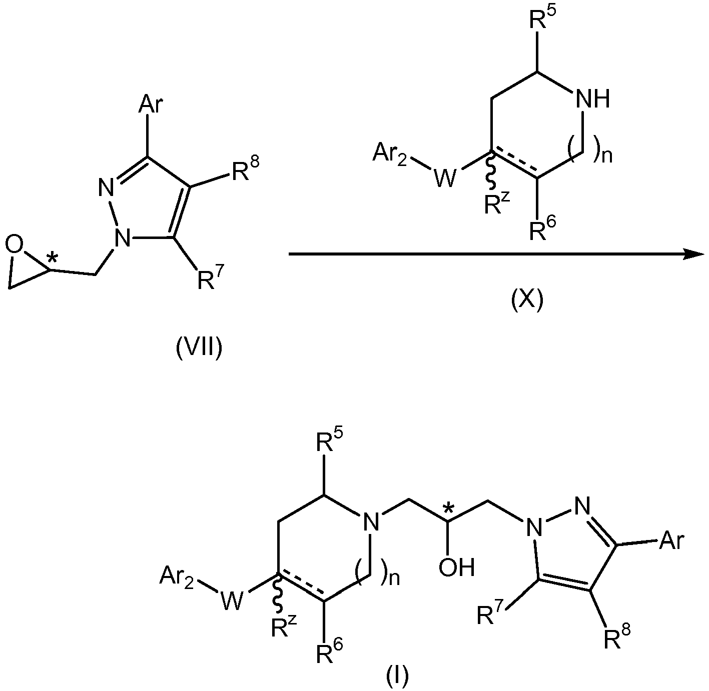 reacting the compound of formula (VII) with a compound of formula (X);
in an organic solvent; to yield the corresponding compound of formula (I).

In an embodiment, the present invention is directed to a process for the preparation of a compound of formula (I-S) 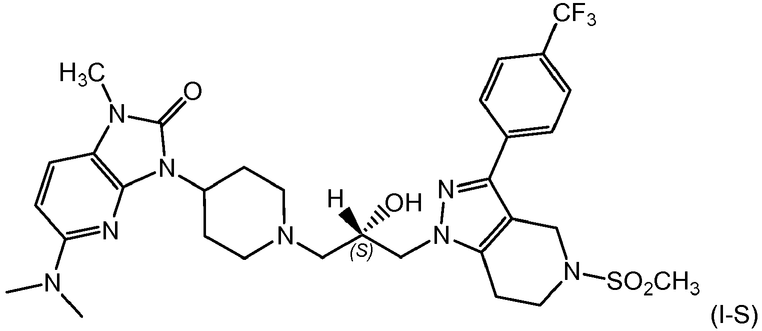 (also known as 5-dimethylamino-3-(1-{2S-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one) or a pharmaceutically acceptable salt, amide or ester thereof; or a stereoisomeric form thereof; comprising 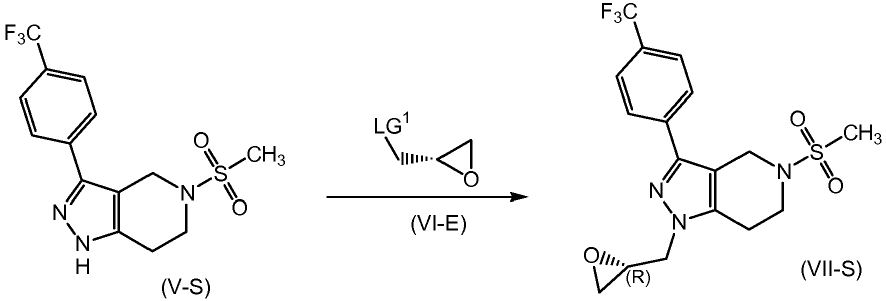 reacting a compound of formula (V-S) with a compound of formula (VIE), wherein LG¹ is a leaving group; in the presence of an organic or inorganic base; in an organic solvent; to yield the corresponding compound of formula (VII-S) 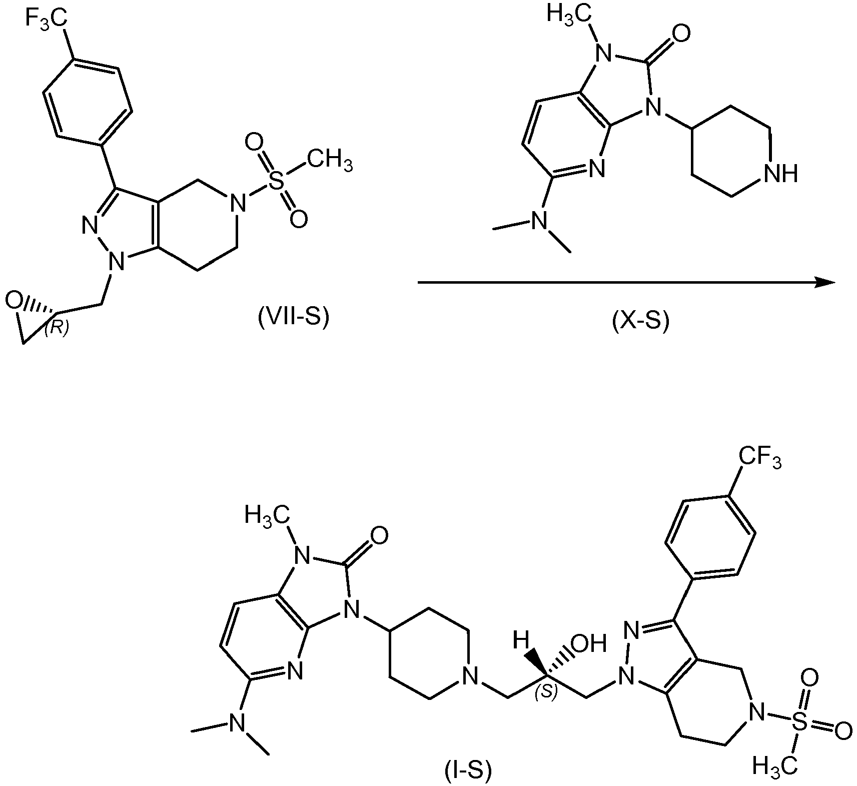 reacting the compound of formula (VII-S) with a compound of formula (X-S); in an organic solvent; to yield the corresponding compound of formula (IS).

The present invention is further directed to a process for the preparation of the compounds of formula (I) 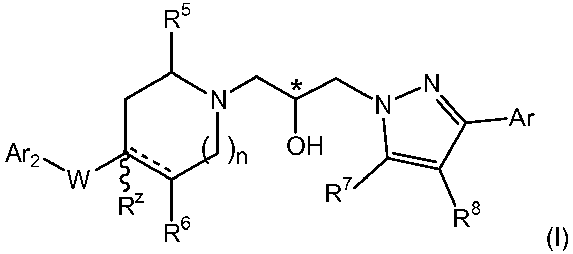 wherein
Ar₂ is a monocyclic or bicyclic ring system, unsaturated, saturated or aromatic, optionally fused, optionally including between 1 and 5 heteroatom ring moieties independently selected from the group consisting of O, S, N, SO₂ and C=O; wherein said Ar₂ ring system is optionally substituted with between 1 and 4 substituents;
W represents O, S, NR²⁷, C=O, (C=O)NH, NH(C=O),CHR²⁸, or a covalent bond;
R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂ or R³⁰R³¹NSO₂; or alternatively, R²⁷ and part of Ar₂ can be taken together to form an optionally substituted 5- to 6-membered heterocyclic ring with optionally 1 to 3 additional heteroatom moieties in the ring selected from O, NR⁹, NR¹⁰, N, SO₂, C=O and S; which ring may be saturated, unsaturated or aromatic; wherein R⁹ and R¹⁰ are independently selected from the group consisting of H, C₁₋₃alkyl, and -CH₂CO₂(C₁₋₄alkyl);
R²⁸ is hydrogen, C₁₋₅alkyl, C₃₋₅alkenyl, hydroxy, phenyl, benzyl, C₁₅heterocyclyl, R²⁹O, R³⁰R³¹NC=O, R²⁹S, R²⁹SO, R²⁹SO₂ or R³⁰R³¹NSO₂;
R²⁹ is C₁₋₅alkyl, C₃₋₅akenyl, phenyl, benzyl or C₁₋₅heterocyclyl;
R³⁰ and R³¹ are each independently selected from the group consisting of hydrogen, C₁₋₅alkyl, C₃₋₅alkenyl, phenyl, benzyl, phenethyl, naphthyl, and C₁₅heteroaryl; alternatively R³⁰ and R³¹ can be taken together to form an optionally substituted 4- to 7-membered ring carbocyclic or heterocyclic ring, which ring may be saturated, unsaturated or aromatic;
R^{z} is H or OH and the dashed line is absent; or R^{z} is absent where the dashed line is an sp² bond;
R⁵ and R⁶ are each independently selected from the group consisting of hydrogen and C₁₋₅alkyl;
n is an integer selected from 0, 1 or 2;
R⁷ and R⁸ are each independently hydrogen, C₁₋₅alkyl, C₂₋₅alkenyl, C₁₅alkoxy, C₁₋₅alkylthio, halogen, or a 4-7 membered carbocyclyl or heterocyclyl;
alternatively, R⁷ and R⁸ can be taken together to form an optionally substituted 5- to 7-membered carbocyclyc or heterocyclic ring, which ring may be unsaturated or aromatic, and may be optionally substituted with between one and three substituents independently selected from the group consisting of halo, cyano, amino, hydroxy, nitro, R⁴, R⁴O-, R⁴S-, R⁴O(C₁₋₅alkylene)-, R⁴O(C=O)-, R4(C=O₎-, R⁴(C=S)-, R⁴(C=O)O-, R⁴O(C=O)(C=O)-, R⁴SO₂, NHR⁴⁴(C=NH₎-, NHR⁴⁴SO₂-, an NHR⁴⁴(C=O)-;
R⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
R⁴⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O, C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl, C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C1-5heterocyclyl; alternatively, R²² and R²³ can be taken together to form an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; comprising 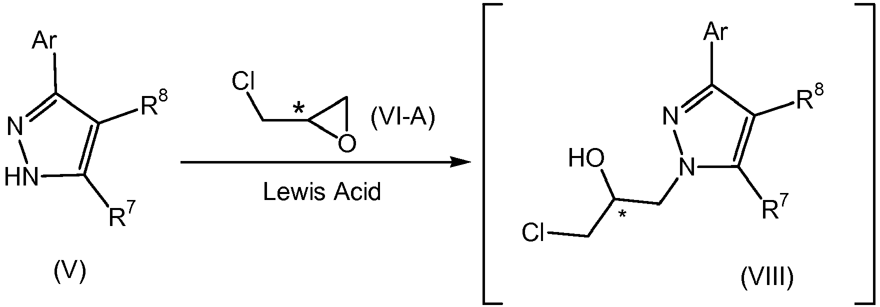 reacting a compound of formula (V) with a compound of formula (VI-A); in the presence of a Lewis acid; in an organic solvent; to yield the corresponding compound of formula (VIII); 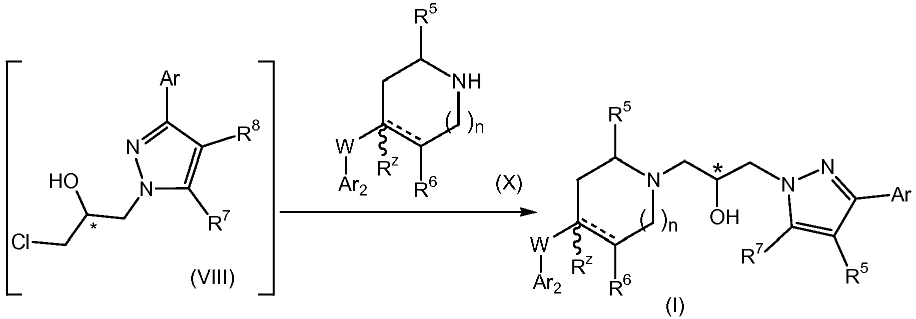 reacting the compound of formula (VIII) with a compound of formula (X); in the presence of an organic or inorganic base; in an organic solvent; to yield the corresponding compound of formula (I).

In an embodiment, the present invention is directed to a process for the preparation of a compound of formula (I-S) 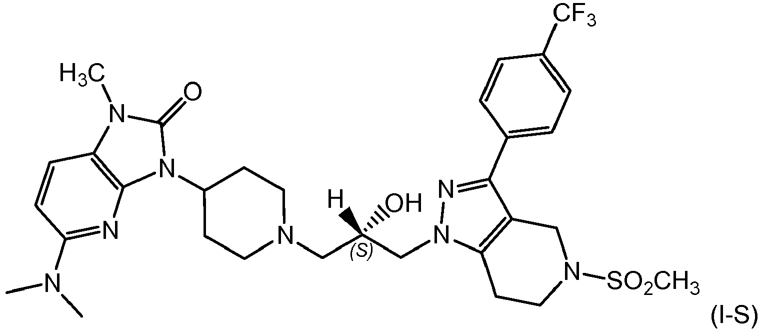 (also known as 5-dimethylamino-3-(1-{2S-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one) or a pharmaceutically acceptable salt, amide or ester thereof; or a stereoisomeric form thereof; comprising 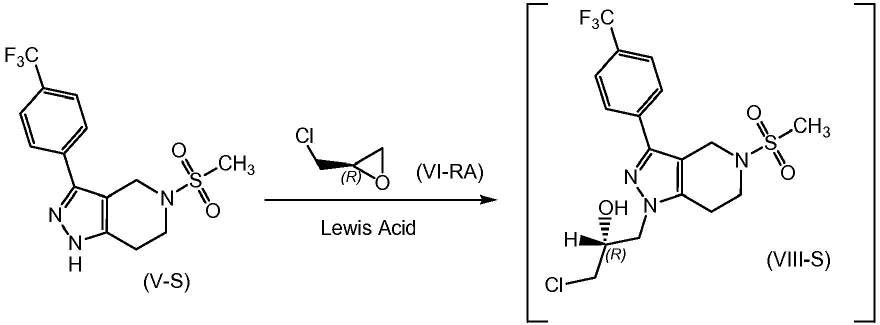 reacting a compound of formula (V-S) with a compound of formula (VI-RA); in the presence of a Lewis acid; in an organic solvent; to yield the corresponding compound of formula (VIII-S); 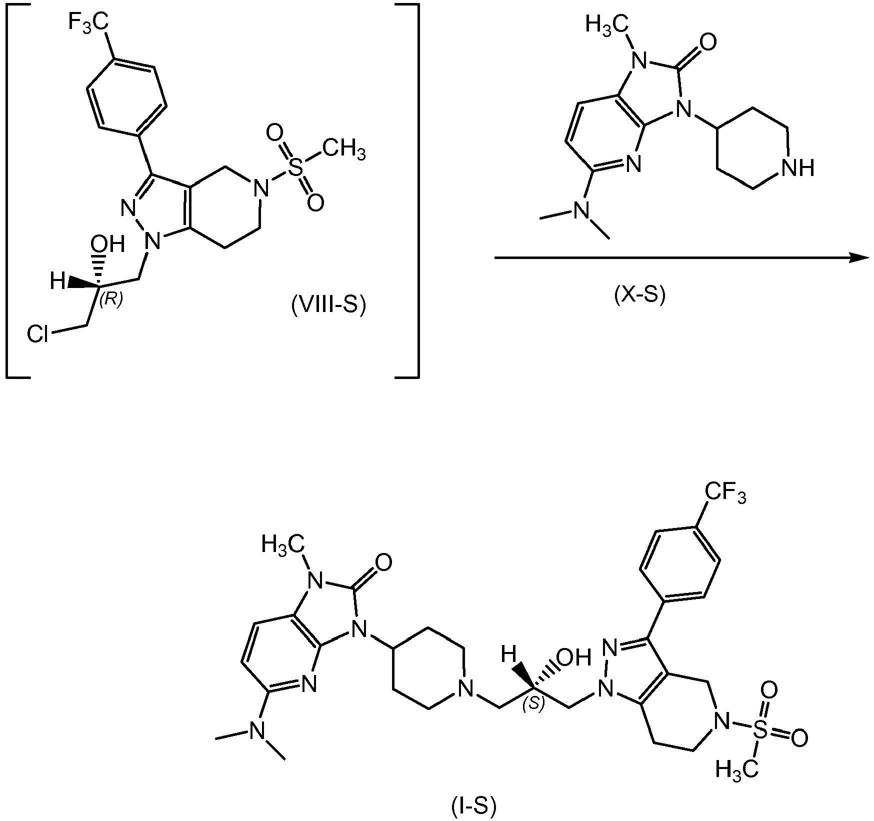 reacting the compound of formula (VIII-S) with a compound of formula (X-S); in the presence of an organic or inorganic base; in an organic solvent; to yield the corresponding compound of formula (I-S).

The present invention is further directed to processes for the preparation of a compound of formula (V-A) 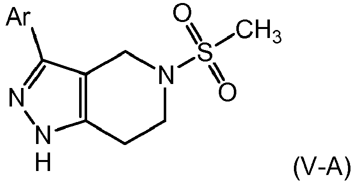 wherein
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R^{23N}C=O C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl,-C₁₅heterocyclyl, C₂₋₈acyl, aroyl, R³⁸OC=O, R²⁵R2^{6N}C=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C₁₋₅heterocyclyl; alternatively, R²² and R²³ can be taken together to form an an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁-₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; as described in more detail in the Schemes and Examples which follow herein. In an embodiment, the present invention is directed to processes for the preparation of the compound of formula (V-S) 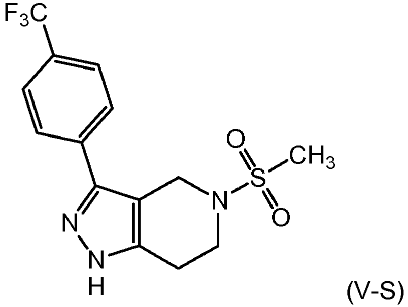 or a pharmaceutically acceptable salt, amide or ester thereof; or a stereoisomeric form thereof; as described in more detail in the Schemes and Examples which follow herein.

The present invention is further directed to a process for the preparation of a compound of formula (VI) 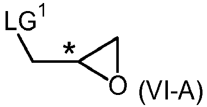 wherein LG¹ is a suitably selected leaving group a such as 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chlorobenzenesulfonyl or 3-chloro-benzenesulfonyl, but not chloro or bromo. The compounds of formula (VI) are useful as intermediates in the synthesis of the compounds of formula (I). In an embodiment, the present invention is directed to a process for the preparation of a compound of formula (VI-S) 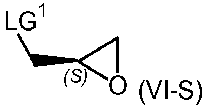 wherein LG¹ is a suitably selected leaving group a suitably selected leaving group such as 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl or 3-chloro-benzenesulfonyl. In another embodiment, the present invention is directed to a process for the preparation of a compound of formula (VI-S), wherein LG¹ is selected from the group consisting of 4-nitrophenyl-sulfonyl and 3-nitrophenyl-sulfonyl. In another embodiment, the present invention is directed to a process for the preparation of a compound of formula (VI-R) 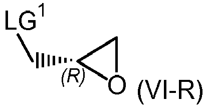 wherein LG¹ is a suitably selected leaving group a suitably selected leaving group such as 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl or 3-chloro-benzenesulfonyl. In another embodiment, the present invention is directed to a process for the preparation of a compound of formula (VI-R), wherein LG¹ is selected from the group consisting of 4-nitrophenyl-sulfonyl and 3-nitrophenyl-sulfonyl.

In another embodiment, the present invention is directed to a process for the preparation of a compound of formula (VI) wherein LG¹ is -O-SO₂-(3-nitrophenyl). In another embodiment, the present invention is directed to a process for the preparation of a compound of formula (VI-S) wherein LG¹ is -O-SO₂-(3-nitrophenyl). In another embodiment, the present invention is directed to a process for the preparation of a compound of formula (VI-R) wherein LG¹ is -O-SO₂-(3-nitrophenyl).

The present invention is further directed to a process for the preparation of a compound of formula (X-A) 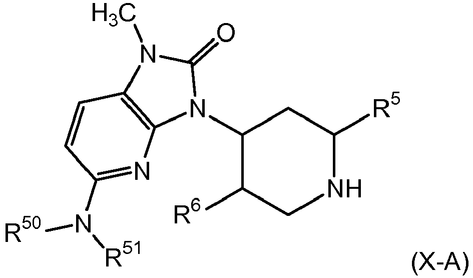 wherein
R⁵ and R⁶ are each independently selected from the group consisting of hydrogen and C₁₋₅alkyl;
R⁵⁰ and R⁵¹ are each independently selected from the group consisting of hydrogen and C₁₋₄alkyl; as described in more detail in the Schemes which follow herein. In an embodiment, the present invention is directed to a process for the preparation of a compound of formula (VIII-S) 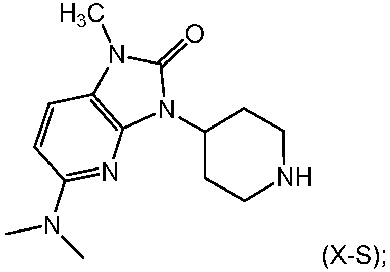
as described in more detail in the Schemes and Examples which follow herein.

The present invention is further directed to a product prepared according to any of the processes described herein.

The present invention is further directed to a crystalline salt of the compound of formula (I-S). In an embodiment, the present invention is directed to a crystalline HCl salt of the compound of formula (I-S). In another embodiment, the present invention is directed to a crystalline HCl salt of the compound of formula (I-S), wherein the crystalline HCl salt of the compound of formula (I-S) is a hexahydrate. In another embodiment, the present invention is directed to a crystalline sulfate salt of the compound of formula (I-S).

In a further aspect, the invention relates to pharmaceutical compositions each comprising an effective amount of a product prepared according to any of the processes described herein and a pharmaceutically acceptable excipient.

In an embodiment, the present invention is directed to methods of treating a disorder mediated by the cathepsin S enzyme (selected from the group consisting of lupus, rheumatoid arthritis and asthma) comprising administering to a subject in need thereof a therapeutically effective amount of a product prepared according to any of the processes described herein or a pharmaceutical composition as described herein. In another embodiment, the present invention is directed to methods of treating, inhibiting or preventing tissue transplant rejection comprising administering to a subject in need thereof a therapeutically effective amount of a product prepared according to any of the processes described herein or a pharmaceutical composition as described herein. In an embodiment of the present inventive method, the disease, disorder, or medical condition mediated by the cathepsin S enzyme is rheumatoid arthritis or asthma.

In another aspect, the present invention is directed to a method for modulating cathepsin S enzyme, comprising exposing the cathepsin S enzyme to an effective amount of a product prepared according to any of the processes described herein.

Another example of the invention is the use of a product prepared according to any of the processes described herein in the preparation of a medicament for treating a disorder mediated by the cathepsin S enzyme, in a subject in need thereof. Another example of the invention is the use of a product prepared according to any of the processes described herein in the preparation of a medicament for treating, inhibiting or preventing tissue transplant rejection, in a subject in need thereof.

An object of the present invention is to overcome or ameliorate at least one of the disadvantages of the conventional methodologies and/or prior art, or to provide a useful alternative thereto.

Additional embodiments, features, and advantages of the invention will be apparent from the following detailed description and through practice of the invention.

### BRIEF DESCRIPTION OF THE FIGURES

Figure 1 illustrates a representative powder XRD spectra for the crystalline, mono-HCL, hexahydrate salt of the compound of formula (I-S).
Figure 2 illustrates a representative powder XRD spectra for the crystalline sulfate salt of the compound of formula (I-S).

### DETAILED DESCRIPTION OF THE INVENTION

The present invention is directed to a process for the preparation of compounds of formula (I) 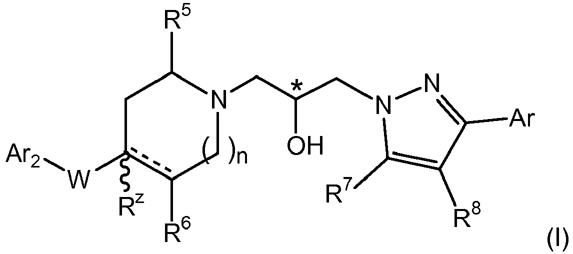 wherein Ar₂, W, R⁵, R², R⁶, n, R⁷, R⁸ and Ar are as herein defined, and pharmaceutically acceptable salts, amides or esters thereof; and stereoisomeric forms thereof. Compounds of formula (I) are inhibitors of the cathepsin S enzyme, and some of them have been proposed as candidates in the treatment of disorders modulated by said enzyme such as lupus, rheumatoid arthritis and asthma and for the treatment, inhibition and / or prevention of tissue transplant rejection.

The present invention is further directed to processes for the preparation of intermediates in the synthesis of the compounds of formula (I). More particularly, the present invention is directed to a process for the preparation of compounds of formula (V-A) 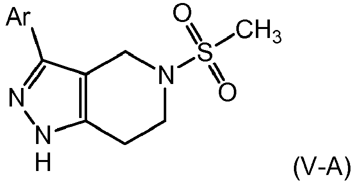 wherein Ar is as herein defined; a process for the preparation of compounds of formula (VI) 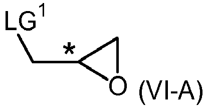 wherein LG¹ is as herein defined; and a process for the preparation of compounds of formula (X-A) 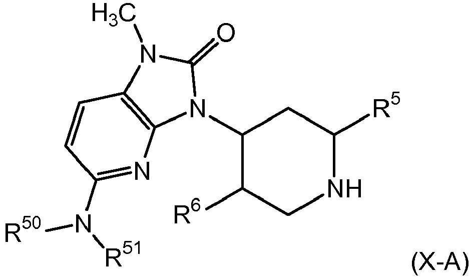 wherein R⁵, R⁶, R⁵⁰ and R⁵¹ are as herein defined. The compounds of formula (V-A), the compounds of formula (VI-A) and the compounds of formula (X-A) are useful as intermediates in the synthesis of the compounds of formula (I).

In an embodiment, the present invention is directed to a compound of formula(I), wherein Ar₂ is selected from 5-7 membered monocyclic rings, and [5,6], [6,6], [6,5], and [5,5] fused bicyclic ring systems, said ring or ring system being carbocyclic or heterocyclic, saturated, unsaturated, or aromatic, and optionally substituted with halo, C₁₋₄alkyl, C₁₋₄haloalkyl, C₁₋₄hydroxyalkyl, nitro, hydroxy, amino, mono- or di-(C₁₋₆alkyl)amino, C₁₋₄alkoxy, C₂₋₄alkoxycarbonyl, C₂₋₆acyl, C₂₋₆acyloxy, C₁₋₅alkylsulfonyl, C₁₋₅alkoxycarbonylC₁₋₄alkoxy, cyano, and mono- or di-(C₁₋₆alkyl)carbamoyl.

In another embodiment, the present invention is directed to a compound of formula (I), wherein (a) Ar₂ is selected from 2,5-di(C₁₋₆ alkyl)aminopyrrolyl or a structure of one of the following 6 formulae: 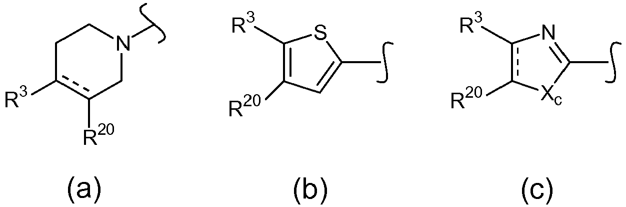 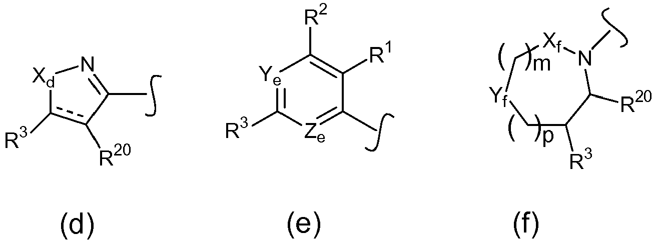
wherein in the formula (a), (b), (c), (d), (e) and (f) as defined above:
   each dashed line is absent or may be an sp² bond;
   m is an integer from 0 or 1; and p is an integer 0 or 1;
   X_{c} is O, S, or N; and X_{d} is O or S;
   Yₑ is nitrogen or R²⁰C; and Zₑ is nitrogen or R²¹C ;
   X_{f} is CHR^{1f}, =N-, NH, C=O, SO₂, CHSR^{1f}; wherein R^{1f} is hydrogen, halogen, C₁₋₅alkoxy, hydroxy, C₁₋₅ alkyl, C₃₋₅alkenyl, cyano, nitro, R³⁹R⁴⁰N, C₂₋₈acyl, C₁₋₅heterocyclyl, (C₁₋₅heterocyclyl)C₁₋₅alkylene, R⁴¹S, R⁴¹SO, R⁴¹SO₂, R³⁹OC=O, R³⁹R⁴⁰NC=O, R³⁹R⁴⁰NSO₂, R⁴¹SO₃- or R³⁹(C=O)O-; wherein R³⁹ and R⁴⁰ are each independently selected from hydrogen, C₁₋₅alkyl, C₃₋₅alkenyl, phenyl, benzyl, phenethyl or C₁₋₅ heteroaryl; and wherein R⁴¹ is C₁₋₅alkyl, C₃₋₅alkenyl, phenyl, benzyl, phenethyl, or C₁₋₅heterocyclyl;
   Y_{f} is CH₂, CHR^{2f}, =CR^{2f}, O, or NR^{2f}, wherein R^{2f} is H, C₁₋₅alkyl, C₃₋₅alkenyl, C₂₋₈acyl, C₁₋₅heterocyclyl, (C₁₋₅heterocyclyl)-C₁₋₅alkylene, C₁₅haloalkyl, C₁₋₅cyanoalkyl, (C₁₋₅alkoxycarbonyl)C₁₋₅alkylene or (phenylcarbonyl)NH-;
   R¹ is hydrogen, halogen, C₁₋₅ alkoxy, hydroxy, C₁₋₅ alkyl, C₂₅alkenyl, cyano, nitro, R^{a}R^{b}N, C₂₋₈acyl, C₁₋₅heterocyclyl, (C₁₋₅heterocyclyl)C₁₋₅alkylene, R¹¹S, R¹¹SO, R¹¹SO₂, R^{c}OC=O, R^{c}R^{d}NC=O, or R^{c}R^{d}NSO₂;
   R^{a} is selected from hydrogen, C₁₋₅ alkyl, C₃₋₅alkenyl, phenyl, benzyl, phenethyl, C₁₋₅heterocyclyl, C₂₋₈acyl, aroyl, R^{j}OC=O, RⁱRⁱNC=O, R¹²SO, R¹²SO₂, R¹²S, and RⁱRⁱNSO₂; wherein Rⁱ and R^{j} are each independently hydrogen, C₁₋₅alkyl, C₃₋₅alkenyl, phenyl, benzyl, phenethyl, or C₁₋₅heteroaryl; alternatively, Rⁱ and R^{j} can be taken together to form an optionally substituted 4- to 7- membered heterocyclic ring, which ring may be saturated, unsaturated or aromatic; and wherein R¹² is C₁₋₅alkyl, C₃₋₅alkenyl, phenyl, benzyl, phenethyl, or C₁₋₅heterocyclyl;
   R^{b} is selected from hydrogen, C₁₋₅ alkyl, C₃₋₅ alkenyl, phenyl, benzyl, phenethyl, and C₁₋₅heteroaryl;
   alternatively, R^{a} and R^{b} can be taken together to form an optionally substituted 4- to 7- membered heterocyclic ring, which ring may be saturated, unsaturated or aromatic;
   R^{c} and R^{d} are each independently are hydrogen, C₁₋₅alkyl, C₃₅alkenyl, phenyl, benzyl, phenethyl, or C₁₋₅ heteroaryl; alternatively, R^{c} and R^{d} can be taken together to form an optionally substituted 4- to 7-membered heterocyclic ring, which ring may be saturated, unsaturated or aromatic;
   R¹¹ is C₁₋₅alkyl, C₃₋₅alkenyl, phenyl, benzyl, phenethyl, or C₁₅heterocyclyl;
   R² is hydrogen, halogen, C₁₋₅alkoxy, hydroxy, C₁₋₅alkyl, C₂₅alkenyl, cyano, nitro, R^{e}R^{f}N, C₁₋₅heterocyclyl, or C₂₋₈acyl;
   R^{e} is selected from hydrogen, C₁₋₅alkyl, C₃₋₅alkenyl, phenyl, benzyl, phenethyl, C₁₋₅heterocyclyl, C₂₋₈acyl, aroyl, R³²C=O, R³²R³³NC=O, R¹³SO, R¹³SO₂, R¹³S, and R³²R³³NSO₂; wherein R³² and R³³ are each independently selected from hydrogen, C₁₋₅alkyl, C₃₅alkenyl, phenyl, benzyl, phenethyl, and C₁₋₅heteroaryl; and wherein R¹³ is C₁₋₅alkyl, C₃₋₅alkenyl, phenyl, benzyl, phenethyl, or C₁₋₅heterocyclyl;
   R^{f} is selected from hydrogen, C₁₋₅alkyl, C₃₋₅alkenyl, phenyl, benzyl, phenethyl, and C₁₋₅heteroaryl;
   alternatively, R^{e} and R^{f} can be taken together to form an optionally substituted 4- to 7- membered heterocyclic ring, which ring may be saturated, unsaturated or aromatic;
   R³ is hydrogen, halogen, C₁₋₅ alkoxy, hydroxy, C₁₋₅ alkyl, C₂₅alkenyl, cyano, nitro, R^{g}R^{h}N, C₂₋₈acyl, C₁₋₅heterocyclyl, R^{h}OC=O, R^{g}R^{h}NC=O, or R^{g}R^{h}NSO₂;
   R^{g} is hydrogen, C₁₋₅alkyl, C₃₋₅alkenyl, phenyl, benzyl, phenethyl, C₁₋₅heterocyclyl, C₂₋₈acyl, aroyl, R¹⁷OC=O, R¹⁷R¹⁸NC=O, R¹⁶S, R¹⁶SO, R¹⁶SO₂, or R¹⁷R¹⁸NSO₂; and R^{h} is hydrogen, C₁₋₅ alkyl, C₃₋₅alkenyl, phenyl, benzyl, phenethyl or C₁₋₅heterocyclyl; alternatively, R^{g} and R^{h} can be taken together to form an optionally substituted 4- to 7-membered heterocyclic ring, which ring may be saturated, unsaturated or aromatic;
   wherein R¹⁷ and R¹⁸ are each independently hydrogen, C₁₋₅alkyl, C₃₋₅alkenyl, phenyl, benzyl, or C₁₋₅heterocyclyl; alternatively, R¹⁷ and R¹⁸ can be taken together to form an optionally substituted 4- to 7-membered heterocyclic ring, which ring may be saturated, unsaturated or aromatic; and wherein R¹⁶ is C₁₋₅ alkyl, C₃₋₅alkenyl, phenyl, benzyl, phenethyl, or C₁₋₅heterocyclyl;
   R²⁰ is hydrogen, halogen, C₁₋₅alkoxy, C₁₋₅alkyl, C₂₋₅alkenyl, cyano, nitro, R^{m}RⁿN, C₂₋₈acyl, R^{m}OC=O, R¹⁴S, R¹⁴SO or R¹⁴SO₂;
   R^{m} is selected from hydrogen, C₁₋₅alkyl, C₃₋₅alkenyl, phenyl, benzyl, phenethyl, C₁₋ heterocyclyl, C₂₋₈acyl, aroyl, R³⁴OC=O, R³⁴R³⁵NC=O, R¹⁵SO, R¹⁵SO₂, R¹⁵S, and R³⁴R³⁵NSO₂; wherein R³⁴ and R³⁵ are each independently selected from hydrogen, C₁₋₅alkyl, C₃₅alkenyl, phenyl, benzyl, phenethyl, and C₁₋₅heteroaryl; and wherein R¹⁵ is C₁₋₅alkyl, C₃₋₅alkenyl, phenyl, benzyl, phenethyl, or C₁₋₅heterocyclyl;
   Rⁿ is selected from hydrogen, C₁₋₅alkyl, C₃₋₅alkenyl, phenyl, benzyl, phenethyl, and C₁₋₅heteroaryl;
   alternatively, R^{m} and Rⁿ can be taken together to form an optionally substituted 4- to 7- membered heterocyclic ring, which ring may be saturated, unsaturated or aromatic;
   R¹⁴ is C₁₋₅alkyl, C₃₋₅alkenyl, phenyl, benzyl, phenethyl, or C₁₅heterocyclyl;
   R²¹ is hydrogen, halogen, C₁₋₅alkoxy, C₁₋₅alkyl, C₂₋₅alkenyl, cyano, nitro, R^{o}R^{p}N, C₂₋₈acyl, R¹⁶OC=O, R¹¹S, R¹¹SO, or R¹¹SO₂;
   R^{o} is selected from hydrogen, C₁₋₅alkyl, C₃₋₅alkenyl, phenyl, benzyl, phenethyl, C₁₋₅heterocyclyl, C₂₋ acyl, aroyl, R³⁶OC=O, R³⁶R³⁷NC=O, R¹⁹SO, R¹⁹SO₂, R¹⁹S, and R³⁶R³⁷NSO₂; wherein R³⁶ and R³⁷ are each independently selected from hydrogen, C₁₋₅alkyl, C₃₅alkenyl, phenyl, benzyl, phenethyl, and C₁₋₅heteroaryl; and wherein R¹⁹ is C₁₋₅alkyl, C₃₋₅alkenyl, phenyl, benzyl, phenethyl, or C₁₋₅heterocyclyl;
   R^{p} is selected from hydrogen, C₁₋₅alkyl, C₃₋₅alkenyl, phenyl, benzyl, phenethyl, and C₁₋₅heteroaryl;
   alternatively, R^{o} and R^{p} can be taken together to form an optionally substituted 4- to 7- membered heterocyclic ring, which ring may be saturated, unsaturated or aromatic;
   R¹⁶ is C₁₋₅alkyl, C₃₋₅alkenyl, phenyl, benzyl, phenethyl, or C₁₅heterocyclyl;
   alternatively, R³ and R²⁰ or R³ and R²¹ can be taken together to form an optionally substituted 5- or 6-membered carbocyclic or heterocyclic ring, which ring may be saturated, unsaturated or aromatic; and wherein said ring may be optionally substituted with halo, di(C₁₅alkyl)amino, C₂₋₅acyl, and C₁₋₅alkoxy;
   and (b) R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂, or R³⁰R³¹NSO₂;
   or alternatively, R²⁷ and R¹ are taken together to form an optionally substituted 5- or 6-membered heterocyclic ring with optionally 1 to 3 additional heteroatom moieties in the ring selected from O, NR⁹, NR¹⁰, N, SO₂, C=O, and S; which ring may be saturated, unsaturated or aromatic; wherein R⁹ and R¹⁰ are independently selected from H, C₁₋₃alkyl, and -CH₂CO₂(C₁₋₄alkyl);
   and wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅alkyl, C₁₋₅alkoxy, -COOH, C₂₋₆acyl, [di(C₁₋₄alkyl)amino]C₂₋₅alkylene, [di(C₁₋₄alkyl)amino]C₂₋ alkyl-NH-CO-, and C₁₋₅haloalkoxy.

In additional embodiments, the present invention is directed to compounds of formula (I) wherein
(a) Ar₂ is selected from formulae (e);
(b) Ar₂ is selected from formulae (f);
(c) Ar₂ is selected from formula (a)-(d);
(d) R¹ is halogen, C₁₋₅ alkoxy, hydroxy, C₁₋₅ alkyl, cyano, nitro, R^{a}R^{b}N or is taken together with R²⁷;
(e) R¹ is taken together with R²⁷;
(f) R¹ and R²⁷ taken together are selected from the group consisting of (1) -CH₂NR⁹-(C=O)-, (2) OCH₂(C=O)-, (3) -CH₂CH₂(C=O)-, (4) -CH₂-O(C=O)-, (5) -CH₂S(C=O)-, (6) -O(C=O)-, (7) -CH₂(C=O)-, (8) -NR⁹(C=O)-, (9) - NR⁹(SO₂)-, (10) -CH₂NR⁹SO₂-, (11) -NR⁹CH₂(C=O)- and -SCH₂(C=O)-;
(g) R¹ and R²⁷ taken together are selected from the group consisting of (1) -CH₂-(C=O)-, (2) -O(C=O)-, (3) -CH₂CH₂-, (4) -S(C=O)-, (5) -N=N-, (6)-NR⁹SO₂-, (7) -N=CR⁹-, (8) -NR⁹(C=O)- and (9) -CH=CH-;
(h) R² is hydrogen, halogen, C₁₋₅ alkoxy, C₁₋₅ alkyl, cyano, or R^{e}R^{f}N, where R^{e} and R^{f} are H or C ₁₋₅ alkyl, or are taken together to form a 5-7 membered heterocyclic ring;
(I R³ is hydrogen, halogen, C₁₋₅ alkoxy, C₁₋₅ alkyl, cyano, nitro, or R⁹R^{h}N, where R^{e} and R^{f} are H or C₁₋₅ alkyl, or are taken together to form a 5-7 membered heterocyclic ring;
(j) R⁵ and R⁶ are independently selected from hydrogen and C₁₋₃alkyl;
(k) one of R⁵ and R⁶ is H;
(l) R⁵ and R⁶ are each H;
(m) one of R⁷ and R⁸ is H and the other is 5-7 membered carbocyclyl or heterocyclyl;
(n) R⁷ and R⁸ are taken together to form an optionally substituted 5- to 7- membered carbocyclic or heterocyclic ring;
(o) R⁷ and R⁸ taken together form a six-membered heterocyclyl;
(p) R⁷ and R⁸ taken together form pyridinyl, pyrimidinyl, or piperazinyl, optionally N-substituted with -(C=O)R⁴, SO₂-R⁴, or -(C=O)NHR⁴;
(q) each of R^{a} , R^{e}, R^{m}, and R^{o} is independently selected from hydrogen, C₁₋₅ alkyl, C₂₋₈ acyl, and the respective ROC=O, RRNC=O, RSO, RSO₂, and RRNSO₂ groups;
(r) each of R^{a}, R^{e}, R^{m}, R^{o}, R^{b}, R^{f}, Rⁿ, and R^{p} is independently selected from hydrogen and C₁₋₅alky!; or, independently, R^{a} and R^{b}, R^{e} and R^{f}, R^{m} and Rⁿ, and R^{o} and R^{p}, taken together, form an optionally substituted 4- to 7-membered carbocyclic or heterocyclic ring;
(s) (1)R^{a} and R^{b} taken together are independently morpholinyl, piperidinyl, or pyrrolidinyl; (2) R^{e} and R^{f} taken together are morpholinyl, piperidinyl, or pyrrolidinyl; or (3) both (1) and (2) apply;
(t) each of R^{c} and R^{d} , Rⁱ and R^{j} is independently hydrogen or C₁₋₅ alkyl, alternatively, R^{c} and R^{d}, Rⁱ and Rj, independently, can be taken together to form an optionally substituted 4- to 7- membered heterocyclic ring, which ring may be saturated, unsaturated or aromatic;
(u) R^{c} and R^{d}, Rⁱ and Rⁱ independently, are taken together to form an optionally substituted 4- to 7- membered heterocyclic ring, which ring may be saturated, unsaturated or aromatic;
(v) each of R^{b}, R^{f}, Rⁿ, R^{p}, R³², R³³, R³⁴, R³⁵, R³⁶, R³⁷, R³⁹, and R⁴⁰ is independently H or C₁₋₅ alkyl;
(w) each of R¹¹, R¹², R¹³, R¹⁴, R¹⁵, R¹⁶, R¹⁹, R³⁸, and R⁴¹ is independently H or C₁₋₅ alkyl;
(x) R^{g} is C₁₋₅alkyl, C₂₋₈ acyl, R¹⁷OC=O, R¹⁷R¹⁸NC=O, R¹⁶S, R¹⁶SO, R¹⁶SO₂, or R¹⁷R¹⁸NSO₂; and R^{h} hydrogen or C₁₋₅ alkyl; alternatively, R^{g} and R^{h} can be taken together to form an optionally substituted 4- to 7- membered heterocyclic ring;
(y) R¹⁷ and R¹⁸ independently are hydrogen or C₁₋₅ alkyl;
(z) n is 1;
(aa) n is 0;
(bb) each of R²⁰ and R²¹ is independently selected from hydrogen, halogen, C₁₋₅ alkoxy, C₁₋₅ alkyl, cyano, nitro, and R^{m}RⁿN or R^{o}R^{p}N, respectively;
(cc) each of R²⁰ and R²¹ is independently selected from hydrogen, halogen, C₁₋₃ alkyl, and R^{m}RⁿN or R^{o}R^{P}N, respectively;
(dd) Ar represents a monocyclic ring, optionally substituted with 1 to 2 substituents selected from halogen, C₁₋₅ alkyl, cyano, nitro, R²²R²³N, halomethyl, and halomethoxy;
(ee) Ar is a six membered ring substituted with between 1 and 2 substituents independently selected from methyl, halogen, CF₃, and OCF₃, said substituent or substituents being at the 4- position, or at the 3- and 4- positions, respectively;
(ff) each of R²², R²³, and R²⁴ is hydrogen or C₁₋₅alkyl;
(gg) R²⁵ and R²⁶ independently are hydrogen or C₁₋₅ alkyl; or, alternatively, R²⁵ and R²⁶ can be taken together to form an optionally substituted 4- to 7- membered heterocyclic ring;
(hh) each of R²⁵ and R²⁶ is independently hydrogen or C₁₋₅ alkyl;
(ii) W is NR²⁷;
(jj) W is CHR²⁸, and R²⁸ is hydrogen or C₁₋₅ alkyl;
(kk) R²⁹ is C₁₋₅ alkyl; or R³⁰ and R³¹ are independently selected from hydrogen and C₁₋₅ alkyl, or R³⁰ and R³¹ are taken together to form a 5-6 membered heterocyclyl;
(II) Ar₂ is formula (e) and R¹ is halogen, C₁₋₅ alkoxy, hydroxy, C₁₋₅ alkyl, cyano, nitro, and R^{a}R^{b}N, or R¹ can be taken together with R²⁷ as provided below; R² is hydrogen, halogen, C₁₋₅ alkoxy, C₁₋₅ alkyl, or R^{e}R^{f}N; R³ is hydrogen, halogen, C₁₋₅ alkoxy, hydroxy, C₁₋₅ alkyl, cyano, R⁹R^{h}N; R⁵ and R⁶ are independently selected from hydrogen and C₁₋₃alkyl;
(mm) R⁷ and R⁸ independently are taken together to form an optionally substituted 5- to 7- membered carbocyclic or heterocyclic ring, which ring may be saturated, unsaturated or aromatic;
(nn) each of R^{a}, R^{e} , R^{m} , and R^{o} is independently selected from hydrogen, C₁₋₅ alkyl, C₂₋₈ acyl, and the respective ROC=O, RRNC=O, RS, RSO, RSO₂, and RRNSO₂ groups;
(oo) each of R^{b} , R^{f} ,Rⁿ, and R^{p}, is independently selected from hydrogen and C₁₋₅ alkyl; each of R¹¹, R¹², R¹³, R¹⁴, R¹⁵, R¹⁶, R¹⁹, and R³⁸ is independently C₁₋₅ alkyl; each of R^{c} and R^{d}, Rⁱ and R^{j}, R³² and R³³, R³⁴ and R³⁵ , R³⁶ and R³⁷ are independently are hydrogen or C₁₋₅ alkyl, or are taken together to form an optionally substituted 4- to 7- membered heterocyclic ring;
(pp) R^{g} is hydrogen, C₁₋₅alkyl, C₂₋₈ acyl, R¹⁷OC=O, R¹⁷R¹⁸NC=O, R¹⁶S, R¹⁶SO, R¹⁶SO₂, or R¹⁷R¹⁸NSO₂; R^{h} is hydrogen or C₁₋₅ alkyl; alternatively, R^{g} and R^{h} can be taken together to form an optionally substituted 4- to 7- membered heterocyclic ring; R¹⁷ and R¹⁸ independently are hydrogen or C₁₋₅ alkyl; n is 0 or 1;
(qq) Yₑ is nitrogen or R²⁰C; Zₑ is nitrogen or R²¹C;
(rr) R²⁰ and R²¹ are independently selected from hydrogen, halogen, C₁₋₅ alkoxy, C₁₋₅alkyl, cyano, nitro, and R^{m}RⁿN or R^{o}R^{P}N, respectively; alternatively, R³ and R²⁰ or R³ and R²¹ can be taken together to form an optionally substituted 5- or 6-membered carbocyclic or heterocyclic ring;
(ss) Ar represents a monocyclic or bicyclic aryl or heteroaryl ring, optionally substituted with between 1 and 3 substituents independently selected from halogen, C₁₋₅ alkoxy, C₁₋₅ alkyl, cyano, nitro, R²²R²³N, R²⁴SO₂, R²⁴OC=O, R²⁵R²⁶NC=O, CF₃, OCF₃, CF₃S, and C₁₋₅ alkylthio; R²² is hydrogen, C₁₋₅alkyl, phenyl, benzyl, phenethyl, C₁₋₅ heterocyclyl, C₂₋₈ acyl, aroyl, R²⁴OC=O, R²⁵R²⁶NC=O, R²⁴SO, R²⁴SO₂, or R²⁵R²⁶NSO₂; R²³ is hydrogen or C₁₋₅alkyl;
(tt) alternatively, R²² and R²³ can be taken together to form an optionally substituted 4- to 7- membered heterocyclic ring; R²⁴ is hydrogen or C₁₋₅ alkyl; R²⁵ and R²⁶ are independently hydrogen or C₁₋₅ alkyl; or, alternatively, R²⁵ and R²⁶ can be taken together to form an optionally substituted 4- to 7- membered heterocyclic; W is NR²⁷ or CHR²⁸; R²⁷ is hydrogen, C₁₋₅ alkyl, R²⁹OC=O, R³⁰R³¹NC=O R²⁹SO R²⁹SO₂, or R³⁰R³¹NSO²; or, alternatively, R²⁷ and R¹ can be taken together to form an optionally substituted 5- or 6-membered heterocyclic ring, which ring may be saturated, unsaturated or aromatic; R²⁸ is hydrogen, hydroxy, C₁₋₅ heterocyclyl, phenyl, or C₁₋₅ alkyl; R²⁹ is C₁₋₅ alkyl; R³⁰ and R³¹ are independently selected from hydrogen, C₁₋₅alkyl; alternatively, R³⁰ and R³¹ can be taken together to form an optionally substituted 4- to 7-membered heterocyclic;\
(uu) one of R⁵ and R⁶ is H; R⁷ and R⁸ are taken together to form an optionally substituted 6- membered carbocyclic or heterocyclic ring; and Ar represents a monocyclic ring, optionally substituted with 1 to 2 substituents selected from halogen, 6₁₋₅ alkyl, cyano, nitro, R²²R²³N, CF₃ and OCF₃;
(vv) both R⁵ and R⁶ are each H, and Ar is a six membered ring substituted with between 1 and 2 substituents independently selected from halogen, methyl, CF₃, and OCF₃, said substituent or substituents being at the 4-position, or at the 3- and 4-positions;
(ww) R⁷ and R⁸ taken together form tetrahydropyridinyl, optionally N-substituted with -(C=O)R⁴, SO₂-R⁴, or -(C=O)NHR⁴;
(xx) X_{f} is C=O, SO₂, or CHR^{1f}, and Y_{f} is O or NR^{2f}, where R^{2f} is H, C₁₋₅ alkyl, C₂₋₅ heterocyclyl, C₁₋₅ cyanoalkyl, or (C₁₋₅ alkoxycarbonyl)C₁₋₅ alkylene;
(yy) R^{2f} is H, C₁₋₃ alkyl, or a C ₂₋₅ heterocyclyl;
(zz) X_{f} is C=O, and Y_{f} is O, CHR^{2f} or NR^{2f}, where R^{2f} is H, C ₁₋₅ alkyl, C ₂₅ heterocyclyl, C₁₋₅ cyanoalkyl, or (C₁₋₅ alkoxycarbonyl)C₁₋₅ alkylene; (aaa) X_{f} is C=O and Y_{f} is O;
(bbb) m is 0 and p is 0; m is 0 and p is 1; or m is 1 and p is 0;
(ccc) p is 0;
(ddd) R^{z} is H;
(eee) R^{z} is OH;
(fff) R^{z} is absent;
(ggg) R²⁰ and R³ taken together are a six-membered carbocyclic or heterocyclic ring optionally substituted with between 1 and 3 substituents independently selected from halo, C ₁₋₃ alkoxy, di(C ₁₋₃ alkyl)amino, and C ₂₋₅ acyl;
(hhh) each of R²⁰ and R³ is H; and
(iii) combinations of the above.

In another embodiment, the present invention is directed to compounds of formula (I) wherein Ar or Ar₁ is selected from the group consisting of 4-trifluoromethylphenyl, 4-bromophenyl, 4-chlorophenyl, 4-chloro-3-methylphenyl and 3,4-dichlorophenyl.

In additional embodiments, the present invention is directed to one or more compounds independently selected from the group consisting of 1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-benzoimidazol-2-one; [3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetonitrile; [3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-2-oxo-2,3-dihydro-benzoimidazol-1-yl]-acetic acid ethyl ester; 5-Chloro-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-benzoimidazol-2-one; 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,5-dimethyl-1,3-dihydro-benzoimidazol-2-one; 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-2-one; 3-(1-{3-[3-(4-Bromo-phenyl)-5-methanesulfonyl-4,5,6,7-tetrahydro-pyrazolo[4,3-c]pyridin-1-yl]-2-hydroxy-propyl}-piperidin-4-yl)-5-methoxy-1,3-dihydro-imidazo[4,5-b]pyridin-2-one; 3-(4-Bromo-phenyl)-1-{2-hydroxy-3-[4-(5-methoxy-2-oxo-1,2-dihydro-imidazo[4,5-b]pyridin-3-yl)-piperidin-1-yl]-propyl}-1,4,6,7-tetrahydro-pyrazolo[4,3-c]pyridine-5-carboxylic acid amide; 3-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-5-methoxy-1-methyl-1,3-dihydro-imidazo[4,5-b]pyridin-2-one; 5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1,3-dihydro-imidazo[4,5-b]pyridin-2-one; 1-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-3,4-dihydro-1H-quinolin-2-one; and 4-(1-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-4H-benzo[1,4]oxazin-3-one.

Synthetic methodologies and other features according to this invention include the following:
1. A process for the preparation of a compound of formula (I) 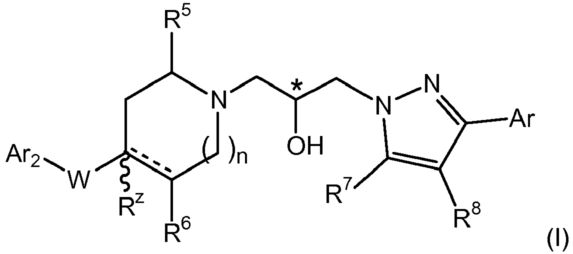 wherein
Ar₂ is a monocyclic or bicyclic ring system, unsaturated, saturated or aromatic, optionally fused, optionally including between 1 and 5 heteroatom ring moieties independently selected from the group consisting of O, S, N, SO₂ and C=O; wherein said Ar₂ ring system is optionally substituted with between 1 and 4 substituents;
W represents O, S, NR²⁷, C=O, (C=O)NH, NH(C=O),CHR²⁸, or a covalent bond;
R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂ or R³⁰R³¹ NSO₂; or alternatively, R²⁷ and part of Ar₂ can be taken together to form an optionally substituted 5- to 6-membered heterocyclic ring with optionally 1 to 3 additional heteroatom moieties in the ring selected from O, NR⁹, NR¹⁰, N, SO₂, C=O and S; which ring may be saturated, unsaturated or aromatic; wherein R⁹ and R¹⁰ are independently selected from the group consisting of H, C₁₋₃alkyl, and -CH₂CO₂(C₁₋₄alkyl);
R²⁸ is hydrogen, C₁₋₅alkyl, C₃₋₅alkenyl, hydroxy, phenyl, benzyl, C₁₅heterocyclyl, R²⁹O, R³⁰R³¹NC=O, R²⁹S, R²⁹SO, R²⁹SO₂ or R³⁰R³¹NSO₂;
R²⁹ is C₁₋₅alkyl, C₃₋₅akenyl, phenyl, benzyl or C₁₋₅heterocyclyl;
R³⁰ and R³¹ are each independently selected from the group consisting of hydrogen, C₁₋₅alkyl, C₃₋₅alkenyl, phenyl, benzyl, phenethyl, naphthyl, and C₁₅heteroaryl; alternatively R³⁰ and R³¹ can be taken together to form an optionally substituted 4- to 7-membered ring carbocyclic or heterocyclic ring, which ring may be saturated, unsaturated or aromatic;
R^{z} is H or OH and the dashed line is absent; or R^{z} is absent where the dashed line is an sp² bond;
R⁵ and R⁶ are each independently selected from the group consisting of hydrogen and C₁₋₅alkyl;
n is an integer selected from 0, 1 or 2;
R⁷ and R⁸ are each independently hydrogen, C₁₋₅alkyl, C₂₋₅alkenyl, C₁₅alkoxy, C₁₋₅alkylthio, halogen, or a 4-7 membered carbocyclyl or heterocyclyl;
alternatively, R⁷ and R⁸ can be taken together to form an optionally substituted 5- to 7-membered carbocyclyc or heterocyclic ring, which ring may be unsaturated or aromatic, and may be optionally substituted with between one and three substituents independently selected from the group consisting of halo, cyano, amino, hydroxy, nitro, R⁴, R⁴O-, R⁴S-, R⁴O(C₁₋₅alkylene)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, an NHR⁴⁴(C=O)-;
R⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
R⁴⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O, C₁₋₅haloalkyl, C₁₅haloalkoxy, C₁₋₅haloalkylthio and C₁₅alkylthio;
R²² is hydrogen, C₁₋₅alkyl, C₃₋₅alkenyl, phenyl, phenethyl, benzyl, C₁₅heterocyclyl, C₂₋₈acyl, aroyl, R³⁸OC=O, R²⁵R²⁶NC=O, R³⁸SO, R³⁸SO2, R³⁸S, or R²⁵R²⁶NSO₂;
R²³ is hydrogen, C₁₋₅alkyl, C₃₋₅alkenyl, phenyl, benzyl or C1-5heterocyclyl; alternatively, R²² and R²³ can be taken together to form an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; comprising 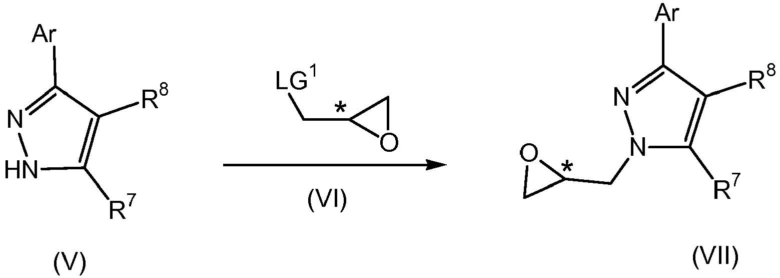 reacting a compound of formula (V) with a compound of formula (VI), wherein LG¹ is a leaving group; in the presence of an organic or inorganic base; to yield the corresponding compound of formula (VII); 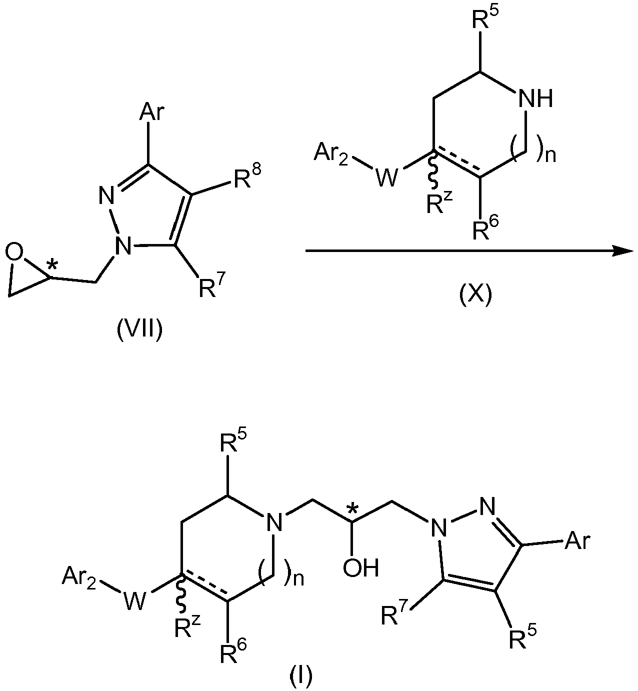 reacting the compound of formula (VII) with a compound of formula (X); in an organic solvent; to yield the corresponding compound of formula (I).
2. A process for the preparation of a compound of formula (I-S) 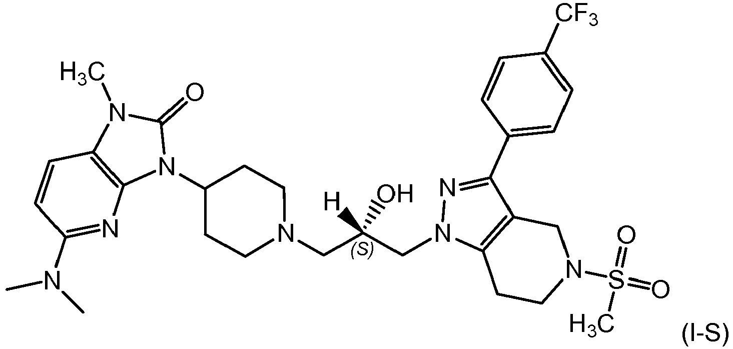 or a pharmaceutically acceptable salt, amide or ester thereof; or a stereoisomeric form thereof; comprising 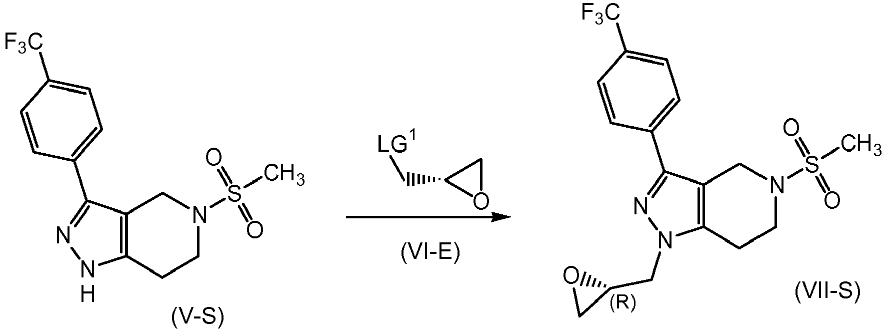 reacting a compound of formula (V-S) with a compound of formula (VIE), wherein LG¹ is a leaving group; in the presence of an organic or inorganic base; to yield the corresponding compound of formula (VII-S); 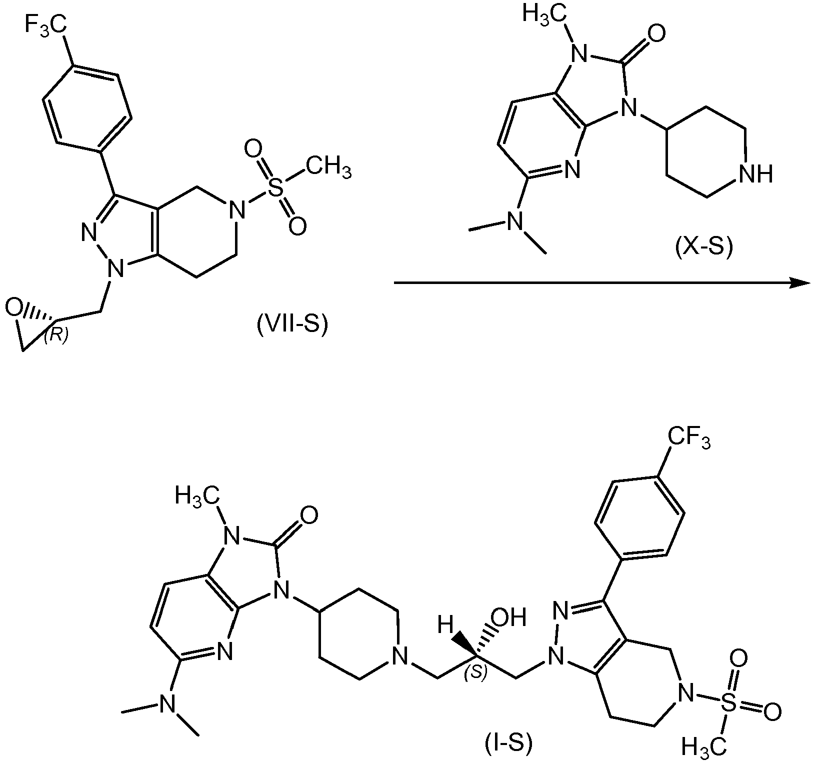 reacting the compound of formula (VII-S) with a compound of formula (X-S); in an organic solvent; to yield the corresponding compound of formula (IS).
3. A process as in item 2 above, wherein LG¹ is selected from the group consisting of 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl and 3-chloro-benzenesulfonyl.
4. A process as in item 3 above, wherein LG¹ is 3-nitrophenyl-sulfonyl.
5. A process as in item 2 above, wherein the compound of formula (VI-E) is present in an amount in the range of from about 0.5 to about 3.0 molar equivalents.
6. A process as in item 5 above, wherein the compound of formula (VI-S) is present in about 1.0 to about 1.5 molar equivalents.
7. A process as in item 2 above, wherein the organic or inorganic base is selected from the group consisting of TEA, DIPEA, pyridine, NaH, KOt-Bu, NaOt-Bu, Cs₂CO₃, K₂CO₃, Na₂CO₃ and CsF.
8. A process as in item 7 above, wherein the organic or inorganic base is Cs₂CO₃.
9. A process as in item 2 above, wherein when the compound of formula (V-S) is reacted with the compound of formula (VI-E) in an organic solvent, the organic solvent is DMF.
10. A process as in item 2 above, wherein the compound of formula (V-S) is reacted with the compound of formula (VI-E) at a temperature in the range of from about 0°C to about 5°C, followed by heating to a temperature in the range of from about room temperature to about 60°C.
11. A process as in item 2 above, wherein the compound of formula (X-S) is present in an amount in the range of from about 0.5 to about 3.0 molar equivalents.
12. A process as in item 11 above, wherein the compound of formula (X-S) is present in an amount of about 1.1 molar equivalents.
13. A process as in item 2 above, wherein when the compound of formula (VII-S) is reacted with the compound of formula (X-S) in an organic solvent, the organic solvent is selected from the group consisting of DCE, ethanol, isopropyl alcohol and DMF.
14. A process as in item 13 above, wherein the organic solvent is ethanol.
15. A process as in item 2 above, wherein the compound of formula (VII-S) is reacted with the compound of formula (X-S) at about solvent reflux temperature,
16. A process as in item 2 above, wherein the compound of formula (VII-S) is reacted with the compound of formula (X-S) in the presence of a Lewis acid selected from the group consisting of titanium propoxide, Yb(OTf)₃ and Al(OTf)₃.
17. A process as in item 16 above, wherein the Lewis acid is present in a catalytic amount.
18. A process for the preparation of the compounds of formula (I) 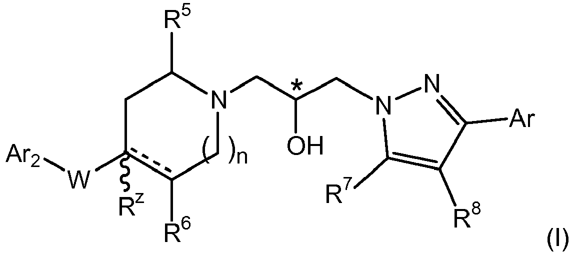 wherein
Ar₂ is a monocyclic or bicyclic ring system, unsaturated, saturated or aromatic, optionally fused, optionally including between 1 and 5 heteroatom ring moieties independently selected from the group consisting of O, S, N, SO₂ and C=O; wherein said Ar₂ ring system is optionally substituted with between 1 and 4 substituents;
W represents O, S, NR²⁷, C=O, (C=O)NH, NH(C=O),CHR²⁸, or a covalent bond;
R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂ or R³⁰R³¹ NSO₂; or alternatively, R²⁷ and part of Ar₂ can be taken together to form an optionally substituted 5- to 6-membered heterocyclic ring with optionally 1 to 3 additional heteroatom moieties in the ring selected from O, NR⁹, NR¹⁰, N, SO₂, C=O and S; which ring may be saturated, unsaturated or aromatic; wherein R⁹ and R¹⁰ are independently selected from the group consisting of H, C₁₋₃alkyl, and -CH₂CO₂(C₁₋₄alkyl);
R²⁸ is hydrogen, C₁₋₅alkyl, C₃₋₅alkenyl, hydroxy, phenyl, benzyl, C₁₅heterocyclyl, R²⁹O, R³⁰R³¹NC=O R²⁹S, R²⁹SO, R²⁹SO₂ or R³⁰R³¹NSO₂;
R²⁹ is C₁₋₅alkyl, C₃₋₅akenyl, phenyl, benzyl or C₁₋₅heterocyclyl;
R³⁰ and R³¹ are each independently selected from the group consisting of hydrogen, C₁₋₅alkyl, C₃₋₅alkenyl, phenyl, benzyl, phenethyl, naphthyl, and C₁₅heteroaryl; alternatively R³⁰ and R³¹ can be taken together to form an optionally substituted 4- to 7-membered ring carbocyclic or heterocyclic ring, which ring may be saturated, unsaturated or aromatic;
R^{z} is H or OH and the dashed line is absent; or R^{z} is absent where the dashed line is an sp² bond;
R⁵ and R⁶ are each independently selected from the group consisting of hydrogen and C₁₋₅alkyl;
n is an integer selected from 0, 1 or 2;
R⁷ and R⁸ are each independently hydrogen, C₁₋₅alkyl, C₂₋₅alkenyl, C₁₅alkoxy, C₁₋₅alkylthio, halogen, or a 4-7 membered carbocyclyl or heterocyclyl;
alternatively, R⁷ and R⁸ can be taken together to form an optionally substituted 5- to 7-membered carbocyclyc or heterocyclic ring, which ring may be unsaturated or aromatic, and may be optionally substituted with between one and three substituents independently selected from the group consisting of halo, cyano, amino, hydroxy, nitro, R⁴, R⁴O-, R⁴S-, R⁴O(C₁₋₅alkylene)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, an NHR⁴⁴(C=O)-;
R⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
R⁴⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O, C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl, C₁₋₅heterocyclyl, C₂₋₈acyl, aroyl, R³⁸OC=O, R²⁵R²⁶NC=O, R³⁸SO, R³⁸SO2, R³⁸S, or R²⁵R²⁶NSO₂;
R²³ is hydrogen, C₁₋₅alkyl, C₃₋₅alkenyl, phenyl, benzyl or C1-5heterocyclyl; alternatively, R²² and R²³ can be taken together to form an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; comprising 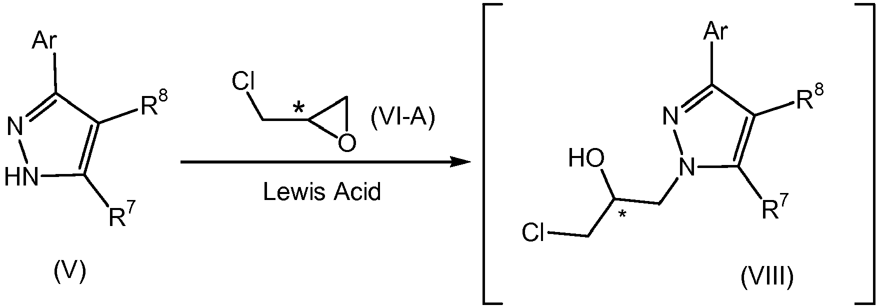 reacting a compound of formula (V) with a compound of formula (VI-A); in the presence of a Lewis acid; in an organic solvent; to yield the corresponding compound of formula (VIII); 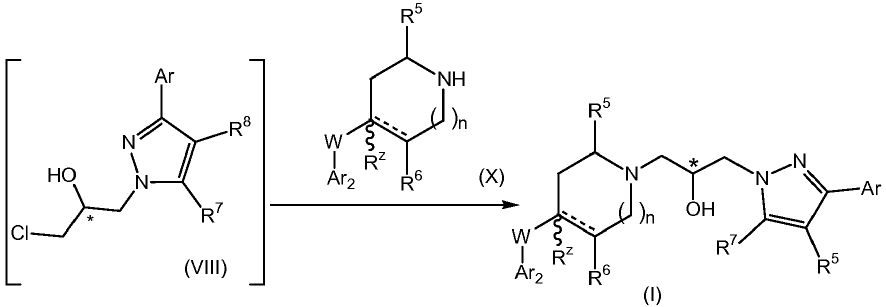 reacting the compound of formula (VIII) with a compound of formula (X); optionally in the presence of an organic or inorganic base; in an organic solvent; to yield the corresponding compound of formula (I).
19. A process for the preparation of a compound of formula (I-S) 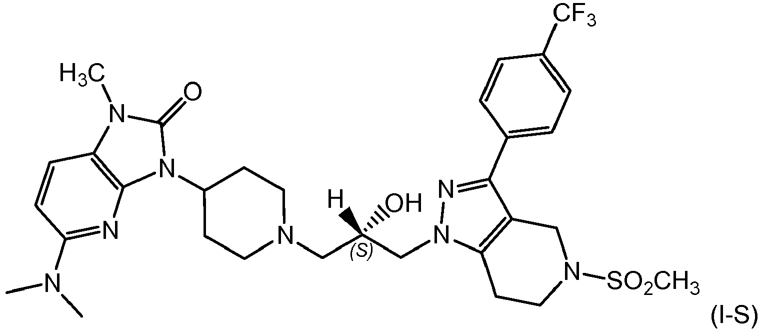 or a pharmaceutically acceptable salt, amide or ester thereof; or a stereoisomeric form thereof; comprising 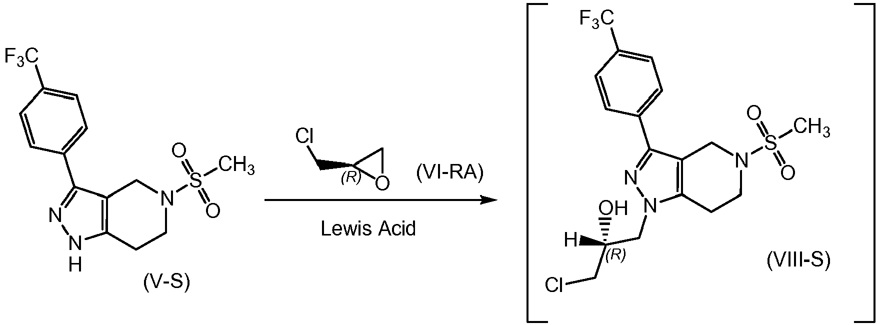 reacting a compound of formula (V-S) with a compound of formula (VI-RA); in the presence of a Lewis acid; in an organic solvent; to yield the corresponding compound of formula (VIII-S); 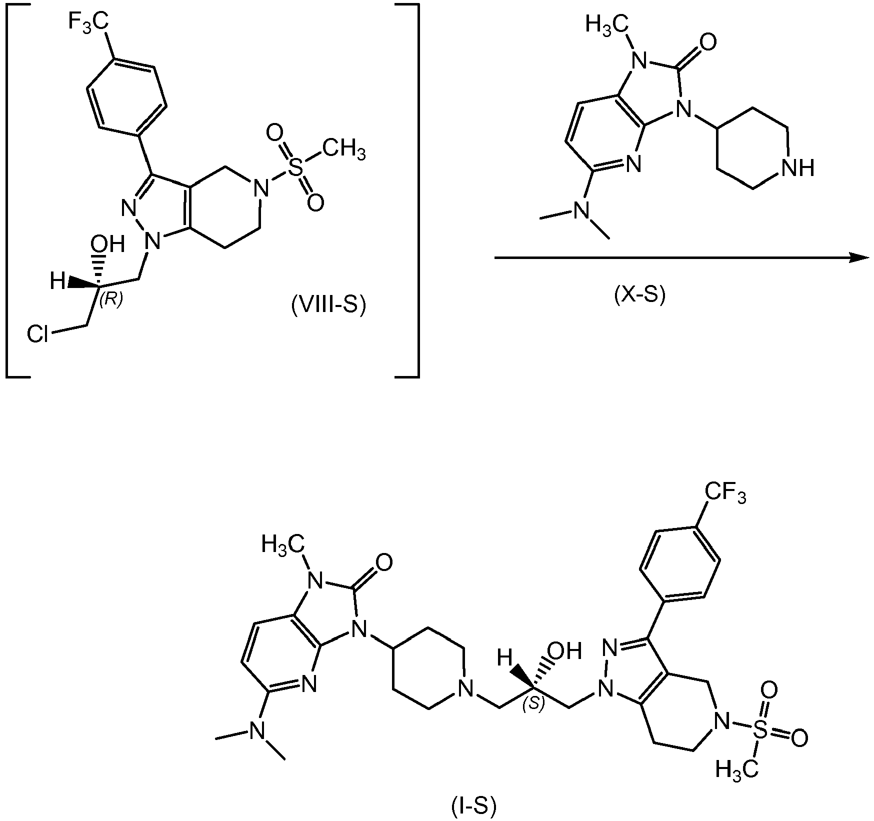 reacting the compound of formula (VIII-S) with a compound of formula (X-S); in an organic solvent; to yield the corresponding compound of formula (IS).
20. A process as in item 19 above, wherein the compound of formula (VI-RA) is present in an amount in the range of from about 1.0 to about 2.0 molar equivalents.
21. A process as in item 20 above, wherein the compound of formula (VI-RA) is present in an amount in the range of from about 1.0 to about 1.1 molar equivalents.
22. A process as in item 19 above, wherein the Lewis acid is selected form the group consisting of titanium propoxide, Yb(OTf)₃ and Al(OTf)₃.
23. A process as in item 19 above, wherein the Lewis acid is present in an amount in the range of from about 0.01 to about 3.0 molar equivalents.
24. A process as in item 23 above, wherein the Lewis acid is present in an amount in the range of from about 0.01 to about 0.5 molar equivalents.
25. A process as in item 24 above, wherein the Lewis acid is present in a catalytic amount.
26. A process as in item 19 above, wherein when the compound of formula (V-S) is reacted with the compound of formula (VI-RA) in an organic solvent, the organic solvent is toluene.
27. A process as in item 19 above, wherein the compound of formula (V-S) is reacted with the compound of formula (VI-RA) at a temperature in the range of from about room temperature to about 80°C.
28. A process as in item 19 above, wherein the compound of formula (X-S) is present in an amount in the range of from about 0.5 to about 3.0 molar equivalents.
29. A process as in item 28 above, wherein the compound of formula (X-S) is present in an amount in the range of from about 1.0 to about 1.5 molar equivalents.
30. A process as in item 19 above, wherein the compound of formula (VIII-S) is reacted with a compound of formula (X-S) in the presence of an organic or inorganic base.
31. A process as in item 30 above, wherein the organic or inorganic base is selected from the group consisting of TEA, DIPEA, pyridine, Cs₂CO₃, K₂CO₃ and Na₂CO₃.
32. A process as in item 31 above, wherein the organic or inorganic base is K₂CO₃.
33. A process as in item 19 above, wherein when the compound of formula (VIII-S) is reacted with the compound of formula (X-S) in an organic solvent, the organic solvent is selected from the group consisting of ethanol, methanol and isopropanol.
34. A process as in item 33 above, wherein the organic solvent is isopropanol.
35. A process as in item 19 above, wherein the compound of formula (VIII-S) is reacted with the compound of formula (X-S) at a temperature in the range of from about 50°C to about solvent reflux temperature/
36. A HCl salt of a compound of formula (I-S) 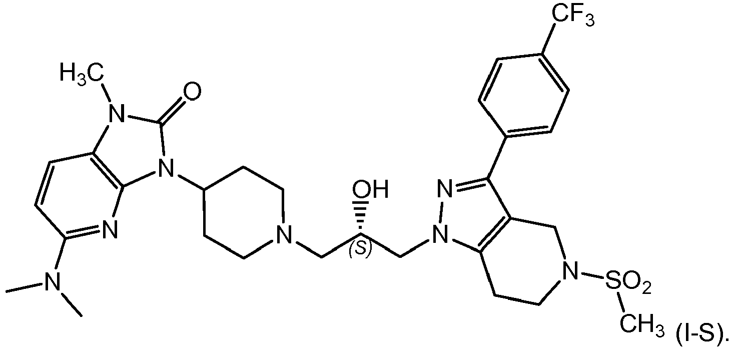
37. An HCl salt of the compound of formula (I-S) as in item 36 above, wherein the salt is crystalline, mono-HCl crystalline.
38. An HCl salt of the compound of formula (I-S) as in item 36 above, wherein the salt is crystalline, mono-HCl and a hexahydrate.
39. A crystalline, hexahydrate mono-HCl salt of a compound of formula (I-S) 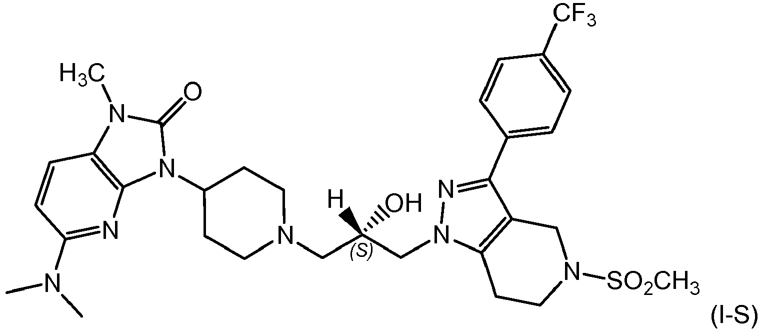 comprising the following X-ray diffraction peaks:

| **Angle (°2θ)** | **d-spacing (Å)** |
|---|---|
| 4.33 | 20.42 |
| 7.58 | 11.67 |
| 8.39 | 10.54 |
| 8.80 | 10.04 |
| 9.16 | 9.66 |
| 12.71 | 6.96 |
| 13.30 | 6.66 |
| 13.69 | 6.47 |
| 14.69 | 6.03 |
| 15.55 | 5.70 |
| 15.96 | 5.55 |
| 18.44 | 4.81 |
| 18.69 | 4.75 |
| 19.07 | 4.65 |

40. A process for the preparation of a crystalline, mono-HCl, hexahydrate salt of a compound of formula (I-S) 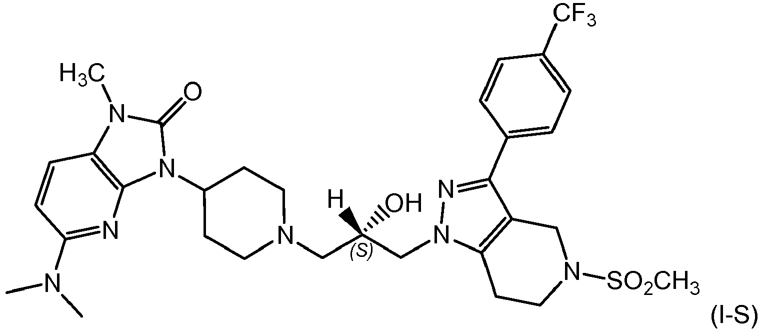 comprising, reacting a compound of formula (I-S) with HCl acid in water.
41. A sulfate salt of a compound of formula (I-S) 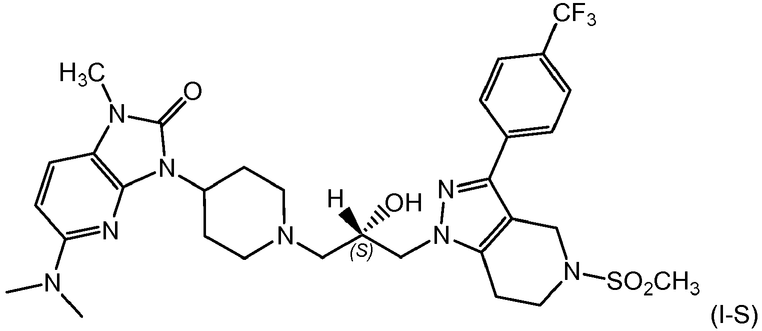
42. An sulfate salt of the compound of formula (I-S) as in item 41 above, wherein the salt is crystalline.
43. A crystalline sulfate salt of a compound of formula (I-S) 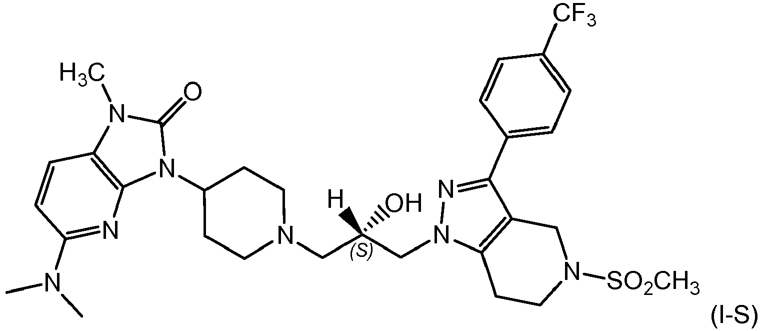 comprising the following X-ray diffraction peaks:

| **Angle (°2θ)** | **d-spacing (Å)** |
|---|---|
| 4.50 | 19.66 |
| 5.56 | 15.91 |
| 6.96 | 12.67 |
| 8.86 | 9.98 |
| 10.82 | 8.18 |
| 11.25 | 7.86 |
| 11.86 | 7.46 |
| 12.33 | 7.18 |
| 14.10 | 6.28 |
| 14.76 | 6.00 |
| 16.11 | 5.50 |
| 17.94 | 4.95 |
| 19.55 | 4.54 |
| 20.88 | 4.25 |
| 22.26 | 3.99 |
| 25.22 | 3.53 |

44. A process for the preparation of a crystalline sulfate salt of a compound of formula (I-S) 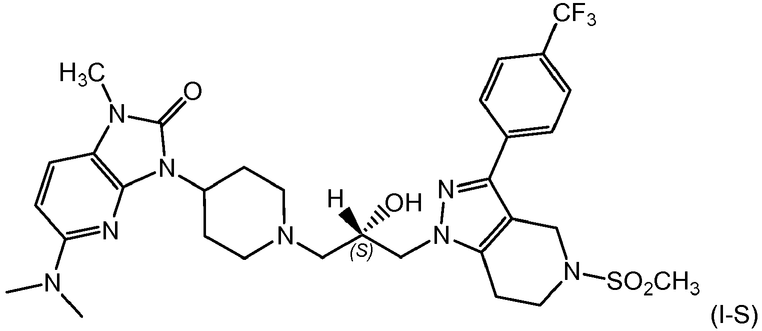 comprising, reacting a compound of formula (I-S) with sulfuric acid.
45. A process for the preparation of a compound of formula (V-A) 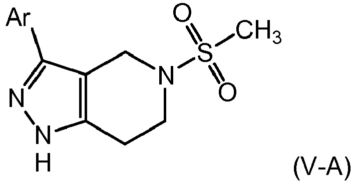 wherein
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl,-C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C₁₋₅heterocyclyl; alternatively, R²² and R²³ can be taken together to form an an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy; comprising 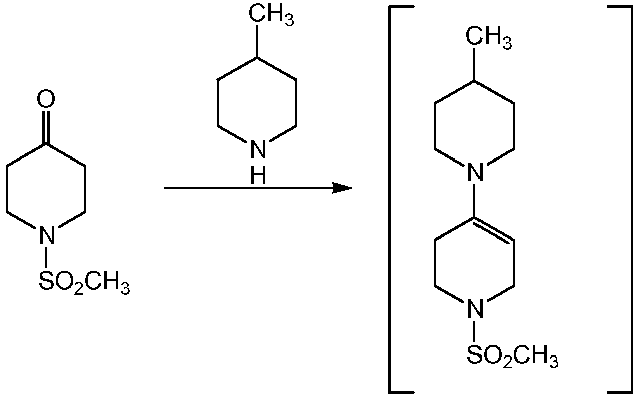 reacting the 1-methanesulfonyl-piperidin-4-one with 4-methyl-piperidine; in the presence of a catalytic amount of an acid; with heating to azeotropically remove any water; to yield the corresponding 1'-methanesulfonyl-4-methyl-3,4,5,6,1',2',3',6'-octahydro-2H-[1,4']bipyridinyl; 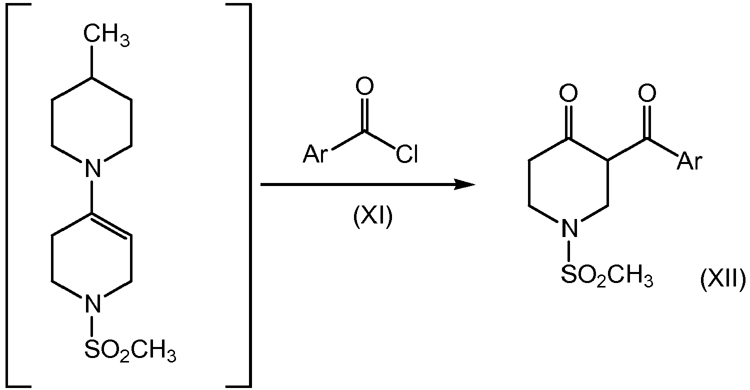 reacting 1'-methanesulfonyl-4-methyl-3,4,5,6, ',2',3',6'-octahydro-2H-[1,4']bipyridinyl with a suitably substituted compound of formula (XI); in the presence of an organic base; in an organic solvent; to yield the corresponding compound of formula (XII); 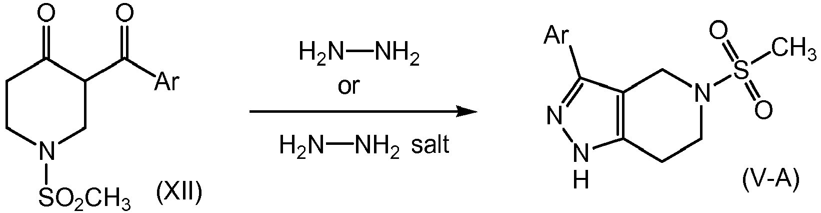 reacting the compound of formula (XII) with hydrazine or its corresponding salt; in the presence of an inorganic or organic base; in a solvent or mixture of solvents; to yield the corresponding compound of formula (V-A).

46. A process for the preparation of a compound of formula (V-S) 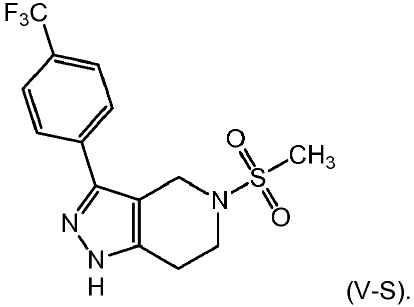 comprising 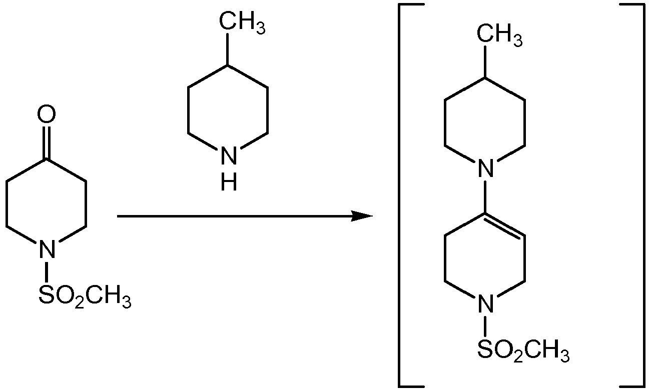 reacting the 1-methanesulfonyl-piperidin-4-one with 4-methyl-piperidine; in the presence of a catalytic amount of an acid; with heating to azeotropically remove any water; to yield the corresponding 1'-methanesulfonyl-4-methyl-3,4,5,6,1',2',3',6'-octahydro-2H-[1,4']bipyridinyl; 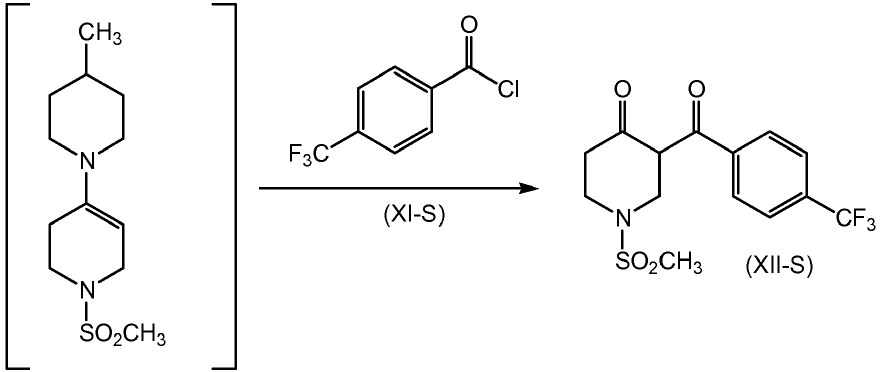 reacting 1'-methanesulfonyl-4-methyl-3,4,5,6, 1',2',3',6'-octahydro-2H-[1,4']bipyridinyl with a suitably substituted compound of formula (XI-S); in the presence of an organic base; in an organic solvent; to yield the corresponding compound of formula (XII-S); 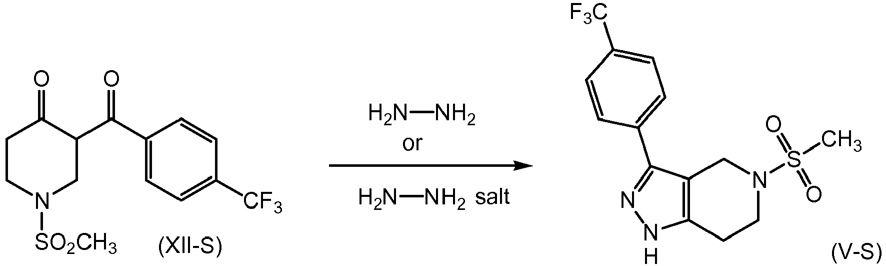 reacting the compound of the compound of formula (XII-S) with hydrazine or its corresponding salt; in the presence of an inorganic or organic base; in a solvent or mixture of solvents; to yield the corresponding compound of formula (V-S).
47. A process for the preparation of a compound of formula (V-A) 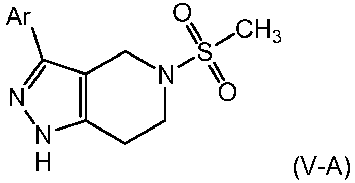 wherein
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl,-C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C₁₋₅heterocyclyl; alternatively, R²² and R²³ can be taken together to form an an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy; comprising 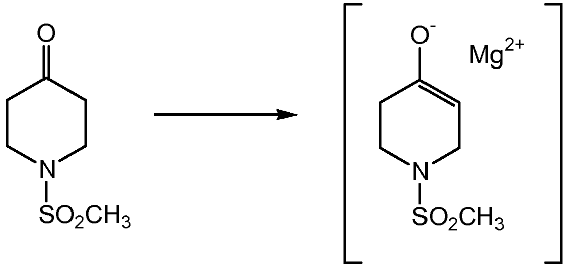 reacting 1-methanesulfonyl-piperidin-4-one with a source of magnesium; in the presence of a base; in an organic solvent; to yield 1-methanesulfonyl-1,2,3,6-tetrahydropyidin-4-olate; 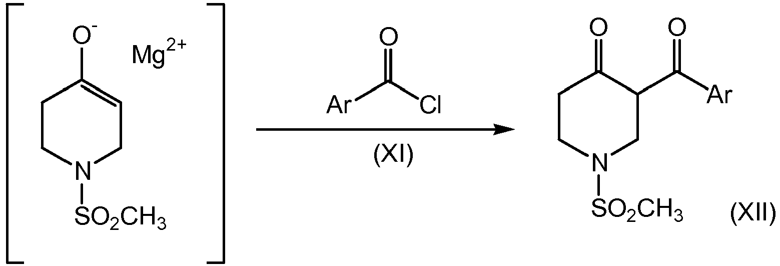 reacting 1-methanesulfonyl-1,2,3,6-tetrahydropyidin-4-olate with a compound of formula (XI); in the presence of an organic base; in an organic solvent; to yield the corresponding compound of formula (XII); 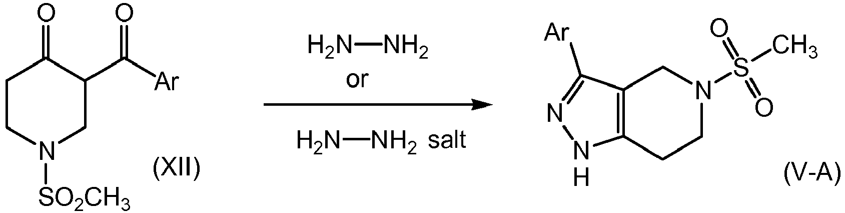 reacting the compound of formula (XII) with hydrazine or its corresponding salt; in the presence of an inorganic or organic base; in a solvent or mixture of solvents; to yield the corresponding compound of formula (V-A).

48. A process for the preparation of a compound of formula (V-S) 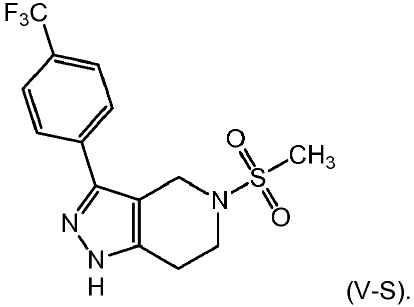 comprising 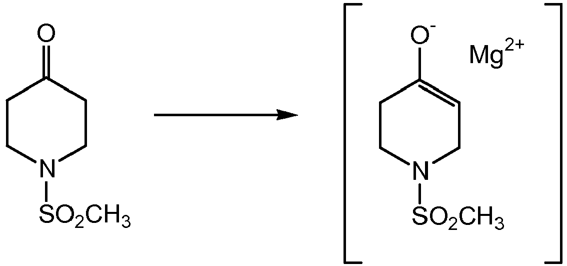 reacting 1-methanesulfonyl-piperidin-4-one with a source of magnesium; in the presence of a base; in an organic solvent; to yield 1-methanesulfonyl-1,2,3,6-tetrahydropyidin-4-olate; 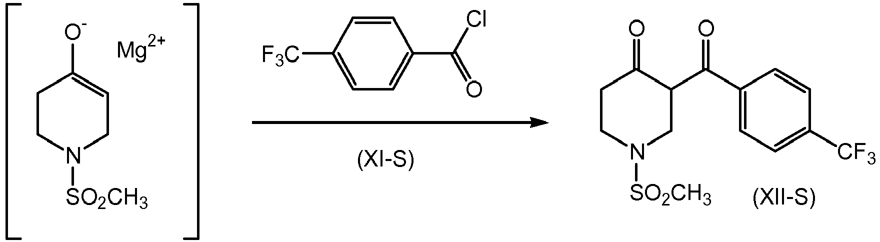 reacting 1-methanesulfonyl-1,2,3,6-tetrahydropyidin-4-olate with a compound of formula (XI-S); in the presence of an organic base; in an organic solvent; to yield the corresponding compound of formula (XII-S); 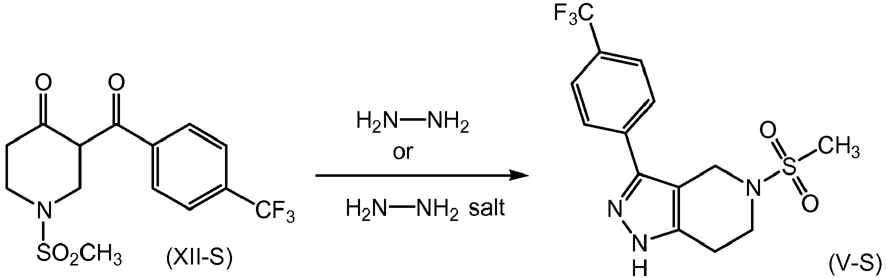 reacting the compound of formula (XII-S) with hydrazine or its corresponding salt; in the presence of an inorganic or organic base; in a solvent or mixture of solvents; to yield the corresponding compound of formula (V-S).
49. A process for the preparation of a compound of formula (V-A) 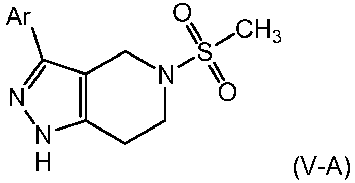 wherein
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl,-C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C₁₋₅heterocyclyl; alternatively, R²² and R²³ can be taken together to form an an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy; comprising 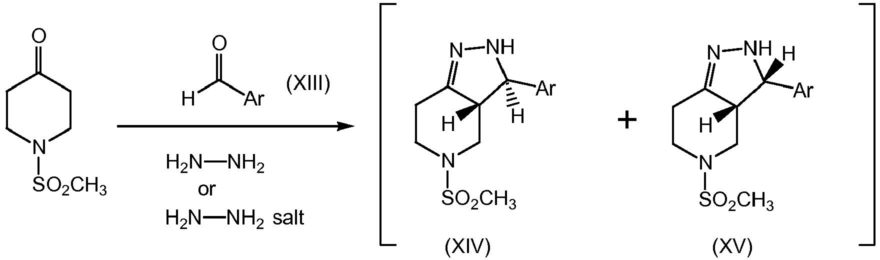 reacting 1-methanesulfonyl-piperidin-4-one with a compound of formula (XIII); in the presence of hydrazine or its corresponding salt; in an organic solvent; to yield a mixture of the corresponding compound of formula (XIV) and the corresponding compound of formula (XV); 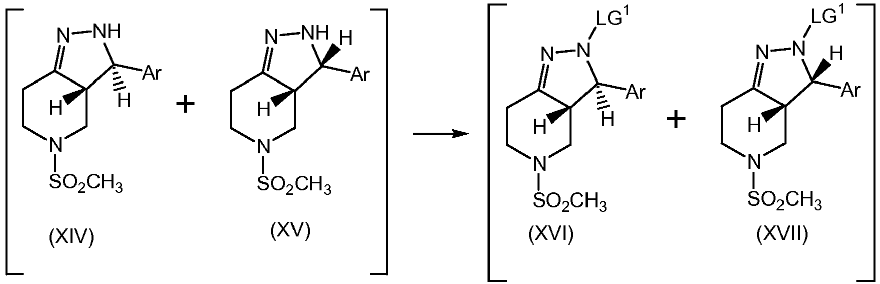 reacting the mixture of the corresponding compound of formula (XIV) and the corresponding compound of formula (XV) with an activating agent; in an organic solvent; to yield a mixture of the corresponding compound of formula (XVI) and the corresponding compound of formula (XVII), wherein LG¹ is the corresponding leaving group; 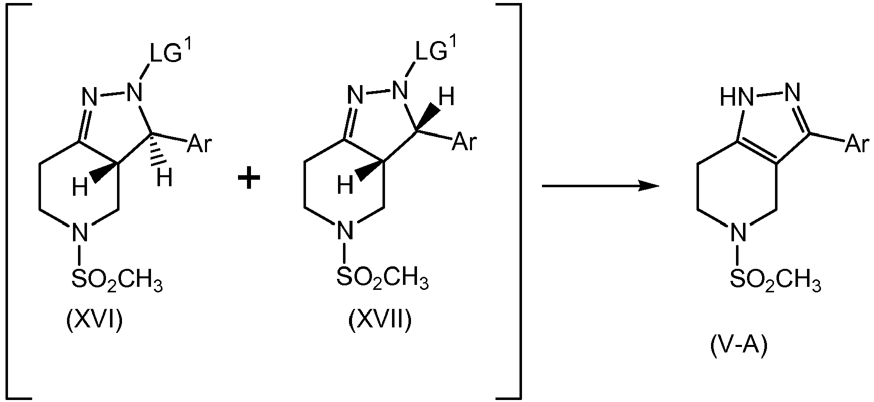 reacting the mixture of the corresponding compound of formula (XVI) and the corresponding compound of formula (XVII); with an organic or inorganic base; in an organic solvent; to yield the corresponding compound of formula (V-A); or
   subject the mixture of the of the corresponding compound of formula (XVI) and the corresponding compound of formula (XVII) to thermal conversion by heating the mixture to a temperature in the range of from about 0°C to about solvent reflux temperature; to yield the corresponding compound of formula (V-A).

50. A process for the preparation of a compound of formula (V-S) 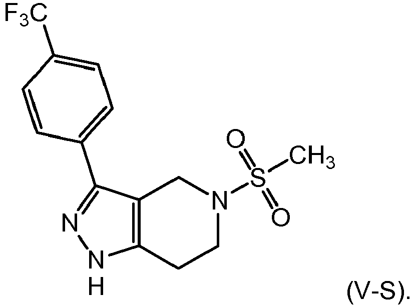 comprising 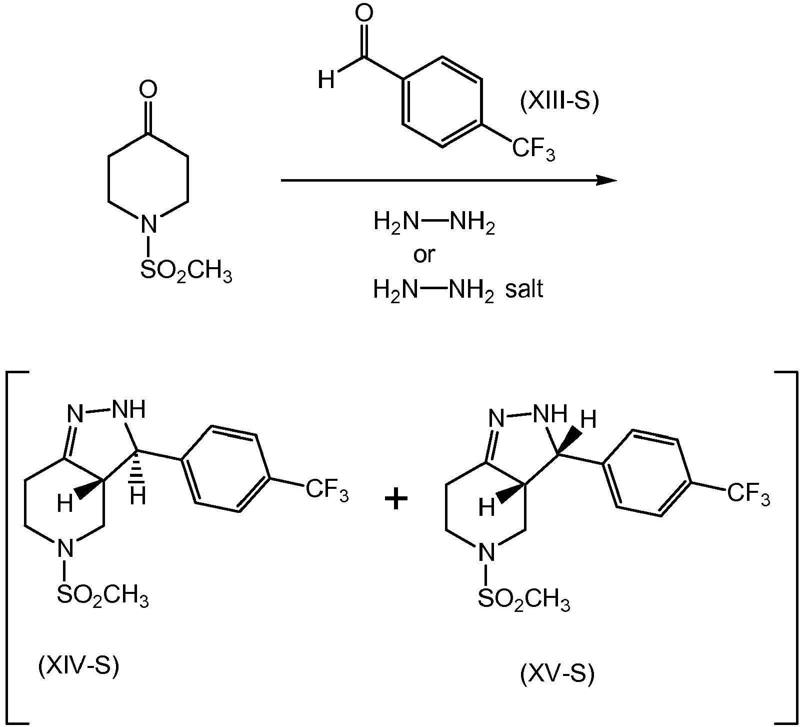 reacting 1-methanesulfonyl-piperidin-4-one with a compound of formula (XIII-S); in the presence of hydrazine or its corresponding salt; in an organic solvent; to yield a mixture of the corresponding compound of formula (XIV-S) and the corresponding compound of formula (XV-S); 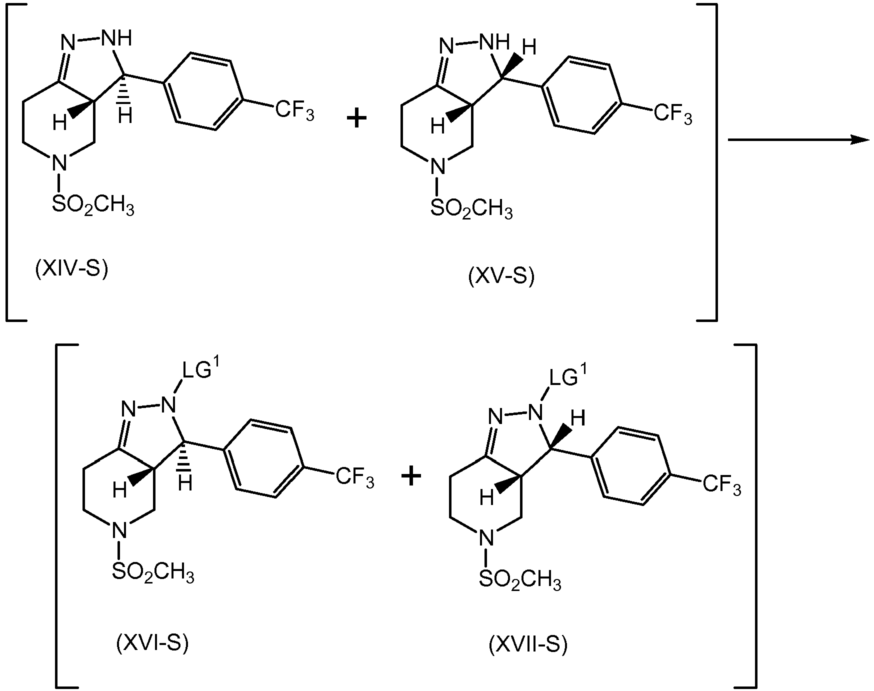 reacting the mixture of the corresponding compound of formula (XIV-S) and the corresponding compound of formula (XV-S) with an activating agent; in an organic solvent; to yield a mixture of the corresponding compound of formula (XVI-S) and the corresponding compound of formula (XVII-S), wherein LG¹ is the corresponding leaving group; 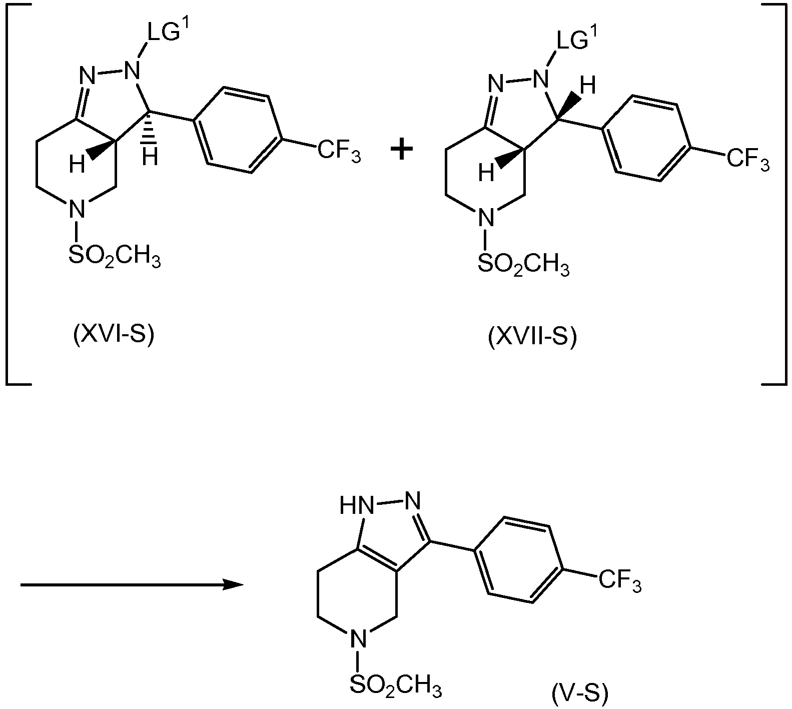 reacting the mixture of the corresponding compound of formula (XVI-S) and the corresponding compound of formula (XVII-S); with an organic or inorganic base; in an organic solvent; to yield the corresponding compound of formula (V-S); or
subject the mixture of the of the corresponding compound of formula (XVI-S) and the corresponding compound of formula (XVII-S) to thermal conversion by heating the mixture to a temperature in the range of from about 0°C to about solvent reflux temperature; to yield the corresponding compound of formula (V-S).
51. A process as in item 50 above, wherein the activating agent is Br₂ and wherein LG¹ is Br.
52. A process for the preparation of a compound of formula (V-A) 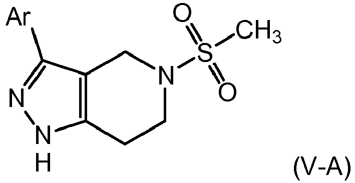 wherein
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl,-C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C₁₋₅heterocyclyl; alternatively, R²² and R²³ can be taken together to form an an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy; comprising 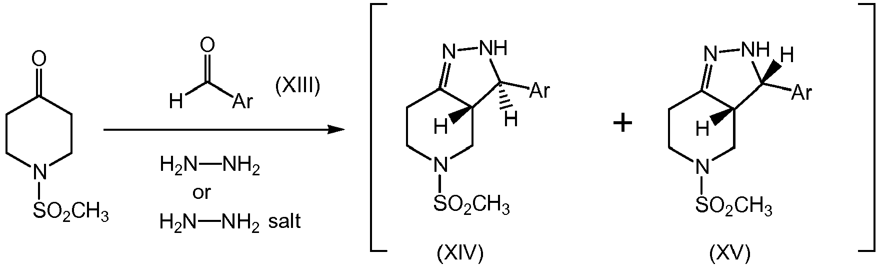 reacting 1-methanesulfonyl-piperidin-4-one with a compound of formula (XIII); in the presence of hydrazine or its corresponding salt; in an organic solvent; to yield a mixture of the corresponding compound of formula (XIV) and the corresponding compound of formula (XV); 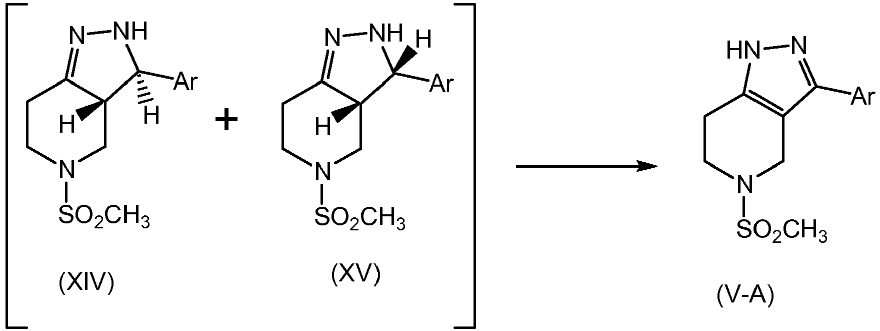 reacting the mixture of the corresponding compound of formula (XIV) and the corresponding compound of formula (XV) with an oxidizing agent; in an organic solvent; followed by thermal conversion at a temperature in the range of from about 0°C to about solvent reflux temperature; to yield the corresponding compound of formula (V-A); or
   reacting the mixture of the corresponding compound of formula (XIV) and the corresponding compound of formula (XV) with an oxidizing agent; in an organic solvent; followed by treatment with an organic or inorganic base; in an organic solvent; to yield the corresponding compound of formula (V-A).

53. A process for the preparation of a compound of formula (V-S) 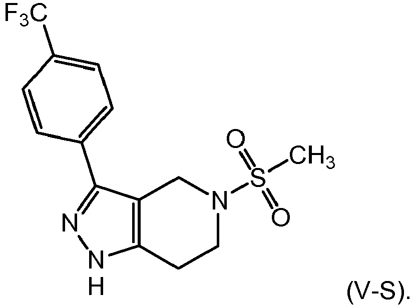 comprising 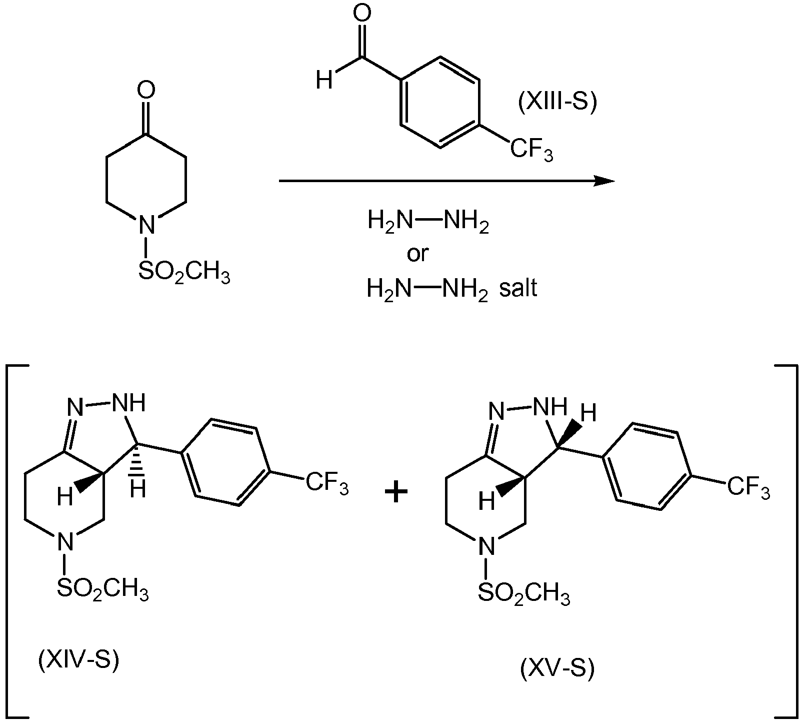 reacting 1-methanesulfonyl-piperidin-4-one with a compound of formula (XIII-S); in the presence of hydrazine or its corresponding salt; in an organic solvent; to yield a mixture of the corresponding compound of formula (XIV-S) and the corresponding compound of formula (XV-S); 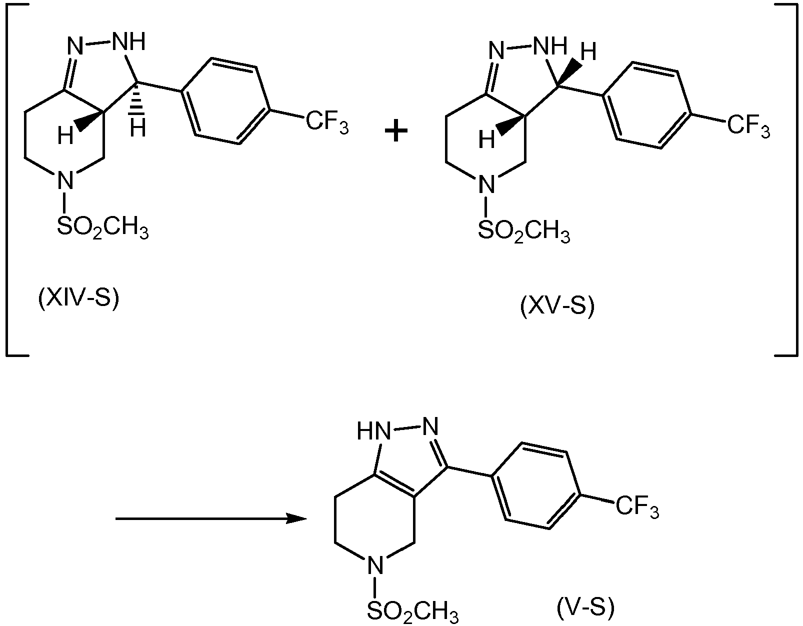 reacting the mixture of the corresponding compound of formula (XIV-S) and the corresponding compound of formula (XV-S) with an oxidizing agent; in an organic solvent; followed by thermal conversion at a temperature in the range of from about 0°C to about solvent reflux temperature; to yield the corresponding compound of formula (V-S); or
reacting the mixture of the corresponding compound of formula (XIV-S) and the corresponding compound of formula (XV-S) with an oxidizing agent; in an organic solvent; followed by treatment with an organic or inorganic base; in an organic solvent; to yield the corresponding compound of formula (V-S).
54. A process for the preparation of a compound of formula (X-A) 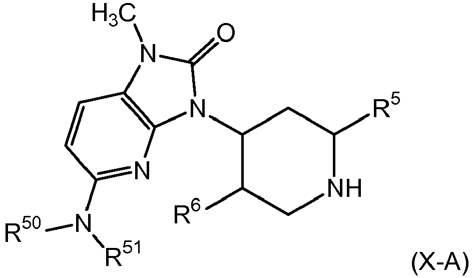 wherein
R⁵ and R⁶ are each independently selected from the group consisting of hydrogen and C₁₋₅alkyl;
R⁵⁰ and R⁵¹ are each independently selected from the group consisting of hydrogen and C¹⁻⁴alkyl; comprising 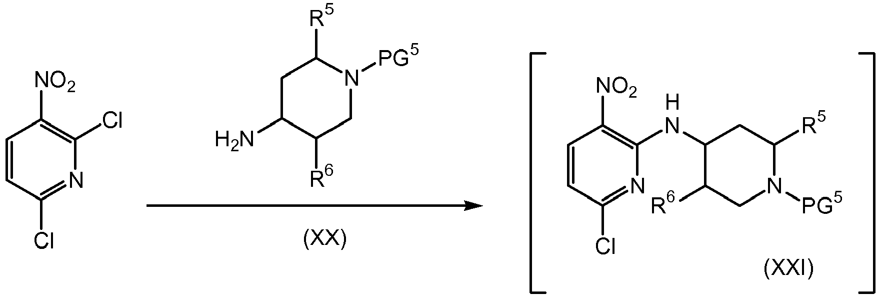 reacting 2,6-dichloro-3-nitro-pyridine with a compound of formula (XX), wherein PG⁵ is a nitrogen protecting group; in the presence of an organic or inorganic base; in an organic solvent; to yield the corresponding compound of formula (XXI); 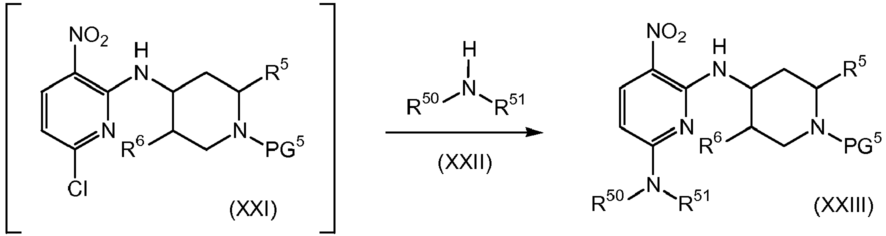 reacting the compound of formula (XXI) with a compound of formula (XXII); in an organic solvent; to yield the corresponding compound of formula (XXIII); 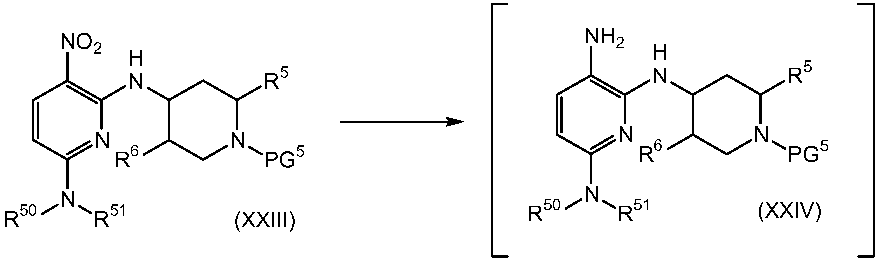 reacting the compound of formula (XXIII) with a reducing agent; in the presence of a catalyst; in an organic solvent; to yield the corresponding compound of formula (XXIV); 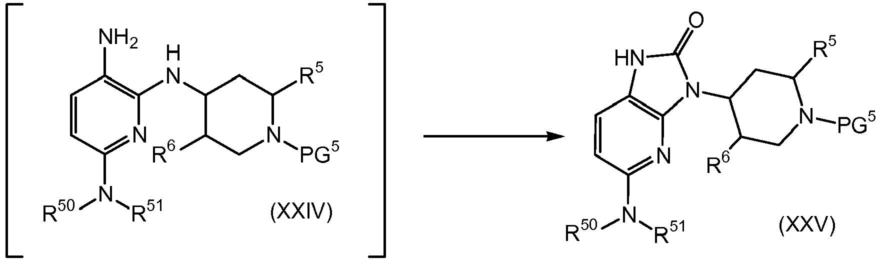 reacting the compound of formula (XXIV) with a reagent selected from the group consisting of CDI, N-methylcarbodiimide, phosgene and triphosgene; in an organic solvent; to yield the corresponding compound of formula (XXV); 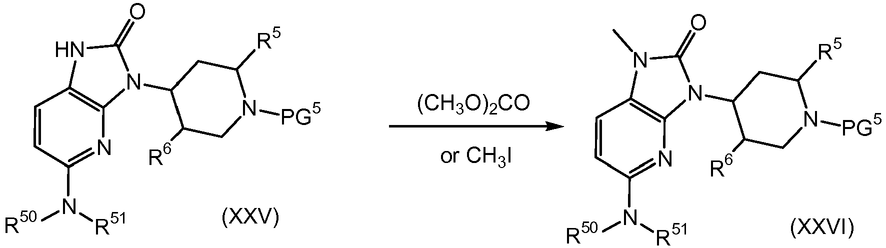 reacting the compound of formula (XXV) with carbonic acid dimethyl ester or CH₃I; in the presence of an inorganic base; in an organic solvent; to yield the corresponding compound of formula (XXVI); 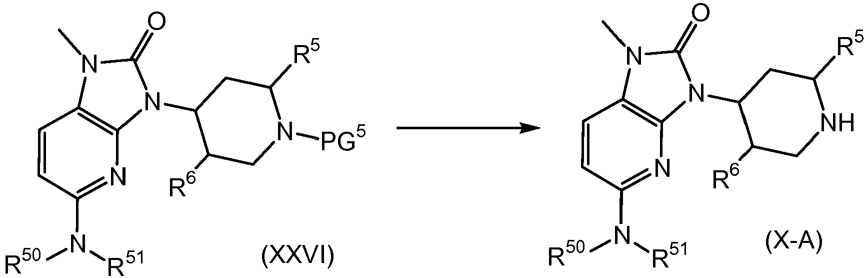 de-protecting the compound of formula (XXVI); to yield the corresponding compound of formula (X-A).

55. A process for the preparation of a compound of formula (X-S) 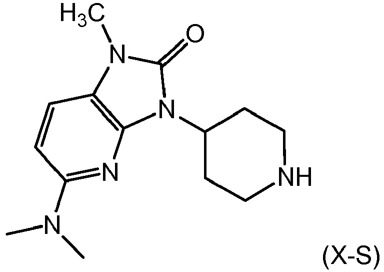 comprising 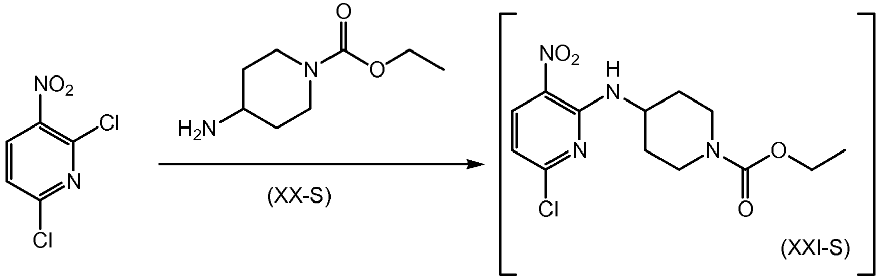 reacting 2,6-dichloro-3-nitro-pyridine with a compound of formula (XX-S), wherein PG⁵ is a nitrogen protecting group; in the presence of an organic or inorganic base; in an organic solvent; to yield the corresponding compound of formula (XXI-S); 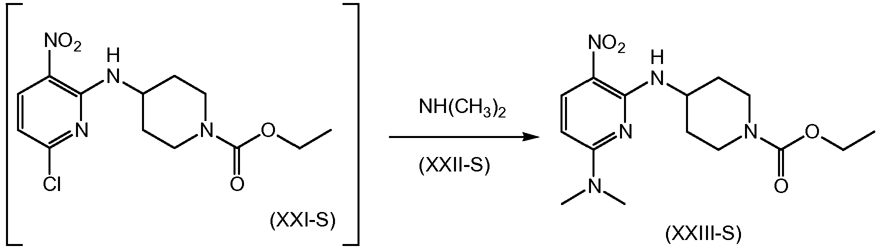 reacting the compound of formula (XXI-S) with a compound of formula (XXII-S); in an organic solvent; to yield the corresponding compound of formula (XXIII-S); 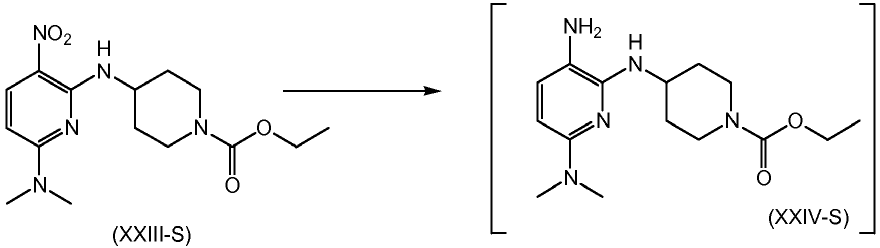 reacting the compound of formula (XXIII-S) with a reducing agent; in the presence of a catalyst; in an organic solvent; to yield the corresponding compound of formula (XXIV-S); 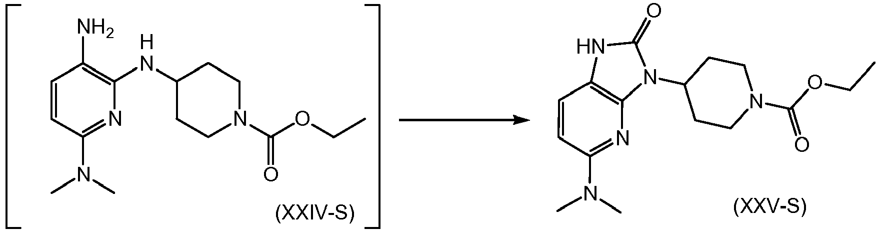 reacting the compound of formula (XXIV-S) with a reagent selected from the group consisting of CDI, N-methylcarbodiimide, phosgene and triphosgene; in an organic solvent; to yield the corresponding compound of formula (XXV-S); 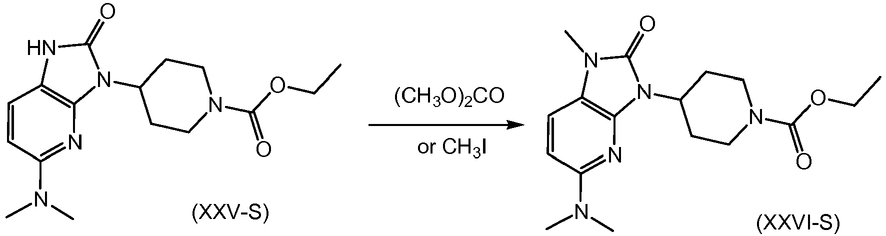 reacting the compound of formula (XXV-S) with carbonic acid dimethyl ester or CH₃I; in the presence of an inorganic base; in an organic solvent; to yield the corresponding compound of formula (XXVI-S); 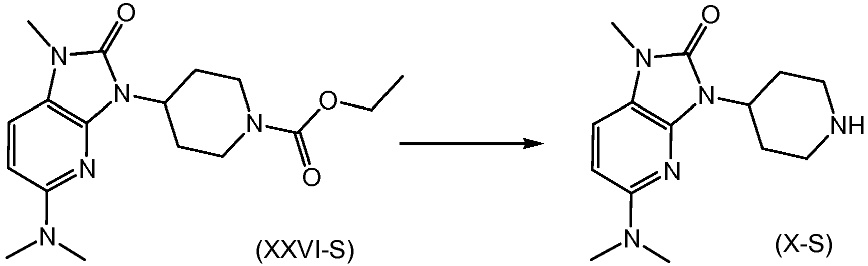 de-protecting the compound of formula (XXVI-S); to yield the corresponding compound of formula (X-S).
56. A process for the preparation of a compound of formula (VI-A) 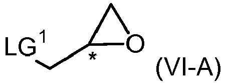 wherein LG¹ is selected from the group consisting of 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl and 3-chloro-benzenesulfonyl; comprising 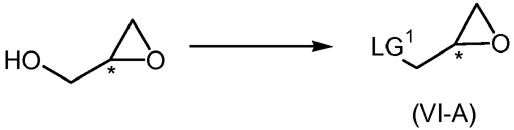 reacting oxiranyl-methanol; wherein the oxiranyl-methanol is present as a racemate or in an enantiomeric excess of one of its corresponding enantiomers; with an activating reagent; to yield the compound of formula (VIA); wherein LG¹ is the corresponding leaving group from the activating reagent.
57. A process as in item 56 above, wherein the activating reagent is 4-nitrophenyl-sulfonyl chloride and wherein the corresponding leaving group LG¹ is 4-nitrophenyl-sulfonyl; or wherein the activating reagent is 3-nitrophenyl-sulfonyl chloride and wherein the corresponding leaving group LG¹ is 3-nitrophenyl-sulfonyl.
58. A process as in item 56 above, wherein the compound of formula (VI-A) is prepared in an enantiomeric excess of its corresponding (S) enantiomer.
59. A process as in item 56 above, wherein the compound of formula (VI-A) is prepared in an enantiomeric excess of its corresponding (R) enantiomer.
60. A process for the preparation of a compound of formula (VI-P) 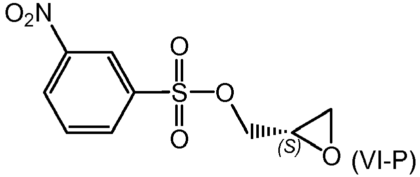 comprising 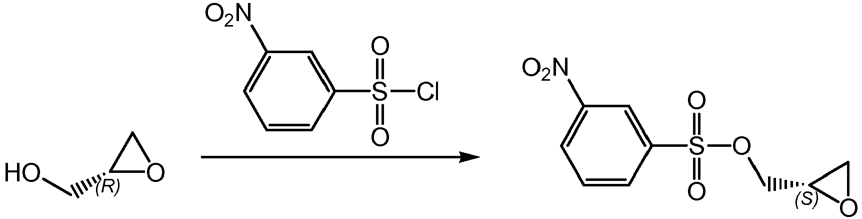 reacting (R)-glycidol with 3-nitrophenyl-sulfonyl chloride; in the presence of an organic or inorganic base; in an organic solvent; to yield the corresponding 3-nitro-benzenesulfonic acid (S)-oxiranylmethyl ester.

The following terms are defined below and by their usage throughout this disclosure.

**"Alkyl"** includes optionally substituted straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl includes cycloalkyl, such as cyclopropyl, cyclobutyl, cyclopentyl, and cyclohexyl.

"Alkenyl" includes optionally substituted straight chain and branched hydrocarbon radicals as above with at least one carbon-carbon double bond (sp²). Alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on. Hydrocarbon radicals having a mixture of double bonds and triple bonds, such as 2-penten-4-ynyl, are grouped as alkynyls herein. Alkenyl includes cycloalkenyl. *Cis* and *trans* or (E) and (Z) forms are included within the invention.

"Alkynyl" includes optionally substituted straight chain and branched hydrocarbon radicals as above with at least one carbon-carbon triple bond (sp). Alkynyls include ethynyl, propynyls, butynyls, and pentynyls. Hydrocarbon radicals having a mixture of double bonds and triple bonds, such as 2-penten-4-ynyl, are grouped as alkynyls herein. Alkynyl does not include cycloalkynyl.

**"Alkoxy"** includes an optionally substituted straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and SO₂. Heteroalkyl includes alkoxy, aminoalkyl, thioalkyl, and so on.

**"Aryl"** includes phenyl, naphthyl, biphenylyl, tetrahydronaphthyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on.

**"Heterocyclyl"** includes optionally substituted aromatic and nonaromatic rings having carbon atoms and at least one heteroatom (O, S, N) or heteroatom moiety (SO₂, CO, CONH, COO) in the ring. Unless otherwise indicated, a heterocyclic radical may have a valence connecting it to the rest of the molecule through a carbon atom, such as 3-furyl or 2-imidazolyl, or through a heteroatom, such as N-piperidyl or 1-pyrazolyl. For example a monocyclic heterocyclyl has between 4 and 7 ring atoms, or between 5 and 6 ring atoms; there may be between 1 and 5 heteroatoms or heteroatom moieties in the ring, and for example between 1 and 3. A heterocyclyl may be saturated, unsaturated, aromatic (e.g., heteroaryl), nonaromatic, or fused.

Heterocyclyl also includes fused, e.g., bicyclic, rings, such as those optionally condensed with an optionally substituted carbocyclic or heterocyclic five- or six-membered aromatic ring. For example, "heteroaryl" includes an optionally substituted six-membered heteroaromatic ring containing 1, 2 or 3 nitrogen atoms condensed with an optionally substituted five- or six-memebered carbocyclic or heterocyclic aromatic ring. Said heterocyclic five- or six-membered aromatic ring condensed with the said five- or six-membered aromatic ring may contain 1, 2 or 3 nitrogen atoms where it is a six-membered ring, or 1, 2 or 3 heteroatoms selected from oxygen, nitrogen and sulfur where it is a five-membered ring.

Examples of heterocyclyls include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imdazolidinyl, imidazolinyl, pyrazolidinyl, pyrazolinyl, piperidyl, piperazinyl, indolinyl, and morpholinyl. For example, heterocyclyls or heterocyclic radicals include morpholinyl, piperazinyl, pyrrolidinyl, pyridyl, cyclohexylimino, cycloheptylimino,and more for example, piperidyl.

Examples illustrating heteroaryl are thienyl, furanyl, pyrrolyl, imidazolyl, oxazolyl, thiazolyl, benzothienyl, benzofuranyl, benzimidazolyl, benzoxazolyl, benzothiazolyl.

**"Acyl"** refers to a carbonyl moiety attached to either a hydrogen atom (i.e., a formyl group) or to an optionally substituted alkyl or alkenyl chain, or heterocyclyl.

**"Halo"** or **"halogen"** includes fluoro, chloro, bromo, and iodo, and for example chloro or bromo as a substituent.

**"Alkanediyl"** or **"alkylene"** represents straight or branched chain optionally substituted bivalent alkane radicals such as, for example, methylene, ethylene, propylene, butylene, pentylene or hexylene.

**"Alkenediyl"** represents, analogous to the above, straight or branched chain optionally substituted bivalent alkene radicals such as, for example, propenylene, butenylene, pentenylene or hexenylene. In such radicals, the carbon atom linking a nitrogen for example should not be unsaturated.

**"Aroyl"** refers to a carbonyl moiety attached to an optionally substituted aryl or heteroaryl group, wherein aryl and heteroaryl have the definitions provided above. In particular, benzoyl is phenylcarbonyl.

As defined herein, two radicals, together with the atom(s) to which they are attached may form an optionally substituted 4- to 7-, 5 - to 7-, or a 5- to 6-membered ring carbocyclic or heterocyclic ring, which ring may be saturated, unsaturated or aromatic. Said rings may be as defined above in the Summary of the Invention section. Particular examples of such rings are as follows in the next section.

**"Pharmaceutically acceptable salts, esters, and amides"** include carboxylate salts (e.g., C ₁₋₈ alkyl, cycloalkyl, aryl, heteroaryl, or non-aromatic heterocyclic) amino acid addition salts, esters, and amides which are within a reasonable benefit/risk ratio, pharmacologically effective and suitable for contact with the tissues of patients without undue toxicity, irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19 which is incorporated herein by reference. Representative pharmaceutically acceptable amides of the invention include those derived from ammonia, primary C ₁₋₆ alkyl amines and secondary di (C ₁₋₆ alkyl) amines. Secondary amines include 5- or 6-membered heterocyclic or heteroaromatic ring moieties containing at least one nitrogen atom and optionally between 1 and 2 additional heteroatoms. Exemplary amides are derived from ammonia, C ₁₋₃ alkyle primary amines, and di (C ₁₋₂ alkyl)amines. Representative pharmaceutically acceptable esters of the invention include C ₁₋₇ alkyl, C ₅₋₇ cycloalkyl, phenyl, and phenyl(C ₁₋₆)alkyl, esters. For example, esters include methyl esters.

**"Patient"** or **"subject"** includes mammals such as humans and animals (dogs, cats, horses, rats, rabbits, mice, non-human primates) in need of observation, experiment, treatment or prevention in connection with the relevant disease or condition. For example, the patient or subject is a human.

**"Composition"** includes a product comprising the specified ingredients in the specified amounts as well as any product which results directly or indirectly from combinations of the specified ingredients in the specified amounts.

**"Therapeutically effective amount"** or **"effective amount"** means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Concerning the various radicals in this disclosure and in the claims, three general remarks are made. The first remark concerns valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent).

Second, radicals or structure fragments as defined herein are understood to include substituted radicals or structure fragments. Hydrocarbyls include monovalent radicals containing carbon and hydrogen such as alkyl, alkenyl, alkynyl, cycloalkyl, and cycloalkenyl (whether aromatic or unsaturated), as well as corresponding divalent radicals such as alkylene, alkenylene, phenylene, and so on. Heterocarbyls include monovalent and divalent radicals containing carbon, hydrogen, and at least one heteroatom. Examples of monovalent heterocarbyls include acyl, acyloxy, alkoxyacyl, heterocyclyl, heteroaryl, aroyl, benzoyl, dialkylamino, hydroxyalkyl, and so on.

Using **"alkyl"** as an example, **"alkyl"** should be understood to include substituted alkyl having one or more substitutions, such as between 1 and 5, 1 and 3, or 2 and 4 substituents. The substituents may be the same (dihydroxy, dimethyl), similar (chlorofluoro), or different (chlorobenzyl- or aminomethyl-substituted). Examples of substituted alkyl include haloalkyl (such as fluoromethyl, chloromethyl, difluoromethyl, perchloromethyl, 2-bromoethyl, perfluoromethyl, and 3-iodocyclopentyl), hydroxyalkyl (such as hydroxymethyl, hydroxyethyl, 2-hydroxypropyl, aminoalkyl (such as aminomethyl, 2-aminoethyl, 3-aminopropyl, and 2-aminopropyl), nitroalkyl, alkylalkyl, and so on. A di(C ₁₋₆ alkyl)amino group includes independently selected alkyl groups, to form, for example, methylpropylamino and isopropylmethylamino, in addition dialkylamino groups having two of the same alkyl group such as dimethyl amino or diethylamino.

Third, only stable compounds are intended. For example, where there is an NR'R" group, and R can be an alkenyl group, the double bond is at least one carbon removed from the nitrogen to avoid enamine formation. Similarly, where a dashed line is an optional sp² bond, if it is absent, the appropriate hydrogen atom(s) is(are) included.

In an example, substitutions for Ar or Ar₁ include methyl, methoxy, fluoromethyl, difluoromethyl, perfluoromethyl (trifluoromethyl), 1-fluoroethyl, 2-fluoroethyl, ethoxy, fluoro, chloro, and bromo, and particularly methyl, bromo, chloro, perfluoromethyl, perfluoromethoxy, methoxy, and fluoro. In another example, substitution patterns for Ar or Ar₁ are 4-substituted or 3,4-disubstituted phenyl.

Reference to a chemical entity herein by naming one of its forms stands for a reference to any one of: (a) the actually recited form of such chemical entity, and (b) any of the forms of such chemical entity in the medium in which the compound is being considered when named. For example, reference herein to a compound such as R-COOH, encompasses reference to any one of, for example, R-COOH₍ₛ₎, R-COOH(ₛₒₗ), and R-COO⁻₍ₛₒₗ₎. In this example, R-COOH₍ₛ₎ refers to the solid compound, as it could be for example in a tablet or some other solid pharmaceutical composition or preparation; R-COOH(ₛₒₗ) refers to the undissociated form of the compound in a solvent; and R-COO⁻(ₛₒₗ) refers to the dissociated form of the compound in a solvent, such as the dissociated form of the compound in an aqueous environment, whether such dissociated form derives from R-COOH, from a salt thereof, or from any other entity that yields R-COO- upon dissociation in the medium being considered. In another example, an expression such as "exposing an entity to compound of formula R-COOH" refers to the exposure of such entity to the form, or forms, of the compound R-COOH that exists, or exist, in the medium in which such exposure takes place. In still another example, an expression such as "reacting an entity with a compound of formula R-COOH" refers to the reacting of (a) such entity in the chemically relevant form, or forms, of such entity that exists, or exist, in the medium in which such reacting takes place, with (b) the chemically relevant form, or forms, of the compound R-COOH that exists, or exist, in the medium in which such reacting takes place. In this regard, if such entity is for example in an aqueous environment, it is understood that the compound R-COOH is in such same medium, and therefore the entity is being exposed to species such as R-COOH_{(aq)} and/or R-COO⁻_{(aq)}, where the subscript "(aq)" stands for "aqueous" according to its conventional meaning in chemistry and biochemistry. A carboxylic acid functional group has been chosen in these nomenclature examples; this choice is not intended, however, as a limitation but it is merely an illustration. It is understood that analogous examples can be provided in terms of other functional groups, including but not limited to hydroxyl, basic nitrogen members, such as those in amines, and any other group that interacts or transforms according to known manners in the medium that contains the compound. Such interactions and transformations include, but are not limited to, dissociation, association, tautomerism, solvolysis, including hydrolysis, solvation, including hydration, protonation, and deprotonation. In another example, a zwitterionic compound is encompassed herein by referring to a compound that is known to form a zwitterions, even if it is not explicitly named in its zwitterionic form. Terms such as zwitterion, zwitterions, and their synonyms zwitterionic compound(s) are standard IUPAC-endorsed names that are well known and part of standard sets of defined scientific names. In this regard, the name zwitterion is assigned the name identification CHEBI:27369 by the Chemical Entities of Biological Inerest (ChEBI) dictionary of molecular entities. (See, for example its on line version at http://www.ebi.ac.uk/chebi/init.do). As generally well known, a zwitterion or zwitterionic compound is a neutral compound that has formal unit charges of opposite sign. Sometimes these compounds are referred to by the term "inner salts". Other sources refer to these compounds as "dipolar ions", although the latter term is regarded by still other sources as a misnomer. As a specific example, aminoethanoic acid (the amino acid glycine) has the formula H₂NCH₂COOH, and it exists in some media (in this case in neutral media) in the form of the zwitterion ⁺H₃NCH₂COO⁻. Zwitterions, zwitterionic compounds, inner salts and dipolar ions in the known and well established meanings of these terms are within the scope of this invention, as would in any case be so appreciated by those of ordinary skill in the art. Because there is no need to name each and every embodiment that would be recognized by those of ordinary skill in the art, no structures of the zwitterionic compounds that are associated with the compounds of this invention are given explicitly herein. They are, however, part of the embodiments of this invention. No further examples in this regard are provided herein because the interactions and transformations in a given medium that lead to the various forms of a given compound are known by any one of ordinary skill in the art.

Abbreviations used in the specification, particularly the Schemes and Examples, are as follows:

| | | |
|---|---|---|
| AL(OTf)₃ | = | Aluminum triflate |
| CDI | = | Carbonyldiimidazole |
| Chloranil | = | 2,3,5,6-Tetrachloro-2,5-cyclohexadiene-1,4-dione |
| DCE | = | 1,1-Dichloroethane |
| DCM | = | Dichloromethane |
| DDQ | = | 2,3-Dichloro-5,6-dicyanobenzoquinone |
| DIPEA or DIEA | = | Diisopropylethylamine |
| DMA or DMAc | = | N,N-Dimethylacetamide |
| DME | = | 1,2-Dimethoxyethane |
| DMF | = | N,N-Dimethylformamide |
| DMSO | = | Dimethylsulfoxide |
| Et₂O | = | Diethyl ether |
| EtOAc | = | Ethyl acetate |
| EtOH | = | Ethanol |
| HPLC | = | High Pressure Liquid Chromatography |
| IPA | = | Isopropyl alcohol (Isopropanol) |
| KHMDS | = | Potassium bis(trimethylsilyl)amide |
| KOt-Bu | = | Potassium t-butoxide |
| MeOH | = | Methanol |
| MTBE | = | Methyl t-Butyl Ether |
| NaHMDS | = | Sodium bis(trimethylsilyl)amide |
| NaOt-Bu | = | Sodium t-Butoxide |
| Pd/C | = | Palladium on carbon (catalyst) |
| Phosgene | = | Carbonyl Chloride (i.e Cl₂CO) |
| Pt/C | = | Platinum on carbon (catalyst) |
| p-TsOH | = | p-Toluenesulfonic Acid |
| t-BOC or Boc | = | Tert-Butoxycarbonyl |
| TEA | = | Triethylamine |
| TFA | = | Trifluoroacetic Acid |
| THF | = | Tetrahydrofuran |
| TMS | = | Trimethylsilane |
| Ti(O-i-Pr)₄ | = | Titanium propoxide |
| TMS | = | Trimethylsilane |
| Triphosgene | = | Bis(trichloromethyl)carbonate |
| XRD | = | X-ray Diffration |
| Yb(OTf)₃ | = | Ytterbium Triflate |

As used herein, the notation "*" shall denote the presence of a stereogenic center.

Where the compounds according to this invention have at least one **chiral center,** they may accordingly exist as enantiomers. Where the compounds possess two or more chiral centers, they may additionally exist as diastereomers. It is to be understood that all such isomers and mixtures thereof are encompassed within the scope of the present invention. In an embodiment, wherein the compound is present as an enantiomer, the enantiomer is present at an enantiomeric excess of greater than or equal to about 80%, for example, at an enantiomeric excess of greater than or equal to about 90%, for example, at an enantiomeric excess of greater than or equal to about 95%, for example, at an enantiomeric excess of greater than or equal to about 98%, for example, at an enantiomeric excess of greater than or equal to about 99%. Similarly, wherein the compound is present as a diastereomer, the diastereomer is present at an diastereomeric excess of greater than or equal to about 80%, for example, at an diastereomeric excess of greater than or equal to about 90%, for example, at an diastereomeric excess of greater than or equal to about 95%, for example, at an diastereomeric excess of greater than or equal to about 98%, for example, at an diastereomeric excess of greater than or equal to about 99%.

Furthermore, some of the crystalline forms for the compounds of the present invention may exist as polymorphs and as such are intended to be included in the present invention. In addition, some of the compounds of the present invention may form solvates with water (i.e., hydrates) or common organic solvents, and such solvates are also intended to be encompassed within the scope of this invention.

Furthermore, some of the crystalline forms for the compounds of the present invention may exist as polymorphs and as such are intended to be included in the present invention. In addition, some of the compounds of the present invention may form solvates with water (i.e., hydrates) or common organic solvents, and such solvates are also intended to be encompassed within the scope of this invention.

As used herein, unless otherwise noted, the term **"isolated form"** shall mean that the compound is present in a form which is separate from any solid mixture with another compound(s), solvent system or biological environment. In an embodiment, the present invention is directed to a process for the preparation of a compound of formula (I), wherein the compound of formula (I) is prepared as an isolated form. In another embodiment, the present invention is directed to a process for the preparation of a compound of formula (I-S), wherein the compound of formula (I-S) is prepared as an isolated form.

As used herein, unless otherwise noted, the term **"substantially pure form"** shall mean that the mole percent of impurities in the isolated compound is less than about 5 mole percent, for example less than about 2 mole percent, more for example, less than about 0.5 mole percent, most for example, less than about 0.1 mole percent. In an embodiment, the present invention is directed to a process for the preparation of a compound of formula (I), wherein the compound of formula (I) is prepared as a substantially pure form. In another embodiment, the present invention is directed to a process for the preparation of a compound of formula (I-S), wherein the compound of formula (I-S) is prepared as a substantially pure form.

As used herein, unless otherwise noted, the term **"substantially free of a corresponding salt form(s)"** when used to described the compound of formula (I) shall mean that mole percent of the corresponding salt form(s) in the isolated base of formula (I) is less than about 5 mole percent, for example less than about 2 mole percent, more for example, less than about 0.5 mole percent, most for example less than about 0.1 mole percent. In an embodiment, the present invention is directed to a process for the preparation of a compound of formula (I), wherein the compound of formula (I) is prepared as form which is substantially free of corresponding salt form(s). In another embodiment, the present invention is directed to a process for the preparation of a compound of formula (I-S), wherein the compound of formula (I-S) is prepared as form which is substantially free of corresponding salt form(s).

As more extensively provided in this written description, terms such as **"reacting"** and **"reacted"** are used herein in reference to a chemical entity that is any one of: (a) the actually recited form of such chemical entity, and (b) any of the forms of such chemical entity in the medium in which the compound is being considered when named.

One skilled in the art will recognize that, where not otherwise specified, the reaction step(s) is performed under suitable conditions, according to known methods, to provide the desired product. One skilled in the art will further recognize that, in the specification and claims as presented herein, wherein a reagent or reagent class/type (e.g. base, solvent, etc.) is recited in more than one step of a process, the individual reagents are independently selected for each reaction step and may be the same of different from each other. For example wherein two steps of a process recite an organic or inorganic base as a reagent, the organic or inorganic base selected for the first step may be the same or different than the organic or inorganic base of the second step. Further, one skilled in the art will recognize that wherein a reaction step of the present invention may be carried out in a variety of solvents or solvent systems, said reaction step may also be carried out in a mixture of the suitable solvents or solvent systems. One skilled in the art will further recognize that wherein two consecutive reaction or process steps are run without isolation of the intermediate product (i.e. the product of the first of the two consecutive reaction or process steps), then the first and second reaction or process steps may be run in the same solvent or solvent system; or alternatively may be run in different solvents or solvent systems following solvent exchange, which may be completed according to known methods.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any range therein.

Examples of suitable solvents, bases, reaction temperatures, and other reaction parameters and components are provided in the detailed descriptions which follows herein. One skilled in the art will recognize that the listing of said examples is not intended, and should not be construed, as limiting in any way the invention set forth in the claims which follow thereafter.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

As used herein, unless otherwise noted, the term **"nitrogen protecting group"** shall mean a group which may be attached to a nitrogen atom to protect said nitrogen atom from participating in a reaction and which may be readily removed following the reaction. Suitable nitrogen protecting groups include, but are not limited to carbamates - groups of the formula -C(O)O-R wherein R is for example methyl, ethyl, t-butyl, benzyl, phenylethyl, and CH₂=CH-CH₂-; amides - groups of the formula -C(O)-R' wherein R' is for example methyl, phenyl, and trifluoromethyl; N-sulfonyl derivatives - groups of the formula -SO₂-R" wherein R" is for example tolyl, phenyl, trifluoromethyl, 2,2,5,7,8-pentamethylchroman-6-yl-, and 2,3,6-trimethyl-4-methoxybenzene. Other suitable nitrogen protecting groups may be found in texts such as T.W. Greene & P.G.M. Wuts, Protective Groups in Organic Synthesis, John Wiley & Sons, 1991.

As used herein, unless otherwise noted, the term **"leaving group"** shall mean a charged or uncharged atom or group which departs during a substitution or displacement reaction. Suitable examples include, but are not limited to, Br, Cl, I, mesylate or tosylate.

As used herein, unless otherwise noted, the term **"aprotic solvent"** shall mean any solvent that does not yield a proton. Suitable examples include, but are not limited to DMF, DMAc 1,4-dioxane, THF, acetonitrile, pyridine, dichloroethane, dichloromethane, MTBE, toluene or acetone.

The present invention is directed to a process for the preparation of the compounds of formula (I) as outlined in Scheme 1, below.

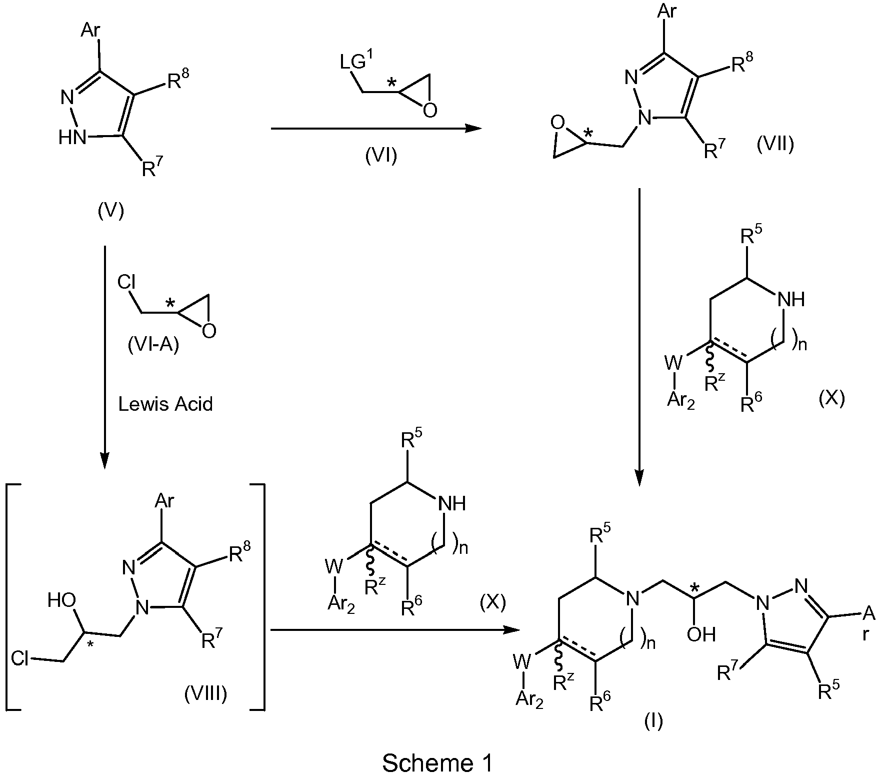

Accordingly, a suitably substituted compound of formula (V), a known compound or compound prepared by known methods is reacted with a suitably substituted compound of formula (VI), wherein LG¹ is a leaving group selected from 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl or 3-chloro-benzenesulfonyl, for example 3-nitrophenyl-sulfonyl; a known compound or compound prepared by known methods; wherein the compound of formula (VI) is present in an amount in the range of from about 0.5 to about 3.0 molar equivalents, for example, the compound is present in an amount in the range of from about 1.0 to about 1.5 molar equivalents, for example, the compound of formula (VI) is present in about 1.0 to about 1.1 molar equivalents, for example, the compound of formula (VI) is present in an amount of about 1.05 molar equivalents;
in the presence of an organic or inorganic base such as TEA, DIPEA, pyridine, NaH, KOt-Bu, NaOt-Bu, Cs₂CO₃, K₂CO₃, Na₂CO₃ or CsF, for example Cs₂CO₃; in an organic solvent such as ethyl acetate, DME, DMF, DMAc or THF, for example DMF; at a temperature in the range of from about 0°C to about 60°C, for example at a temperature in the range of from about 0°C to about 5°C, followed by heating to a temperature in the range of from about room temperature to about 60°C, for example to about 30°C, over about 12 hours; to yield the corresponding compound of formula (VII).

One skilled in the art will recognize that in an embodiment of the present invention, the leaving group LG¹ is selected to provide a highly reactive center on the compound of formula (VI); such that when the compound of formula (VI) is reacted with the compound of formula (V), any undesired coupling byproducts are minimized.

The compound of formula (VII) is reacted with a suitably substituted compound of formula (X), a known compound or compound prepared by known methods; wherein the compound of formula (X) is present in an amount in the range of from about 0.5 to about 3.0 molar equivalents, for example in an amount in the range of from about 1.0 to about 1.5 molar equivalents, for example in an amount of about 1.1 molar equivalents;
optionally in the presence of a Lewis acid such as titanium propoxide, Al(OTf)₃, or Yb(OTf)₃; wherein the Lewis acid is present in an amount in the range of from about 0.01 to about 1.0 molar equivalents, for example in an amount in the range of from about 0.01 to about 0.5 molar equivalents, for example, in an amount of about 0.04 molar equivalents, for example in a catalytic amount;
in an organic solvent such as DCE, DCM, methanol , ethanol, isopropyl alcohol, EtOAc, THF, N,N-dimethylformamide or DMAc, for example in ethanol; at a temperature in the range of from about room temperature to about solvent reflux temperature, for example at about 78°C (ethanol reflux temperature); to yield the corresponding compound of formula (I).

Alternatively, a suitably substituted compound of formula (V), a known compound of compound prepared by known methods, is reacted with a suitably substituted compound of formula (VI-A); wherein the compound of formula (VI) is present in an amount in the range of from about 0.5 to about 3.0 molar equivalents, for example, the compound is present in an amount in the range of from about 1.0 to about 2.0 molar equivalents, for example, the compound of formula (VI-A) is present in about 1.0 to about 1.5 molar equivalents, for example, the compound of formula (VI) is present in an amount of about 1.05 molar equivalents;
in the presence of a Lewis acid such as titanium propoxide, Al(OTf)₃, or Yb(OTf)₃; wherein the Lewis acid is present in an amount in the range of from about 0.01 to about 1.0 molar equivalents, for example in an amount in the range of from about 0.01 to about 0.5 molar equivalents, for example, in an amount of about 0.04 molar equivalents, for example in a catalytic amount;
in an organic solvent such as toluene, THF, DCE or methylene chloride, for example in toluene; at a temperature in the range of from about room temperature to about 80°C, for example, at about 65°C; to yield the corresponding compound of formula (VIII), which compound, in an example of the present invention, is not isolated.

The compound of formula (VIII) is reacted with a suitably substituted compound of formula (X), a known compound or compound prepared by known methods; wherein the compound of formula (X) is present in an amount in the range of from about 0.5 to about 3.0 molar equivalents, for example in an amount in the range of from about 1.0 to about 1.5 molar equivalents, for example in an amount of about 1.1 molar equivalents;
optionally, in the presence of an organic or inorganic base such as TEA, DIPEA, pyridine, Cs₂CO₃, K₂CO₃ or Na₂CO₃, for example K₂CO₃; in an organic solvent such as IPA, ethanol, methanol, EtOAc, THF, DMAc or N,N-dimethylformamide, for example in IPA; at a temperature in the range of from about 50°C to about solvent reflux temperature, for example at about solvent reflux temperature; to yield the corresponding compound of formula (I).

One skilled in the art will recognize that wherein the process as outlined in Scheme 1 above, the compound of formula (VI) is present in an enantiomeric excess of one of the enantiomers (as defined by the stereo-center denoted by the "*" symbol), then the compound of formula (I) will be prepared in an enantiomeric excess of the corresponding enantiomer. For example, wherein the compound of formula (VI) is present in an enantiomeric excess of the (S) enantiomer, then the compound of formula (I) will be prepared in an enantiomeric excess of the corresponding (S) enantiomer.

In an embodiment, the present invention is directed to a process for the preparation of the compound of formula (I-S) as outlined in Scheme 2 below.

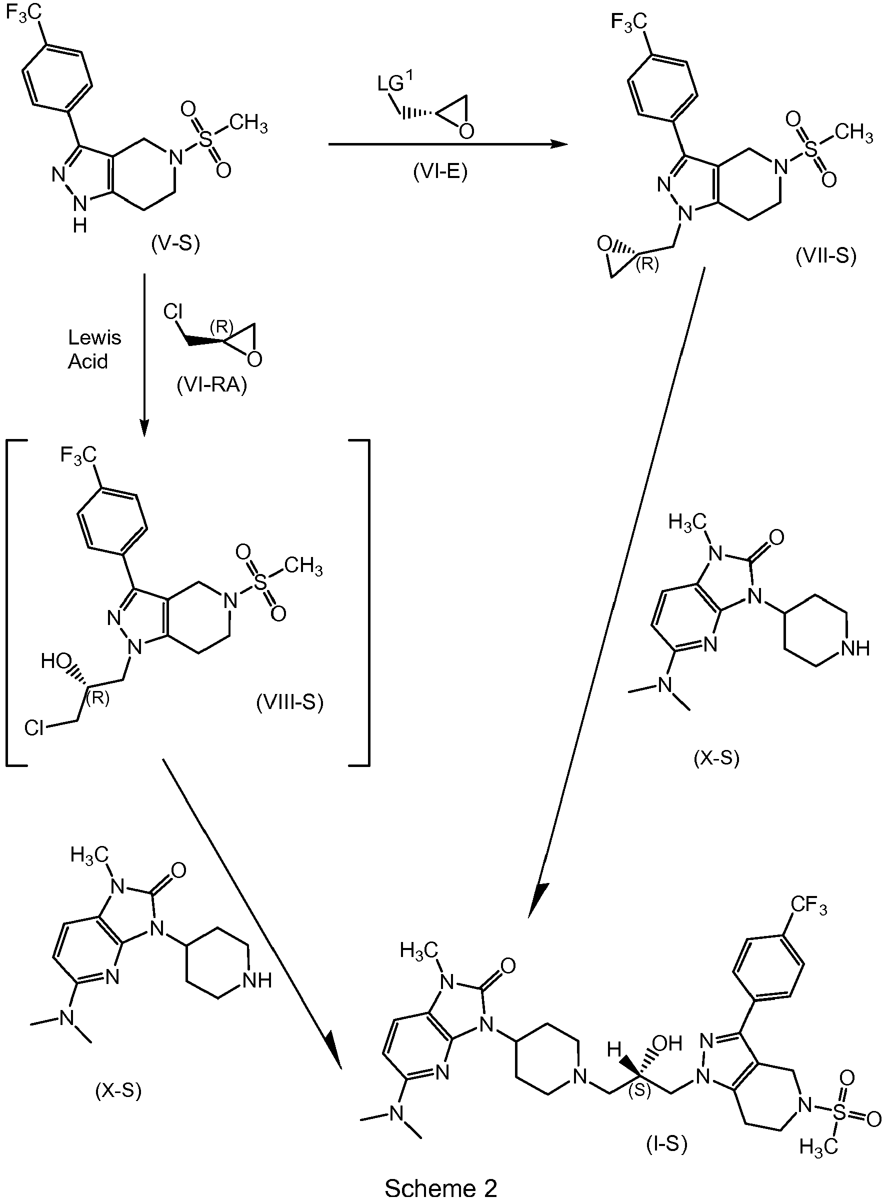

Accordingly, a suitably substituted compound of formula (V-S), a known compound or compound prepared by known methods is reacted with a suitably substituted compound of formula (VI-E). wherein LG¹ is a leaving group selected from 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl or 3-chloro-benzenesulfonyl, for example 3-nitrophenyl-sulfonyl; a known compound or compound prepared by known methods; wherein the compound of formula (VI-E) is present in an amount in the range of from about 0.5 to about 3.0 molar equivalents, for example, the compound is present in an amount in the range of from about 1.0 to about 1.5 molar equivalents, for example, in about 1.0 to about 1.1 molar equivalents, for example, in an amount of about 1.05 molar equivalents;

in the presence of an organic or inorganic base such as TEA, DIPEA, pyridine, NaH, KOt-Bu, NaOt-Bu, Cs₂CO₃, K₂CO₃, Na₂CO₃ or CsF for example Cs₂CO₃; in an organic solvent such as ethyl acetate, DME, DMF, DMAc or THF, for example DMF; at a temperature in the range of from about 0°C to about 60°C, for example at a temperature in the range of from about 0°C to about 5°C, followed by heating to a temperature in the range of from about room temperature to about 60°C, for example to about 30°C, over about 12 hours; to yield the corresponding compound of formula (VII-S).

One skilled in the art will recognize that in an embodiment of the present invention, the leaving group LG¹ is selected to provide a highly reactive center on the compound of formula (VI-E); such that when the compound of formula (VI-E) is reacted with the compound of formula (V-S), any undesired coupling byproducts are minimized.

The compound of formula (VII-S) is reacted with a suitably substituted compound of formula (X-S), a known compound or compound prepared by known methods; wherein the compound of formula (X-S) is present in an amount in the range of from about 0.5 to about 3.0 molar equivalents, for example in an amount in the range of from about 1.0 to about 1.5 molar equivalents, for example in an amount of about 1.1 molar equivalents;
optionally in the presence of a Lewis acid such as titanium propoxide, Al(OTf)₃, or Yb(OTf)₃; wherein the Lewis acid is present in an amount in the range of from about 0.01 to about 1.0 molar equivalents, for example in an amount in the range of from about 0.01 to about 0.5 molar equivalents, for example, in an amount of about 0.04 molar equivalents, for example in a catalytic amount;
in an organic solvent such as DCE, DCM, methanol, ethanol, isopropyl alcohol, EtOAc, THF, N,N-dimethylformamide or DMAc, for example in ethanol; at a temperature in the range of from about room temperature to about solvent reflux temperature, for example at about 78°C (ethanol reflux temperature); to yield the corresponding compound of formula (I-S).

Alternatively, a suitably substituted compound of formula (V-S), a known compound of compound prepared by known methods, is reacted with a suitably substituted compound of formula (VI-RA); wherein the compound of formula (VI-RA) is present in an amount in the range of from about 0.5 to about 3.0 molar equivalents, for example, the compound is present in an amount in the range of from about 1.0 to about 2.0 molar equivalents, for example, in an amount in the range of from about 1.0 to about 1.5 molar equivalents, for example, in an amount of about 1.05 molar equivalents;
in the presence of a Lewis acid such as titanium propoxide, Al(OTf)₃, or Yb(OTf)₃; wherein the Lewis acid is present in an amount in the range of from about 0.01 to about 1.0 molar equivalents, for example in an amount in the range of from about 0.01 to about 0.5 molar equivalents, for example, in an amount of about 0.04 molar equivalents, for example in a catalytic amount;
in an organic solvent such as toluene, THF, DCE or methylene chloride, for example in toluene; at a temperature in the range of from about room temperature to about 80°C, for example, at about 65°C; to yield the corresponding compound of formula (VIII-S), which compound, in an example of the present invention, is not isolated.

The compound of formula (VIII-S) is reacted with a suitably substituted compound of formula (X-S), a known compound or compound prepared by known methods; wherein the compound of formula (X-S) is present in an amount in the range of from about 0.5 to about 3.0 molar equivalents, for example in an amount in the range of from about 1.0 to about 1.5 molar equivalents, for example in an amount of about 1.1 molar equivalents;
optionally in the presence of an organic or inorganic base such as TEA, DIPEA, pyridine, Cs₂CO₃, K₂CO₃ or Na₂CO₃, for example K₂CO₃; in an organic solvent such as IPA, ethanol, methanol, EtOAc, THF, DMAc or N,N-dimethylformamide, for example in IPA; at a temperature in the range of from about 50°C to about solvent reflux temperature, for example at about solvent reflux temperature; to yield the corresponding compound of formula (I-S).

One skilled in the art will recognize that wherein the compound of formula (I), the (S) enantiomer at the starred position is desired, the compound may be prepared according to the processes as outlined in Schemes 1 and 2 by using the suitably selected enantiomer of the compound of formula (VI).

The present invention is further directed to a process for the preparation of a compound of formula (VI) wherein LG¹ is a suitably selected leaving group such as a suitably selected leaving group such as 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl or 3-chloro-benzenesulfonyl (but not chloro or bromo), as outlined in Scheme 3 below.

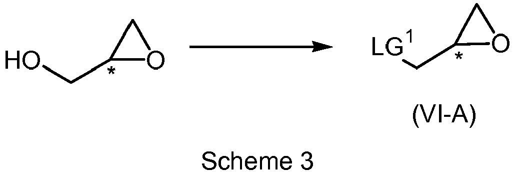

Accordingly, oxiranyl-methanol (either as a racemate or in an enantiomeric excess of one of its corresponding enantiomers), a known compound, is reacted with a suitably selected activating reagent, such as 3-nitrophenyl-sulfonyl chloride, according to known methods, to yield the compound of formula (VI), wherein LG¹ is the corresponding leaving group. For example, wherein the activating reagent is 3-nitrophenyl-sulfonyl chloride, then LG¹ (the corresponding leaving group) in the compound of formula (VI) is 3-nitrophenyl-sulfonyl.

In an embodiment, the present invention is directed to a process for the preparation of a compound of formula (VI-S), wherein LG¹ is 3-nitrophenyl-sulfonyl. In an embodiment, the present invention is directed to a process for the preparation of a compound of formula (VI-S), wherein LG¹ is 3-nitrophenyl-sulfonyl and wherein the compound of formula (VI-S) is present in enantiomeric excess of the (S)-enantiomer of greater than about 0%, for example, in enantiomeric excess of greater than about 50%, for example, in enantiomeric excess of greater than about 75%, for example, in enantiomeric excess of greater than about 85%, for example, in enantiomeric excess of greater than about 90%, for example, in enantiomeric excess of greater than about 95%, for example, in enantiomeric excess of greater than about 98%, for example, in enantiomeric excess of greater than about 99%.

In another embodiment, the present invention is directed to a process for the preparation of a compound of formula (VI-R), wherein LG¹ is 3-nitrophenyl-sulfonyl. In an embodiment, the present invention is directed to a process for the preparation of a compound of formula (VI-R), wherein LG¹ is 3-nitrophenyl-sulfonyl and wherein the compound of formula (VI-R) is present in enantiomeric excess of the (R)-enantiomer of greater than about 0%, for example, in enantiomeric excess of greater than about 50%, for example, in enantiomeric excess of greater than about 75%, for example, in enantiomeric excess of greater than about 85%, for example, in enantiomeric excess of greater than about 90%, for example, in enantiomeric excess of greater than about 95%, for example, in enantiomeric excess of greater than about 98%, for example, in enantiomeric excess of greater than about 99%.

The present invention is directed to a process for the preparation of the compound of formula (VI-S) wherein LG¹ is 3-nitrophenylsulfonyl, as outlined in more detail in Scheme 4 below.

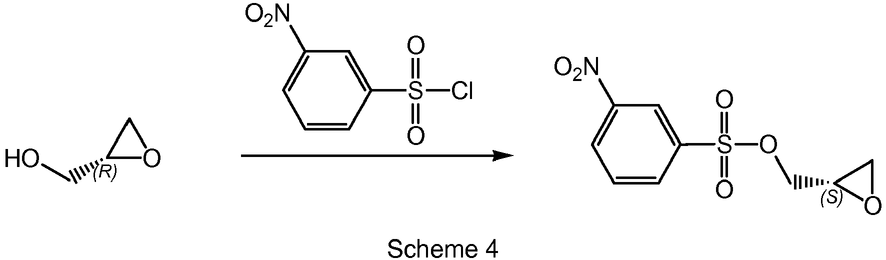

Accordingly, (R)-glycidol, a known compound is reacted with 3-nitrophenyl-sulfonyl chloride, a known compound, in the presence of an organic or inorganic base such as Cs₂CO₃, TEA, DIPEA or pyridine, for example an organic base; in an organic solvent such as DCM, DCE or DMA; for example at a temperature in the range of from about 0 to about 5°C, yield the corresponding 3-nitro-benzenesulfonic acid (S)-oxiranylmethyl ester. One skilled in the art will recognize that in an analogous process, (R)-glycidol may be reacted with 4-nitrophenylsulfonyl chloride, as outlined above, to yield the corresponding 4-nitro-benzenesulfonic acid (S)-oxiranylmethyl ester.

In an embodiment of the present invention, in any of the processes described herein, the compound of formula (VI) is present in an enantiomeric excess (of either the corresponding (S) or (R) enantiomer) of greater than or equal to about 0%ee. In another embodiment, the compound of formula (VI) is present in an enantiomeric excess (of either the (S) or (R) enantiomer) of greater than or equal to about 50%ee. In another embodiment, the compound of formula (VI) is present in an enantiomeric excess (of either the (S) or (R) enantiomer) of greater than or equal to about 75%ee. In another embodiment, the compound of formula (VI) is present in an enantiomeric excess (of either the (S) or (R) enantiomer) of greater than or equal to about 85%ee. In another embodiment, the compound of formula (VI) is present in an enantiomeric excess (of either the (S) or (R) enantiomer) of greater than or equal to about 90%ee. In another embodiment, the compound of formula (VI) is present in an enantiomeric excess (of either the (S) or (R) enantiomer) of greater than or equal to about 95%ee. In another embodiment, the compound of formula (VI) is present in an enantiomeric excess (of either the (S) or (R) enantiomer) of greater than or equal to about 98%ee. In another embodiment, the compound of formula (VI) is present in an enantiomeric excess (of either the (S) or (R) enantiomer) of greater than or equal to about 99%ee.

The present invention is further directed to processes for the preparation of compounds of formula (V-A), as outlined in Scheme 5 below.

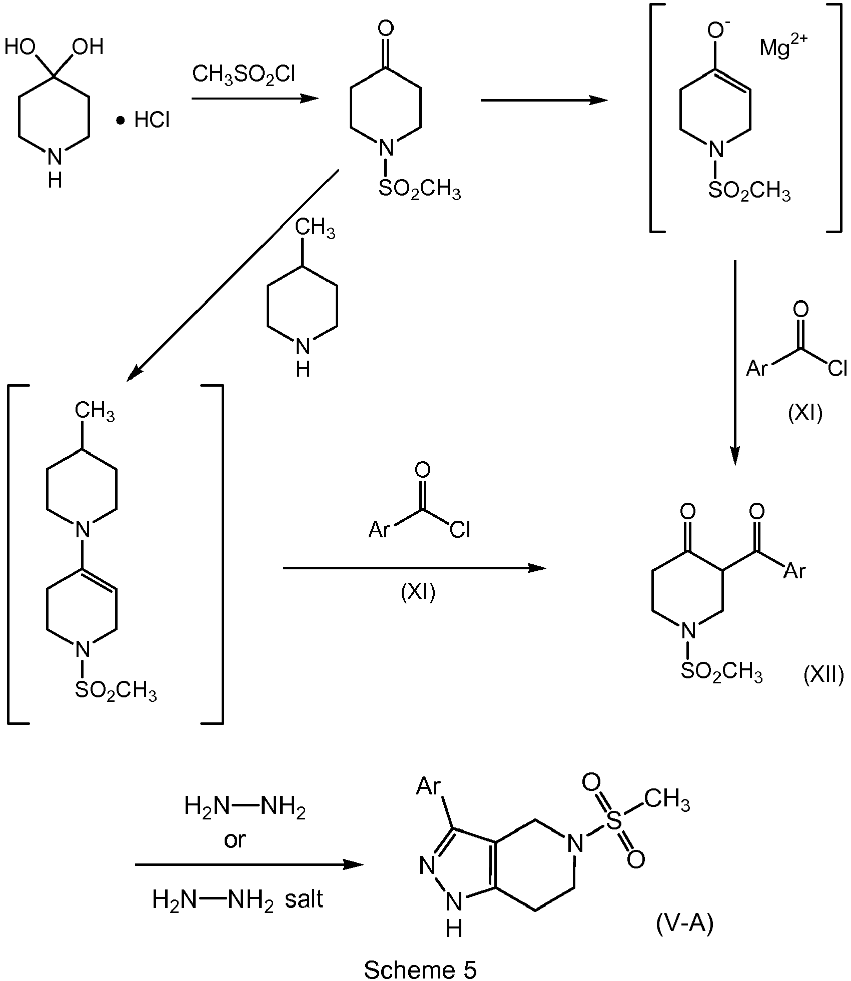

Accordingly, piperidine-4,4-diol HCl, a known compound, is reacted with methanesulfonyl chloride, a known compound; wherein the piperidine-4,4-diol HCl is present in an amount in the range of from about 1.0 to about 3.0 molar equivalents, or any range therein, for example at about 1.5 molar equivalents;
in the presence of an organic or inorganic base such as K₂CO₃, NaHCO₃, Na₂CO₃, TEA, DIPEA or pyridine, for example in the presence of aqueous NaHCO₃, wherein the base is present in an amount in the range of from about 1.0 to about 5.0 molar equivalents, or any range therein, for example, an amount in the range of from about 3.0 to about 4.0 molar equivalents, for example about 3.4 equivalents;
in an organic solvent such as acetonitrile, methylene chloride, or chloroform, for example in acetonitrile, to yield the corresponding 1-methanesulfonyl-piperidin-4-one.

The 1-methanesulfonyl-piperidin-4-one is reacted with 4-methyl-piperidine, a known compound; wherein the 4-methyl-piperidine is present in an amount in the range of from about 1.0 to about 2.0 molar equivalents, or any range therein, for example in an amount in the range of from about 1.0 to about 1.1 molar equivalents, for example, in an amount of about 1.0 equivalents;
in the presence of a catalytic amount of an acid such as p-toluene sulfonic acid; in an organic solvent such as ethyl acetate, benzene or toluene, for example in toluene; wherein the reaction mixture is heated to azeotropically remove any water; to yield the corresponding 1'-methanesulfonyl-4-methyl-3,4,5,6,1',2',3',6'-octahydro-2H-[1,4']bipyridinyl, which in an example of the process is not isolated.

One skilled in the art will recognize that the 1-methanesulfonyl-piperidin-4-one may alternatively be reacted with morpholine (instead of with 4-methyl-piperidine), in the presence of a catalytic amount of an acid such as p-toluene sulfonic acid, in an organic solvent such as benzene, to yield the 4-(1-methanesulfonyl-1,2,3,6-tetrahydro-pyridin-4-yl)-morpholine, which may be substituted for the 1'-methanesulfonyl-4-methyl-3,4,5,6,1',2',3',6'-octahydro-2H-[1,4']bipyridinyl in the next step of the process.

The 1'-methanesulfonyl-4-methyl-3,4,5,6,1',2',3',6'-octahydro-2H-[1,4']bipyridinyl is reacted with a suitably substituted compound of formula (XI), a suitably substituted acid chloride, a known compound or compound prepared by known methods; wherein the compound of formula (XI) is present in an amount in the range of from about 1.0 to about 3.0 molar equivalents, or any range therein, for example, in an amount of from about 1.0 to about 1.5 molar equivalents, for example in an amount in about 1.05 molar equivalents;
in the presence of an organic base such as TEA, DIPEA or pyridine, for example in the presence of TEA, wherein the base is present in an amount in the range of from about 1.0 to about 3.0 molar equivalents, or any range therein, for example, in an amount in the range of from about 1.0 to about 1.5 molar equivalents, for example in an amount of about 1.1 molar equivalents;
in an organic solvent such as toluene, DCE or DCM; for example, in toluene; for example in the same solvent as used in the previous reaction step; to yield the corresponding compound of formula (XII).

Alternatively, 1-methanesulfonyl-piperidin-4-one is reacted with a suitably selected source of magnesium, such as Mgl₂ or MgBr₂, for example, Mgl₂; for example, wherein the source of magnesium is MgBr₂, then the weight % of water is about 0.1%; and for example, wherein the source of magnesium is Mgl₂, then the weight % of water in the source of magnesium is about 1 %; and wherein the source of magnesium is present in an amount in the range of from about 0.75 to about 3.0 molar equivalents (relatives to the moles of 1-methanesulfonyl-piperidin-4-one), for example, in an amount in the range of from about 1.0 to about 2.0 molar equivalents, for example, in an amount of about 1.2 molar equivalents;
in the presence of a base such as TEA, DIPEA or pyridine, for example TEA; wherein the base is for example present in an amount in the range of from about 1.0 to about 5.0 molar equivalents (relatives to the moles of 1-methanesulfonyl-piperidin-4-one), for example, in an amount in the range of from about 2.0 to about 4.0 molar equivalents, for example, in an amount of about 3.0 molar equivalents;
in an organic solvent such as DCM, DCE or chloroform, for example DCM; for example at a temperature in the range of from about 20°C to about 40°C; to yield the corresponding magnesium 1-methanesulfonyl-1,2,3,6-tetrahydro-pyridin-4-olate, which compound, in an example of the present invention, not isolated.

The magnesium 1-methanesulfonyl-1,2,3,6-tetrahydro-pyridin-4-olate is reacted with a suitably substituted compound of formula (XI), a suitably substituted acid chloride, a known compound or compound prepared by known methods; wherein the compound of formula (XI) is present in an amount in the range of from about 1.0 to about 3.0 molar equivalents, or any range therein, for example, in an amount of from about 1.0 to about 1.5 molar equivalents, for example in an amount in about 1.2 molar equivalents;
in the presence of an organic base such as TEA, DIPEA or pyridine, for example in the presence of TEA, wherein the base is present in an amount in the range of from about 1.0 to about 5.0 molar equivalents, or any range therein, for example, in an amount in the range of from about 1.0 to about 4.0 molar equivalents, for example in an amount of about 3.0 molar equivalents;
in an organic solvent such as toluene, DCE, DCM or chloroform, for example, in DCM; for example in the same solvent as used in the previous reaction step; to yield the corresponding compound of formula (XII).

The compound of formula (XII) is reacted with hydrazine or its corresponding salt, for example hydrazine, hydrazine hydrochloride, hydrazine dihydrochloride or hydrazine sulfate, for example, hydrazine hydrochloride, a known compound; wherein the hydrazine or corresponding salt is present in an amount in the range of from about 0.5 to about 3.0 molar equivalents, or any range therein, for example, in an amount in the range of from about 0.5 to about 2.0 molar equivalents, for example in an amount of about 1.0 molar equivalent;
in the presence of an inorganic or organic base such as NaOH, NaOCH₃, KOt-Bu, TEA, DIPEA or pyridine, for example in the presence of 1 N aqueous NaOH; wherein the base is present in an amount in the range of from about 1.0 to about 3.0 molar equivalents, or any range therein, for example, the base is present in an amount of about 1.0 to about 1.5 molar equivalents, for example in an amount of about 1.1 molar equivalents;
in a solvent or mixture of solvents such as ethanol, methanol, IPA or ethanol/water mixture, for example in an ethanol/water mixture; to yield the corresponding compound of formula (V-A).

The compound of formula (V-A) is optionally isolated according to known methods, for example by filtration. The compound of formula (V-A) is further optionally further purified according to known methods, for example by column chromatography or by recrystallization.

In an embodiment, the present invention is directed to a process for the preparation of a compound of formula (V-S), as outlined in Scheme 6, below.

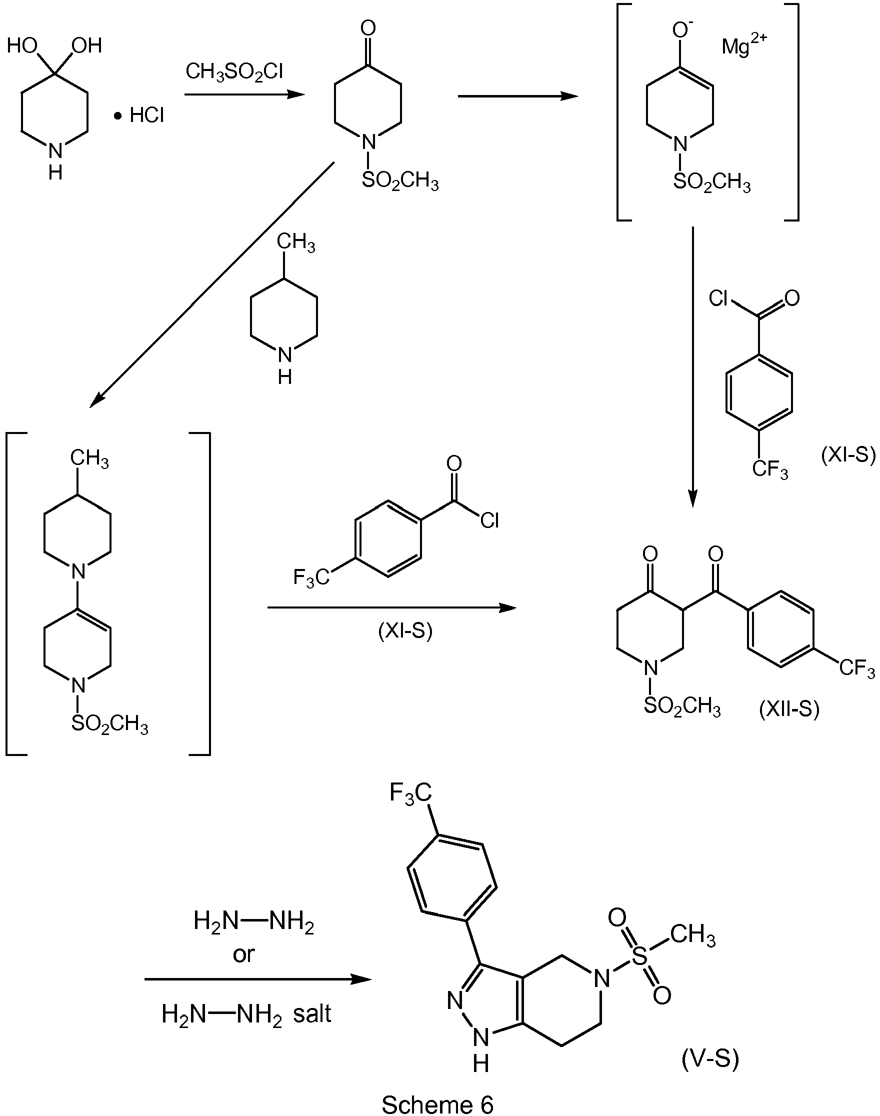

Accordingly, piperidine-4,4-diol HCl, a known compound, is reacted with methanesulfonyl chloride, a known compound; wherein the piperidine-4,4-diol HCl is present in an amount in the range of from about 1.0 to about 3.0 molar equivalents, or any range therein, for example at about 1.5 molar equivalents;
in the presence of an organic or inorganic base such as K₂CO₃, NaHCO₃, Na₂CO₃, TEA, DIPEA or pyridine, for example in the presence of aqueous NaHCO₃, wherein the base is present in an amount in the range of from about 1.0 to about 5.0 molar equivalents, or any range therein, for example, an amount in the range of from about 3.0 to about 4.0 molar equivalents, for example about 3.4 equivalents;
in an organic solvent such as acetonitrile, methylene chloride, or chloroform, for example in acetonitrile, to yield the corresponding 1-methanesulfonyl-piperidin-4-one.

The 1-methanesulfonyl-piperidin-4-one is reacted with 4-methyl-piperidine, a known compound; wherein the 4-methyl-piperidine is present in an amount in the range of from about 1.0 to about 2.0 molar equivalents, or any range therein, for example in an amount in the range of from about 1.0 to about 1.1 molar equivalents, for example, in an amount of about 1.0 equivalents;
in the presence of a catalytic amount of an acid such as p-toluene sulfonic acid; in an organic solvent such as ethyl acetate, benzene or toluene, for example in toluene; wherein the reaction mixture is heated to azeotropically remove any water; to yield the corresponding 1'-methanesulfonyl-4-methyl-3,4,5,6,1',2',3',6'-octahydro-2H-[1,4']bipyridinyl, which in an example of the process is not isolated.

One skilled in the art will recognize that the 1-methanesulfonyl-piperidin-4-one may alternatively be reacted with morpholine (instead of with 4-methyl-piperidine), in the presence of a catalytic amount of an acid such as p-toluene sulfonic acid, in an organic solvent such as benzene, to yield the 4-(1-methanesulfonyl-1,2,3,6-tetrahydro-pyridin-4-yl)-morpholine, which may be substituted for the 1'-methanesulfonyl-4-methyl-3,4,5,6,1',2',3',6'-octahydro-2H-[1,4']bipyridinyl in the next step of the process.

The 1 '-methanesulfonyl-4-methyl-3,4,5,6, ',2',3',6'-octahydro-2H-[1,4']bipyridinyl is reacted with a suitably substituted compound of formula (XI), a suitably substituted acid chloride, a known compound or compound prepared by known methods; wherein the compound of formula (XI-S) is present in an amount in the range of from about 1.0 to about 3.0 molar equivalents, or any range therein, for example, in an amount of from about 1.0 to about 1.5 molar equivalents, for example in an amount in about 1.05 molar equivalents;
in the presence of an organic base such as TEA, DIPEA or pyridine, for example in the presence of TEA, wherein the base is present in an amount in the range of from about 1.0 to about 3.0 molar equivalents, or any range therein, for example, in an amount in the range of from about 1.0 to about 1.5 molar equivalents, for example in an amount of about 1.1 molar equivalents;
in an organic solvent such as toluene, DCE or DCM; for example, in toluene; for example in the same solvent as used in the previous reaction step; to yield the corresponding compound of formula (XII-S).

Alternatively, 1-methanesulfonyl-piperidin-4-one is reacted with a suitably selected source of magnesium, such as Mgl₂ or MgBr₂, for example, Mgl₂; for example, wherein the source of magnesium is MgBr₂, then the weight % of water is about 0.1%; and for example, wherein the source of magnesium is Mgl₂, then the weight % of water in the source of magnesium is about 1 %; and wherein the source of magnesium is present in an amount in the range of from about 0.75 to about 3.0 molar equivalents (relatives to the moles of 1-methanesulfonyl-piperidin-4-one), for example, in an amount in the range of from about 1.0 to about 2.0 molar equivalents, for example, in an amount of about 1.2 molar equivalents;
in the presence of a base such as TEA, DIPEA or pyridine, for example TEA; wherein the base is for example present in an amount in the range of from about 1.0 to about 5.0 molar equivalents (relatives to the moles of 1-methanesulfonyl-piperidin-4-one), for example, in an amount in the range of from about 2.0 to about 4.0 molar equivalents, for example, in an amount of about 3.0 molar equivalents;
in an organic solvent such as DCM, DCE or chloroform, for example DCM; for example at a temperature in the range of from about 20°C to about 40°C; to yield the corresponding magnesium 1-methanesulfonyl-1,2,3,6-tetrahydro-pyridin-4-olate, which compound, in an example of the present invention, not isolated.

The magnesium 1-methanesulfonyl-1,2,3,6-tetrahydro-pyridin-4-olate is reacted with a suitably substituted compound of formula (XI-S), a suitably substituted acid chloride, a known compound or compound prepared by known methods; wherein the compound of formula (XI-S) is present in an amount in the range of from about 1.0 to about 3.0 molar equivalents, or any range therein, for example, in an amount of from about 1.0 to about 1.5 molar equivalents, for example in an amount in about 1.2 molar equivalents;
in the presence of an organic base such as TEA, DIPEA or pyridine, for example in the presence of TEA, wherein the base is present in an amount in the range of from about 1.0 to about 5.0 molar equivalents, or any range therein, for example, in an amount in the range of from about 1.0 to about 4.0 molar equivalents, for example in an amount of about 3.0 molar equivalents;
in an organic solvent such as toluene, DCE, DCM or chloroform, for example, DCM; for example in the same solvent as used in the previous reaction step; to yield the corresponding compound of formula (XII-S).

The compound of formula (XII-S) is reacted with hydrazine or its corresponding salt, for example hydrazine, hydrazine hydrochloride, hydrazine dihydrochloride or hydrazine sulfate, for example, hydrazine hydrochloride, a known compound; wherein the hydrazine or corresponding salt is present in an amount in the range of from about 0.5 to about 3.0 molar equivalents, or any range therein, for example, in an amount in the range of from about 0.5 to about 2.0 molar equivalents, for example in an amount of about 1.0 molar equivalent;
in the presence of an inorganic or organic base such as NaOH, NaOCH₃, KOt-Bu, TEA, DIPEA or pyridine, for example in the presence of 1N aqueous NaOH; wherein the base is present in an amount in the range of from about 1.0 to about 3.0 molar equivalents, or any range therein, for example, the base is present in an amount of about 1.0 to about 1.5 molar equivalents, for example in an amount of about 1.1 molar equivalents;
in a solvent or mixture of solvents such as ethanol, methanol, IPA or ethanol/water mixture, for example in an ethanol/water mixture; to yield the corresponding compound of formula (V-S).

The compound of formula (V-S) is optionally isolated according to known methods, for example by filtration. The compound of formula (V-S) is further optionally further purified according to known methods, for example by column chromatography or by recrystallization.

The present invention is further directed to a process for the preparation of compounds of formula (V-A) as outlined in more detail in Scheme 7 below.

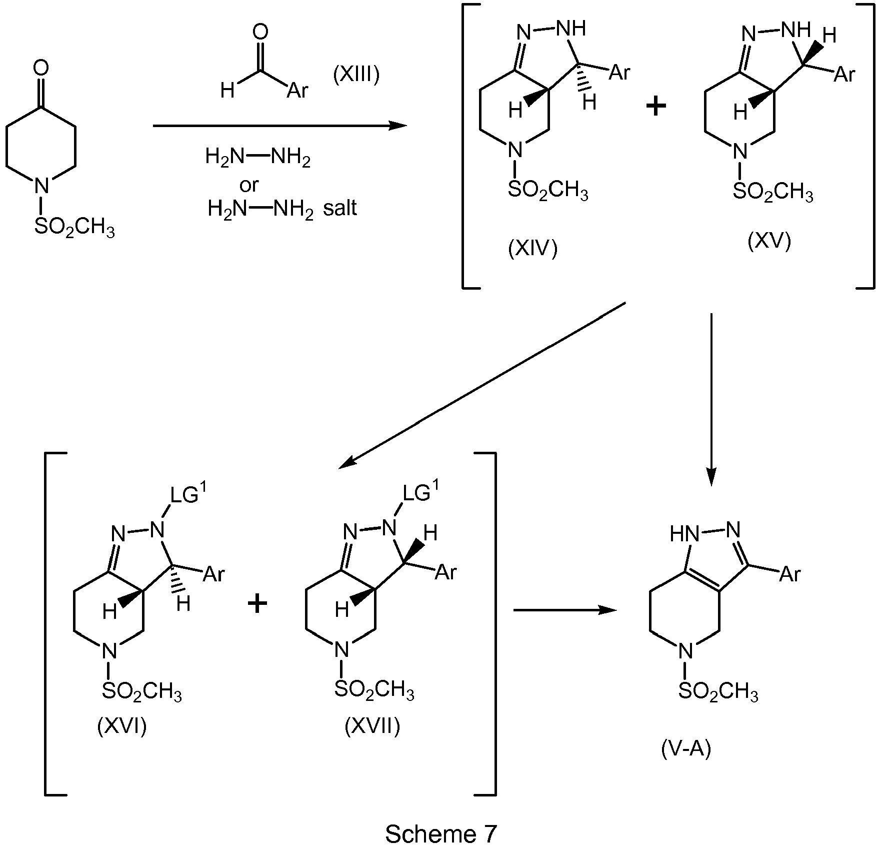

Accordingly, 1-methanesulfonyl-piperidin-4-one, a known compound or compound prepared according to for example, the process outlined in Scheme 5 above, is reacted with a suitably substituted compound of formula (XIII), a known compound or compound prepared by known methods, wherein the compound of formula (XIII) is present in an amount in the range of from about 1.0 to about 2.0 molar equivalents (relative to the moles of 1-methanesulfonylpiperidin-4-one), for example, in an amount in the range of from about 1.0 to about 2.0 molar equivalents, for example, in an amount of about 1.0 molar equivalents;
in the presence of hydrazine or its corresponding salt, for example, hydrazine, hydrazine hydrochloride, hydrazine dihydrochloride or hydrazine sulfate, for example, hydrazine hydrochloride, a known compound; wherein the hydrazine or corresponding salt is present in an amount in the range of from about 1.0 to about 3.0 molar equivalents (relative to the moles of 1-methanesulfonyl-piperidin-4-one), for example, in an amount in the range of from about 1.0 to about 1.5 molar equivalents, for example, in an amount of about 1.0 molar equivalents;
in an organic solvent such as methanol, ethanol or IPA, for example ethanol; for example at a temperature in the range of from about 10°C to about 65°C; to yield a mixture of the corresponding compound of formula (XIV) and the corresponding compound of formula (XV).

In an example of the present invention, the compounds of formula (XIV) and (XV) are not separated. In another example of the present invention, the compounds of formula (XIV) and (XV) are not isolated.

The mixture of the compound of formula (XIV) and the compound of formula (XV) is converted to the corresponding compound of formula (V-A) according to any of the following reaction sequences, Methods A through D as outlined in detail below.

Method A: The mixture of the compound of formula (XIV) and the compound of formula (XV) is reacted with a suitably selected activating agent such as Br₂, Cl₂, I₂, I₂/ NaOCH₃, for example Br₂ wherein the bromine is present in an amount in the range of from about 0.5 to about 3.0 molar equivalents, for example, in an amount in the range of from about 1.0 to about 2.0 molar equivalents, for example, in an amount of about 1.25 molar equivalents;

in an organic solvent such as methanol, ethanol or isopropanol, for example ethanol; for example at a temperature in the range of from about 10°C to about room temperature; to yield a mixture of the corresponding compound of formula (XVI) and the corresponding compound of formula (XVII); wherein LG¹ is the corresponding leaving group. Wherein the mixture of the compound of formula (XIV) and the compound of formula (XV) is reacted with Br₂, then LG¹ is Br; with Cl₂, then LG¹ is Cl; or with I₂ or I₂/NaOCH₃, then LG¹ is I.

The mixture of the compound of formula (XVI) and the compound of formula (XVII) is reacted with organic and inorganic base such as sodium methoxide, sodium t-butoxide, potassium methoxide, potassium t-butoxide, KOH, NaOH, K₂CO₃, Na₂CO₃, Cs₂CO₃, pyridine, TEA, DIPEA, for example, a 30% alcoholic solution of sodium methoxide; wherein the alcoholic base is present in an amount in the range of from about 1.0 to about 5.0 molar equivalents, for example in an amount in the range of from about 2.0 to about 4.0 molar equivalents for example, in an amount of about 2.5 molar equivalents;
in an organic solvent such as methanol, ethanol or isopropanol, for example ethanol; for example, at about room temperature; to yield the corresponding compound of formula (V-A).

Method B: The mixture of the compound of formula (XIV) and the compound of formula (XV) is reacted with a suitably selected activating agent such as Br₂, Cl₂, I₂, I₂/ NaOCH₃, for example Br₂ wherein the bromine is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature; to yield a mixture of the corresponding compound of formula (XVI) and the corresponding compound of formula (XVII); wherein LG¹ is the corresponding leaving group. Wherein the mixture of the compound of formula (XIV) and the compound of formula (XV) is reacted with Br₂, then LG¹ is Br; with Cl₂, then LG¹ is Cl; or with I₂ or I₂/NaOCH₃, then LG¹ is I.

The mixture of the compound of formula (XVI) and the compound of formula (XVII) is subjected to thermal conversion; for example, at a temperature in the range of from about 0°C to about solvent reflux temperature, for example at a temperature in the range of from about 25°C to about solvent reflux temperature; in an organic solvent such as methanol, ethanol or isopropanol, for example ethanol; for example, at about room temperature; to yield the corresponding compound of formula (V-A).

Method C: The mixture of the compound of formula (XIV) and the compound of formula (XV) is reacted with a suitably selected oxidizing agent such as MnO₂, DDQ, Chloranil, H₂O₂, Fe(NO₃)₃ or K₃[Fe(CN)₆]; wherein the oxidizing agent is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature;
followed by treatment with an organic and inorganic base such as sodium methoxide, sodium t-butoxide, potassium methoxide, potassium t-butoxide, KOH, NaOH, K₂CO₃, Na₂CO₃, Cs₂CO₃, pyridine, TEA, DIPEA, for example, a 30% alcoholic solution of sodium methoxide; wherein the alcoholic base is present in an amount in the range of from about 1.0 to about 5.0 molar equivalents, for example in an amount in the range of from about 2.0 to about 4.0 molar equivalents for example, in an amount of about 2.5 molar equivalents;
in an organic solvent such as methanol, ethanol or isopropanol, for example ethanol; for example, at about room temperature; to yield the corresponding compound of formula (V-A).

Method D: The mixture of the compound of formula (XIV) and the compound of formula (XV) is reacted with a suitably selected oxidizing agent such as MnO₂, DDQ, Chloranil, H₂O₂, Fe(NO₃)₃ or K₃[Fe(CN)₆]; wherein the oxidizing agent is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature;
followed by thermal conversion; for example, at a temperature in the range of from about 0°C to about solvent reflux temperature, for example at a temperature in the range of from about 25°C to about solvent reflux temperature; in an organic solvent such as methanol, ethanol or isopropanol, for example ethanol; for example, at about room temperature; to yield the corresponding compound of formula (V-A).

The compound of formula (V-A) is optionally isolated and further, optionally separated from the mixture, according to known methods, for example by filtration.

In an embodiment, the present invention is directed to a process for the preparation of a compound of formula (V-S), as outlined in more detail in Scheme 8, below.

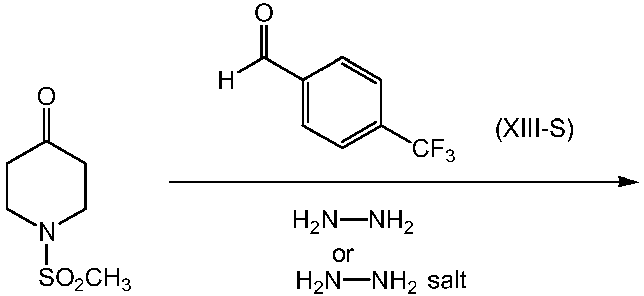

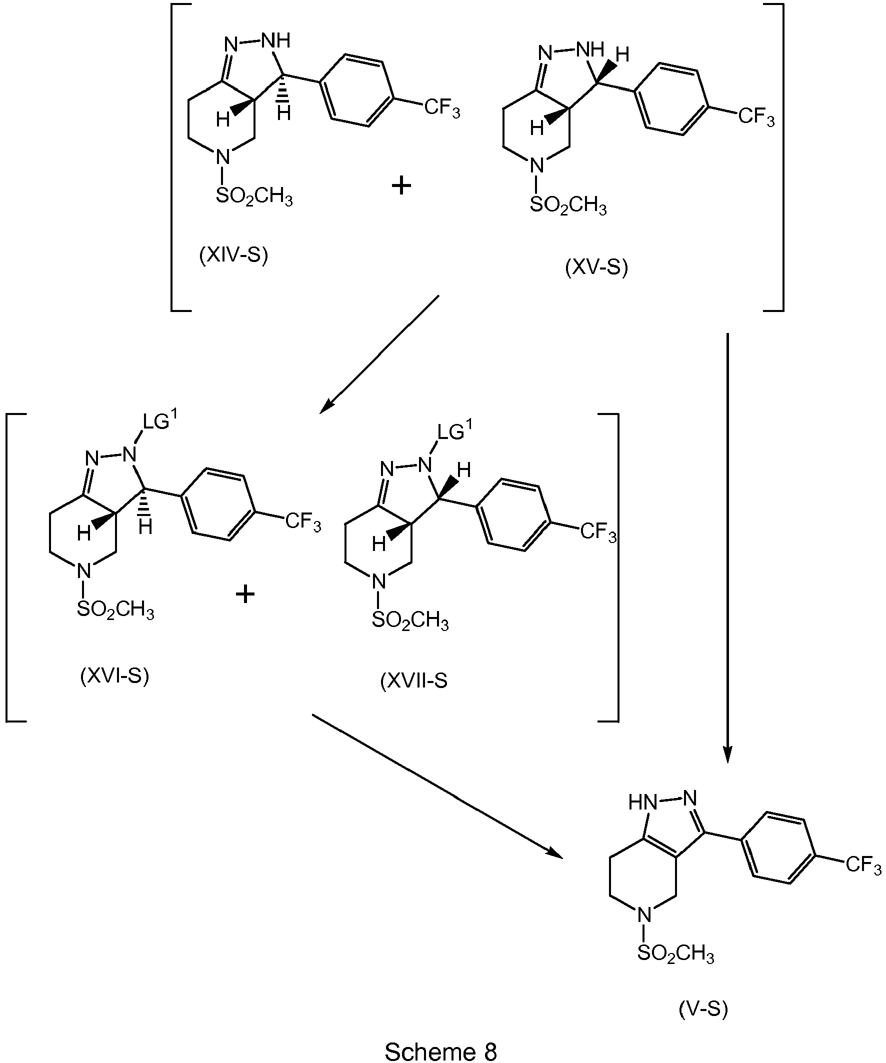

Accordingly, 1-methanesulfonyl-piperidin-4-one, a known compound or compound prepared according to for example, the process outlined in Scheme 5 above, is reacted with a suitably substituted compound of formula (XIII-S), a known compound or compound prepared by known methods, wherein the compound of formula (XIII-S) is present in an amount in the range of from about 1.0 to about 2.0 molar equivalents (relative to the moles of 1-methanesulfonyl-piperidin-4-one), for example, in an amount in the range of from about 1.0 to about 2.0 molar equivalents, for example, in an amount of about 1.0 molar equivalents;

in the presence of hydrazine or its corresponding salt, for example, hydrazine, hydrazine hydrochloride, hydrazine dihydrochloride or hydrazine sulfate, for example, hydrazine hydrochloride, a known compound; wherein the hydrazine or corresponding salt is present in an amount in the range of from about 1.0 to about 3.0 molar equivalents (relative to the moles of 1-methanesulfonyl-piperidin-4-one), for example, in an amount in the range of from about 1.0 to about 1.5 molar equivalents, for example, in an amount of about 1.0 molar equivalents;
in an organic solvent such as methanol, ethanol or IPA, for example ethanol; for example at a temperature in the range of from about 10°C to about 65°C; to yield a mixture of the corresponding compound of formula (XIV-S) and the corresponding compound of formula (XV-S).
In an example of the present invention, the compounds of formula (XIV-S) and (XV-S) are not separated. In another example of the present invention, the compounds of formula (XIV-S) and (XV-S) are not isolated.

The mixture of the compound of formula (XIV-S) and the compound of formula (XV-S) is converted to the corresponding compound of formula (V-S) according to any of the following reaction sequences, Methods A through D as outlined in detail below.

Method A: The mixture of the compound of formula (XIV-S) and the compound of formula (XV-S) is reacted with a suitably selected activating agent such as Br₂, Cl₂, I₂, I₂/ NaOCH₃, for example Br₂ wherein the bromine is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature; to yield a mixture of the corresponding compound of formula (XVI-S) and the corresponding compound of formula (XVII-S); wherein LG¹ is the corresponding leaving group. Wherein the mixture of the compound of formula (XIV-S) and the compound of formula (XV-S) is reacted with Br₂, then LG¹ is Br; with Cl₂, then LG¹ is Cl; or with I₂ or I₂/NaOCH₃, then LG¹ is I.

The mixture of the compound of formula (XVI-S) and the compound of formula (XVII-S) is reacted with organic and inorganic base such as sodium methoxide, sodium t-butoxide, potassium methoxide, potassium t-butoxide, KOH, NaOH, K₂CO₃, Na₂CO₃, C_{S2}CO₃, pyridine, TEA, DIPEA, for example, a 30% alcoholic solution of sodium methoxide; wherein the alcoholic base is present in an amount in the range of from about 1.0 to about 5.0 molar equivalents, for example in an amount in the range of from about 2.0 to about 4.0 molar equivalents for example, in an amount of about 2.5 molar equivalents;
in an organic solvent such as methanol, ethanol or isopropanol, for example ethanol; for example, at about room temperature; to yield the corresponding compound of formula (V-S).

Method B: The mixture of the compound of formula (XIV-S) and the compound of formula (XV-S) is reacted with a suitably selected activating agent such as Br₂, Cl₂, I₂, I₂/ NaOCH₃, for example Br₂ wherein the bromine is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature; to yield a mixture of the corresponding compound of formula (XVI-S) and the corresponding compound of formula (XVII-S); wherein LG¹ is the corresponding leaving group. Wherein the mixture of the compound of formula (XIV-S) and the compound of formula (XV-S) is reacted with Br₂, then LG¹ is Br; with Cl₂, then LG¹ is Cl; or with I₂ or I₂/NaOCH₃, then LG¹ is I.

The mixture of the compound of formula (XVI-S) and the compound of formula (XVII-S) is subjected to thermal conversion; for example, at a temperature in the range of from about 0°C to about solvent reflux temperature, for example at a temperature in the range of from about 25°C to about solvent reflux temperature; in an organic solvent such as methanol, ethanol or isopropanol, for example ethanol; for example, at about room temperature; to yield the corresponding compound of formula (V-S).

Method C: The mixture of the compound of formula (XIV-S) and the compound of formula (XV-S) is reacted with a suitably selected oxidizing agent such as MnO₂, DDQ, Chloranil, H₂O₂, Fe(NO₃)₃ or K₃[Fe(CN)₆]; wherein the oxidizing agent is present in an amount in the range of from about 0.5 to about 3.0 molar equivalents, for example, in an amount in the range of from about 1.0 to about 2.0 molar equivalents, for example, in an amount of about 1.25 molar equivalents;
in an organic solvent such as methanol, ethanol or isopropanol, for example ethanol; for example at a temperature in the range of from about 10°C to about room temperature;
followed by treatment with an organic and inorganic base such as sodium methoxide, sodium t-butoxide, potassium methoxide, potassium t-butoxide, KOH, NaOH, K₂CO₃, Na₂CO₃, Cs₂CO₃, pyridine, TEA, DIPEA, for example, a 30% alcoholic solution of sodium methoxide; wherein the alcoholic base is present in an amount in the range of from about 1.0 to about 5.0 molar equivalents, for example in an amount in the range of from about 2.0 to about 4.0 molar equivalents for example, in an amount of about 2.5 molar equivalents;
in an organic solvent such as methanol, ethanol or isopropanol, for example ethanol; for example, at about room temperature; to yield the corresponding compound of formula (V-S).

Method D: The mixture of the compound of formula (XIV-S) and the compound of formula (XV-S) is reacted with a suitably selected oxidizing agent such as MnO₂, DDQ, Chloranil, H₂O₂, Fe(NO₃)₃ or K₃[Fe(CN)₆]; wherein the oxidizing agent is present in an amount in the range of from about 0.5 to about 3.0 molar equivalents, for example, in an amount in the range of from about 1.0 to about 2.0 molar equivalents, for example, in an amount of about 1.25 molar equivalents;

in an organic solvent such as methanol, ethanol or isopropanol, for example ethanol; for example at a temperature in the range of from about 10°C to about room temperature;
followed by thermal conversion; for example, at a temperature in the range of from about 0°C to about solvent reflux temperature, for example at a temperature in the range of from about 25°C to about solvent reflux temperature; in an organic solvent such as methanol, ethanol or isopropanol, for example ethanol; for example, at about room temperature; to yield the corresponding compound of formula (V-S).

The compound of formula (V-S) is optionally isolated and further, optionally separated from the mixture, according to known methods, for example by filtration.

The present invention is further directed to a process for the preparation of compounds of formula (X-A), as outlined in more detail in Scheme 9 below.

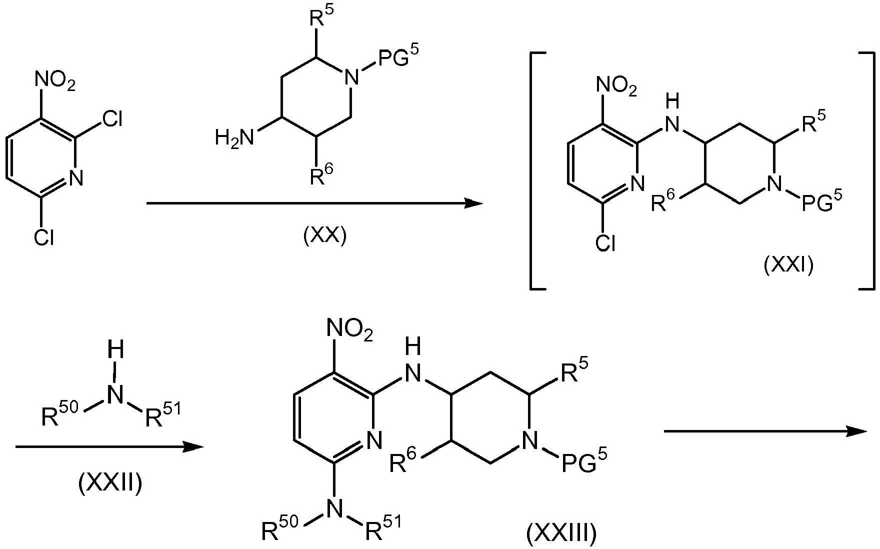

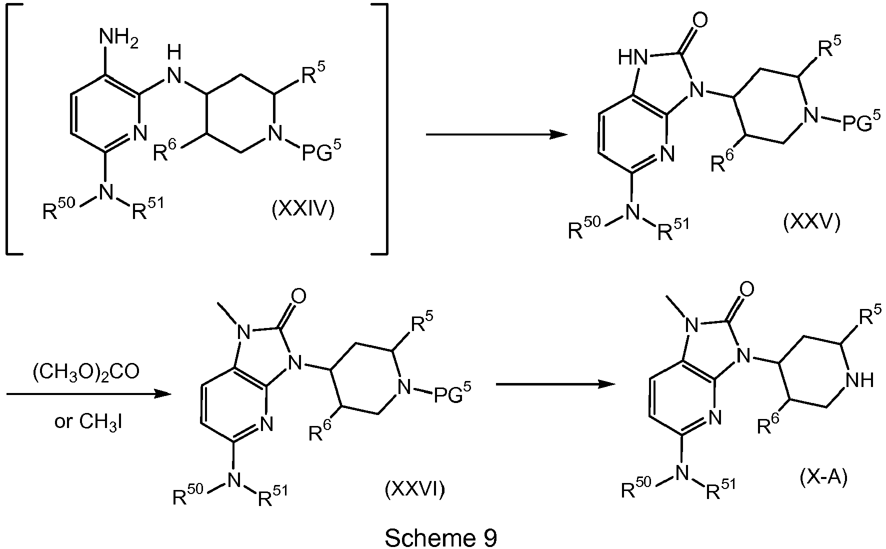

Accordingly, 2,6-dichloro-3-nitro-pyridine, a known compound, is reacted with a compound of formula (XX), wherein PG⁵ is a suitably selected nitrogen protecting group such as -C(O)O-ethyl, -C(O)O-methyl or -C(O)-methyl, for example -C(O)O-ethyl, a known compound or compound prepared by known methods; wherein the compound of formula (XX) is present in an amount in the range of from about 0.75 to about 2.0 molar equivalents, or any range therein, for example, in an amount in the range of from about 0.95 to about 1.1 molar equivalents, for example in an amount of about 1.0 molar equivalents;
in the presence of an organic or inorganic base such as DIPEA, TEA, pyridine, potassium carbonate or cesium carbonate, for example in the presence of DIPEA, wherein the base is present in an amount in the range of from about 1.0 to about 5.0 molar equivalents, or any range therein, for example, in an amount in the range of from about 2.0 to about 2.5 molar equivalents, for example in an amount of about 2.35 equivalents;
in an organic solvent such as acetonitrile, DMF, DME or THF, for example in acetonitrile; at a temperature in the range of from about 0°C to about 25 °C, or any range therein, for example at about 20°C, to yield the corresponding compound of formula (XXI). In an example of the process, the compound of formula (XXI) is not isolated.

The compound of formula (XXI) is reacted with a suitably substituted compound of formula (XXII), a known compound or compound prepared by known methods; wherein the compound of formula (XXII) may be present as a gas or in solution; wherein the compound of formula (XXII) is present in an amount in the range of from about 0.75 to about 2.0 molar equivalents, for example, in an amount in the range of from about 1.0 to about 1.6 molar equivalents, for example in an amount of about 1.15 molar equivalents;
in an organic solvent such as acetonitrile, THF, DCM, methanol, or a mixture of organic solvents, for example in acetonitrile, to yield the corresponding compound of formula (XXIII).

The compound of formula (XXIII) is reacted with a suitably selected reducing agent such as H₂ gas in the presence of a suitably selected catalyst such as a suitably selected catalyst, for example, with hydrogen gas in the presence of a catalytic amount of Pt/C;
in an organic solvent such as ethyl acetate, ethanol, toluene or THF, for example in ethyl acetate; to yield the corresponding compound of formula (XXIV). In an example of the process, the compound of formula (XXIV) is not isolated.

The compound of formula (XXIV) reacted with a suitably selected reagent such as CDI, N-methylcarbodiimide, phosgene or triphosgene, for example with CDI, wherein the reagent is present in an amount in the range of from about 1.0 to about 2.0 molar equivalents, for example in an amount in the range of from about 1.1 to about 1.5 molar equivalents, for example in an amount of from about 1.25 to about 1.3 molar equivalents; in an organic solvent such as ethyl acetate, THF, for example in ethyl acetate, to yield the corresponding compound of formula (XXV).

The compound of formula (XXV) is reacted with carbonic acid dimethyl ester, a known compound; wherein carbonic acid dimethyl ester is present in an amount in the range of from about 1.0 to about 5.0 molar equivalents, for example, in an amount in the range of from about 1.0 to about 3.0 molar equivalents, for example in an amount of about 2.2 molar equivalents;
in the presence of an inorganic base such as K₂CO₃, Na₂CO₃, Cs₂CO₃, for example K₂CO₃; wherein the base is present in an amount in the range of from about 0.75 to about 1.5 molar equivalents, for example, in an amount in the range of from about 0.8 to about 1.25 molar equivalents, for example, the in an amount of about 1.0 molar equivalents;
in an organic solvent such as DMF or DMA, for example in DMF; at a temperature in the range of from about 50°C to about solvent reflux temperature, for example at about solvent reflux temperature, to yield the corresponding compound of formula (XXVI).

Alternatively, the compound of formula (XXV) is reacted with CH₃I, a known compound, wherein the CH₃l is present in an amount in the range of from about 1.0 to about 3.0 molar equivalents, for example in an amount in the range of from about 1.0 to about 1.2 molar equivalents; in the presence of a base such as KHMDS or NaHMDS; wherein the base is present in an amount in the range of from about 1.0 to about 2.0 molar equivalents, for example in an amount in the range of from about 1.1 to about 1.5 molar equivalents; in an organic solvent such as THF, DMF or methylene chloride, for example THF; to yield the corresponding compound of formula (XXVI).

The compound of formula (XXVI) is de-protected according to known methods, to yield the corresponding compound of formula (X-A). For example, wherein PG⁵ is -C(O)O-ethyl, the compound of formula (XXVI) is reacted with a suitably selected base such as KOH or NaOH, for example with aqueous KOH; wherein the base is present in an amount in range of from about 5.0 to about 10.0 molar equivalents, for example, in an amount in the range of from about 7.0 to about 9.0 molar equivalents, for example in an amount of about 8.7 molar equivalents; in an organic solvent such as ethanol, methanol or isopropanol, for example in ethanol; to yield the corresponding compound of formula (X-A).

In an embodiment, the present invention is directed to a process for the preparation of the compound of formula (X-S) as outlined in more detail in Scheme 10 below.

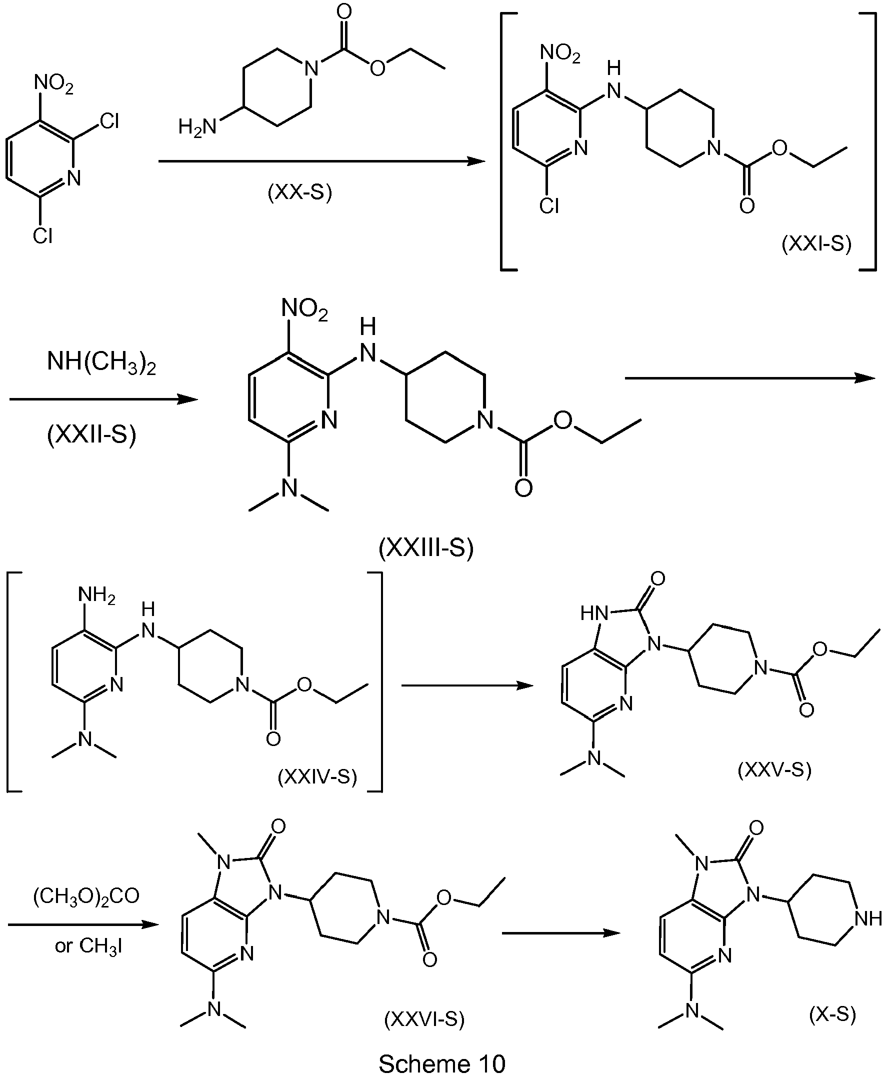

Accordingly, 2,6-dichloro-3-nitro-pyridine, a known compound, is reacted with a compound of formula (XX-S), also known as 4-amino-piperidine-1-carboxylic acid ethyl ester, a known compound; wherein the compound of formula (XX-S) is present in an amount in the range of from about 0.75 to about 2.0 molar equivalents, or any range therein, for example, in an amount in the range of from about 0.95 to about 1.1 molar equivalents, for example in an amount of about 1.0 molar equivalents;
in the presence of an organic or inorganic base such as DIPEA, TEA, pyridine, potassium carbonate or cesium carbonate, for example in the presence of DIPEA, wherein the base is present in an amount in the range of from about 1.0 to about 5.0 molar equivalents, or any range therein, for example, in an amount in the range of from about 2.0 to about 2.5 molar equivalents, for example in an amount of about 2.35 equivalents;
in an organic solvent such as acetonitrile, DMF, DME or THF, for example in acetonitrile; at a temperature in the range of from about 0°C to about 25 °C, or any range therein, for example at about 20°C, to yield the corresponding compound of formula (XXI-S). In an example of the process, the compound of formula (XXI-S) is not isolated.

The compound of formula (XXI-S) is reacted with a compound of formula (XXII-S), also known as dimethylamine, a known compound; wherein the compound of formula (XXII-S) may be present as a gas or in solution, for example in a 10% THF solution or a 60% water solution; wherein the compound of formula (XXII-S) is present in an amount in the range of from about 0.75 to about 2.0 molar equivalents, for example, in an amount in the range of from about 1.0 to about 1.6 molar equivalents, for example in an amount of about 1.15 molar equivalents;
in an organic solvent such as acetonitrile, THF, DCM, methanol, or a mixture of organic solvents, for example in acetonitrile, to yield the corresponding compound of formula (XXIII-S).

The compound of formula (XXIII-S) is reacted with a suitably selected reducing agent such as H₂ gas in the presence of a suitably selected catalyst such as a suitably selected catalyst, for example, with hydrogen gas in the presence of a catalytic amount of Pt/C;
in an organic solvent such as ethyl acetate, ethanol, toluene or THF, for example in ethyl acetate; to yield the corresponding compound of formula (XXIV-S). In an example of the process, the compound of formula (XXIV-S) is not isolated.

The compound of formula (XXIV-S) reacted with a suitably selected reagent such as CDI, N-methylcarbodiimide, phosgene or triphosgene, for example with CDI, wherein the reagent is present in an amount in the range of from about 1.0 to about 2.0 molar equivalents, for example in an amount in the range of from about 1.1 to about 1.5 molar equivalents, for example in an amount of from about 1.25 to about 1.3 molar equivalents; in an organic solvent such as ethyl acetate, THF, for example in ethyl acetate, to yield the corresponding compound of formula (XXV-S).

The compound of formula (XXV-S) is reacted with carbonic acid dimethyl ester, a known compound; wherein the carbonic acid dimethyl ester is present in an amount in the range of from about 1.0 to about 5.0 molar equivalents, for example, in an amount in the range of from about 1.0 to about 3.0 molar equivalents, for example in an amount of about 2.2 molar equivalents;
in the presence of an inorganic base such as K₂CO₃, Na₂CO₃, Cs₂CO₃, for example K₂CO₃; wherein the base is present in an amount in the range of from about 0.75 to about 1.5 molar equivalents, for example, in an amount in the range of from about 0.8 to about 1.25 molar equivalents, for example, the in an amount of about 1.0 molar equivalents;
in an organic solvent such as DMF or DMA, for example in DMF; at a temperature in the range of from about 50°C to about solvent reflux temperature, for example at about solvent reflux temperature, to yield the corresponding compound of formula (XXVI-S).

Alternatively, the compound of formula (XXV-S) is reacted with CH₃I, a known compound, wherein the CH₃I is present in an amount in the range of from about 1.0 to about 3.0 molar equivalents, for example in an amount in the range of from about 1.0 to about 1.2 molar equivalents; in the presence of a base such as KHMDS or NaHMDS; wherein the base is present in an amount in the range of from about 1.0 to about 2.0 molar equivalents, for example in an amount in the range of from about 1.1 to about 1.5 molar equivalents; in an organic solvent such as THF, DMF or methylene chloride, for example THF; to yield the corresponding compound of formula (XXVI-S).

The compound of formula (XXVI-S) is reacted with a suitably selected base such as KOH or NaOH, for example with aqueous KOH; wherein the base is present in an amount in range of from about 5.0 to about 10.0 molar equivalents, for example, in an amount in the range of from about 7.0 to about 9.0 molar equivalents, for example in an amount of about 8.7 molar equivalents; in an organic solvent such as ethanol, methanol or isopropanol, for example in ethanol; to yield the corresponding compound of formula (X-S).

The present invention is further directed to crystalline salts of the compound of formula (I-S), more particularly, crystalline mono-HCI and crystalline sulfate salts of the compound of formula (I-S).
In an embodiment, the present invention is directed to a crystalline mono-HCI salt of the compound of formula (I-S). In another embodiment of the present invention, the crystalline mono-HCl salt of the compound of formula (IS) is a hexahydrate. The HCl salt of the compound of formula (I-S) may be prepared by reacting the compound of formula (I-S) with HCI acid, in a solvent such as water, ethanol or methanol; for example in water.
In an embodiment, the present invention is directed to a crystalline sulfate salt of the compound of formula (I-S). The sulfate salt of the compound of formula (I-S) may be prepared by reacting the compound of formula (I-S) with sulfuric acid, in a solvent such as water, ethanol or methanol.

The crystalline hexhydrate mono-HCl salt of the compound of formula (IS) of the present invention was characterized by their respective X-ray powder diffraction (XRD) patterns utilizing a powder X-ray diffractometer, using a long fine-focus Cu K_{α} radiation source 1.5406Å, 45KV, 40mA; optics of 1° divergence slit; scanning from 3°2θ to 35°2θ at a scan speed of 0.0170°2θ and a scan step time of 10.16 sec.

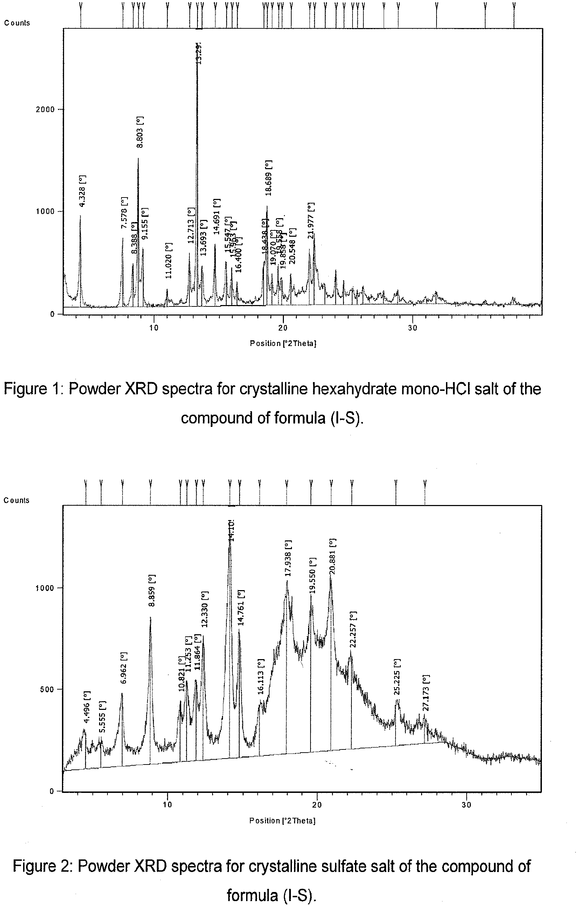

A representative powder XRD spectra for a representative sample of the crystalline hexahydrate mono-HCl salt of the compound of formula (I-S) is shown in Figure 1 which follows here. The crystalline hexahydrate mono-HCl salt of the compound of formula (I-S) may be characterized by its X-ray diffraction pattern, comprising the following peaks:

**Table 1: XRD Peaks, mono-HCl, Hexahydrate Salt of (I-S)**

| **Angle (°2θ)** | **FWHM (°2θ)** | **d-spacing (Å)** | **Relative Intensity (%)** |
|---|---|---|---|
| 4.33 | 0.07 | 20.42 | 31.88 |
| 7.58 | 0.08 | 11.67 | 24.01 |
| 8.39 | 0.10 | 10.54 | 15.06 |
| 8.80 | 0.10 | 10.04 | 52.79 |
| 9.16 | 0.10 | 9.66 | 20.10 |
| 11.02 | 0.08 | 8.03 | 6.00 |
| 12.71 | 0.15 | 6.96 | 18.31 |
| 13.30 | 0.08 | 6.66 | 100.00 |
| 13.69 | 0.12 | 6.47 | 14.02 |
| 14.69 | 0.10 | 6.03 | 21.44 |
| 15.55 | 0.10 | 5.70 | 15.11 |
| 15.96 | 0.10 | 5.55 | 13.36 |
| 16.40 | 0.08 | 5.40 | 7.98 |
| 18.44 | 0.10 | 4.81 | 14.30 |
| 18.69 | 0.12 | 4.75 | 35.05 |
| 19.07 | 0.10 | 4.65 | 10.32 |

In an embodiment, the present invention is directed to a crystalline hexahydrate ono-HCl salt of the compound of formula (I-S) as characterized by its XRD peaks, whose relative intensity is greater than or equal to about 10%, as listed in Table 2 below.

**Table 2: XRD Peaks, mono-HCl, Hexahydrate Salt of (I-S)**

| **Angle (°2θ)** | **FWHM (°2θ)** | **d-spacing (Å)** | **Relative Intensity (%)** |
|---|---|---|---|
| 4.33 | 0.07 | 20.42 | 31.88 |
| 7.58 | 0.08 | 11.67 | 24.01 |
| 8.39 | 0.10 | 10.54 | 15.06 |
| 8.80 | 0.10 | 10.04 | 52.79 |
| 9.16 | 0.10 | 9.66 | 20.10 |
| 12.71 | 0.15 | 6.96 | 18.31 |
| 13.30 | 0.08 | 6.66 | 100.00 |
| 13.69 | 0.12 | 6.47 | 14.02 |
| 14.69 | 0.10 | 6.03 | 21.44 |
| 15.55 | 0.10 | 5.70 | 15.11 |
| 15.96 | 0.10 | 5.55 | 13.36 |
| 18.44 | 0.10 | 4.81 | 14.30 |
| 18.69 | 0.12 | 4.75 | 35.05 |
| 19.07 | 0.10 | 4.65 | 10.32 |

In another embodiment, the present invention is directed to a crystalline hexahydrate mono-HCl salt of the compound of formula (I-S) as characterized by its XRD peaks, whose whose relative intensity is greater than or equal to about 20%, as listed in Table 3, below.

**Table 3: XRD Peaks, mono-HCl, Hexahydrate Salt of (I-S)**

| **Angle (°2θ)** | **FWHM (°2θ)** | **d-spacing (Å)** | **Relative Intensity (%)** |
|---|---|---|---|
| 4.33 | 0.07 | 20.42 | 31.88 |
| 7.58 | 0.08 | 11.67 | 24.01 |
| 8.80 | 0.10 | 10.04 | 52.79 |
| 9.16 | 0.10 | 9.66 | 20.10 |
| 13.30 | 0.08 | 6.66 | 100.00 |
| 14.69 | 0.10 | 6.03 | 21.44 |
| 18.69 | 0.12 | 4.75 | 35.05 |

The crystalline sulfate salt of the compound of formula (I-S) of the present invention was characterized by their respective X-ray powder diffraction (XRD) patterns utilizing a powder X-ray diffractometer, using a long fine-focus Cu K_{α} radiation source 1.5406Å, 45KV, 40mA; Optics of 0.25° divergence slit; scanning from 3°2θ to 40°2θ at a scan speed of 0.0170°2θ and a scan step time of 19.68 sec.

A representative powder XRD spectra for a representative sample of the crystalline sulfate salt of the compound of formula (I-S) is shown in Figure 2 which follows here. The crystalline sulfate salt of the compound of formula (I-S) may be characterized by its X-ray diffraction pattern, comprising the following peaks:

**Table 4: XRD Peaks, Sulfate Salt of (I-S)**

| **Angle (°2θ)** | **FWHM (°2θ)** | **d-spacing (Å)** | **Relative Intensity (%)** |
|---|---|---|---|
| 4.50 | 0.20 | 19.66 | 14.28 |
| 5.56 | 0.54 | 15.91 | 10.02 |
| 6.96 | 0.12 | 12.67 | 28.72 |
| 8.86 | 0.13 | 9.98 | 58.69 |
| 10.82 | 0.20 | 8.18 | 23.82 |
| 11.25 | 0.23 | 7.86 | 31.50 |
| 11.86 | 0.20 | 7.46 | 31.97 |
| 12.33 | 0.15 | 7.18 | 48.66 |
| 14.10 | 0.08 | 6.28 | 100.00 |
| 14.76 | 0.20 | 6.00 | 48.17 |
| 16.11 | 0.27 | 5.50 | 20.13 |
| 17.94 | 0.27 | 4.95 | 66.31 |
| 19.55 | 0.20 | 4.54 | 58.68 |
| 20.88 | 0.23 | 4.25 | 65.80 |
| 22.26 | 0.20 | 3.99 | 36.52 |
| 25.22 | 0.23 | 3.53 | 15.97 |

In an embodiment, the present invention is directed to a crystalline sulfate salt of the compound of formula (I-S) as characterized by its XRD peaks, whose relative intensity is greater than or equal to about 20%, as listed in Table 5, below.

**Table 5: XRD Peaks, Sulfate Salt of (I-S)**

| **Angle (°2θ)** | **FWHM (°2θ)** | **d-spacing (Å)** | **Relative Intensity (%)** |
|---|---|---|---|
| 6.96 | 0.12 | 12.67 | 28.72 |
| 8.86 | 0.13 | 9.98 | 58.69 |
| 10.82 | 0.20 | 8.18 | 23.82 |
| 11.25 | 0.23 | 7.86 | 31.50 |
| 11.86 | 0.20 | 7.46 | 31.97 |
| 12.33 | 0.15 | 7.18 | 48.66 |
| 14.10 | 0.08 | 6.28 | 100.00 |
| 14.76 | 0.20 | 6.00 | 48.17 |
| 16.11 | 0.27 | 5.50 | 20.13 |
| 17.94 | 0.27 | 4.95 | 66.31 |
| 19.55 | 0.20 | 4.54 | 58.68 |
| 20.88 | 0.23 | 4.25 | 65.80 |
| 22.26 | 0.20 | 3.99 | 36.52 |

The present invention further comprises pharmaceutical compositions containing a product prepared according to any of the processes described herein with a pharmaceutically acceptable excipient. Pharmaceutical compositions containing one or more of the compounds of the invention described herein as the active ingredient can be prepared by intimately mixing the compound or compounds with a pharmaceutical excipient according to conventional pharmaceutical compounding techniques. The excipient may take a wide variety of forms depending upon the desired route of administration (e.g., oral, parenteral). Thus for liquid oral preparations such as suspensions, elixirs and solutions, suitable excipients and additives include water, glycols, oils, alcohols, flavoring agents, preservatives, stabilizers or coloring agents; for solid oral preparations, such as powders, capsules and tablets, suitable excipients and additives include starches, sugars, diluents, granulating agents, lubricants, binders or disintegrating agents. Solid oral preparations may also be coated with substances such as sugars or be enteric-coated so as to modulate major site of absorption. For parenteral administration, the excipient will usually consist of sterile water and other ingredients may be added to increase solubility or preservation. Injectable suspensions or solutions may also be prepared utilizing aqueous excipients along with appropriate additives.

To prepare the pharmaceutical compositions of this invention, one or more products of the present invention as the active ingredient is intimately admixed with a pharmaceutical excipient according to conventional pharmaceutical compounding techniques, which excipient may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable excipients and additives include water, glycols, oils, alcohols, flavoring agents, preservatives or coloring agents; for solid oral preparations such as, for example, powders, capsules, caplets, gelcaps and tablets, suitable excipients and additives include starches, sugars, diluents, granulating agents, lubricants, binders or disintegrating agents. Because of their ease in administration, tablets and capsules represent the most advantageous oral dosage unit form, in which case solid pharmaceutical excipients are obviously employed. If desired, tablets may be sugar coated or enteric coated by standard techniques. For parenterals, the excipient will usually comprise sterile water, through other ingredients, for example, for purposes such as aiding solubility or for preservation, may be included. Injectable suspensions may also be prepared, in which case appropriate liquid excipients or suspending agents may be employed. The pharmaceutical compositions herein will contain, per dosage unit, e.g., tablet, capsule, powder, injection or teaspoonful, an amount of the active ingredient necessary to deliver an effective dose as described above. The pharmaceutical compositions herein will contain, per unit dosage unit, e.g., tablet, capsule, powder, injection, suppository or teaspoonful, of from about 0.01-1000 mg or any range therein, and may be given at a dosage of from about 0.01-300 mg/kg/day, or any range therein, for example from about 0.5-100 mg/kg/day, or any range therein, for example from about 1.0-50 mg/kg/day, or any range therein. The dosages, however, may be varied depending upon the requirement of the patients, the severity of the condition being treated and the compound being employed. The use of either daily administration or post-periodic dosing may be employed.

For example these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical excipient, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.01 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The methods described in the present invention may also be carried out using a pharmaceutical composition comprising any of the compounds as defined herein and a pharmaceutically acceptable excipient. The pharmaceutical composition may contain between about 0.01 mg and 1000 mg of the compound, or any range therein; for example about 1.0 to 500 mg of the compound, or any range therein, for example, about 10 to about 500 mg of the compound, or any rage therein, and may be constituted into any form suitable for the mode of administration selected. Excipients include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert excipient such as ethanol, glycerol or water. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate or sodium chloride. Disintegrators include, without limitation, starch, methyl cellulose, agar, bentonite or xanthan gum.

The liquid forms in suitably flavored suspending or dispersing agents such as the synthetic and natural gums, for example, tragacanth, acacia or methyl-cellulose. For parenteral administration, sterile suspensions and solutions are desired. Isotonic preparations which generally contain suitable preservatives are employed when intravenous administration is desired.

The compound of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds of the present invention may also be delivered by the use of monoclonal antibodies as individual excipients to which the compound molecules are coupled. The compounds of the present invention may also be coupled with soluble polymers as targetable drug excipients. Such polymers can include polyvinylpyrrolidone, pyran copolymer, polyhydroxypropylmethacrylamidephenol, polyhydroxy-ethylaspartamidephenol, or polyethyl eneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyeric acid, polyorthoesters, polyacetals, polydihydropyrans, polycyanoacrylates and cross-linked or amphipathic block copolymers of hydrogels.

To prepare a pharmaceutical composition of the present invention, a product prepared according to any of the processes described herein as the active ingredient is intimately admixed with a pharmaceutical excipient according to conventional pharmaceutical compounding techniques, which excipient may take a wide variety of forms depending of the form of preparation desired for administration (e.g. oral or parenteral). Suitable pharmaceutically acceptable excipients are well known in the art. Descriptions of some of these pharmaceutically acceptable excipients may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

The following Examples are set forth to aid in the understanding of the invention, and are not intended and should not be construed to limit in any way the invention set forth in the claims which follow thereafter.

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, or other form.

Examples 1 through 11 describe recipes / procedures for the synthesis of the title compounds. Several batches of the said compounds were prepared according to the recipes / procedures as described below. Physical properties listed at the end of said experimental descriptions correspond to physical properties measured for a representative sample of the compound prepared in the experimental description which precedes it.

### Example 1: Preparation of 4-(6-Dimethylamino-3-nitro-pyridin-2-ylmethyl)-piperidine-1-carboxylic acid ethyl ester

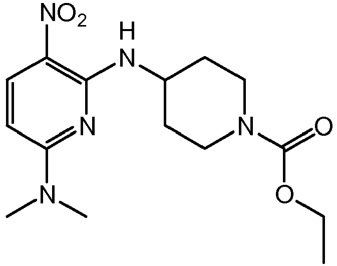

Acetonitrile (1.002 L) was added to a 3L flask. To the flask was then added 2,6-dichloro-3-nitropyridine (188.26 g, 0.878 mo) and DIPEA (353 mL), 2.007 mol) and the reaction mixture cooled to 0-5°C. To the resulting mixture was added 4-amino-1-piperidine carboxylic acid ethyl ester (150.0 g, 0.836 mol), dropwise over 10 minutes. The resulting susenstion was agitated at 0-5°C for one hour and then allowed to warm to room temperature over about 90 min. The resulting mixture was agitated at room temperature overnight. To the resulting mixture was added dropwise over about 20 minutes, dimethylamine (660 mL, 1.32 mol) in 2M THF. The resulting mixture was separated into two equal portions. To each portion was then added distilled water (760 mL) over about 15 min. The resulting slurry was agitated for 4 to 24 hours, then filtered to yield a yellow solid. A1:1 mixture of acetonitrile:water (400 mL) was used to rinse the flask and filtercake. The filtercake was dried at 50°C to yield the title compound as a yellow solid.

### Example 2: Preparation of 4-(5-Dimethylamino-2-oxo-1,2-dihydro-imidazo[4,5-b]pyridin-3-yl)-piperidine-1-carboxylic acid ethyl ester

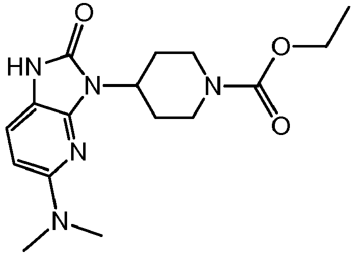

To a 1 Lvessel was added 4-(6-Dimethylamino-3-nitro-pyridin-2-ylmethyl)-piperidine-1-carboxylic acid ethyl ester (120.0 g, 0.355 mol) and ethyl acetate (600.0 mL) and the resulting mixture stirred. To the resulting slurry was added Englehard Catalyst 43191 (1 % Pt/C, 40.42% water, 32.8 g) and the reaction vessel was purged with nitrogen (3x) and then hydrogen. The reaction mixture was pressurized 40 psi and heated at 45°C until no more hydrogen was consumed. The reaction mixture was then purged with nitrogen (3x). The resulting suspension was filtered through a CELITE^{®} pad (120g, 2" height), which was washed with ethyl acetate (120 mL, 2X). The filtrate was washed with saturated brine (once with 300 mL, once with 150 mL). The ethyl acetate solvent (∼300 mL) was removed under vacuum (40°C bath temperature) and the resulting residue was stored at 4°C overnight. The residue was then added to a 1 L flask and stirred. To the mixture was then added 1,1'-carbonyldiimidazole (71.9 g, 0.430 mol) in six equal portions over time and the reaction mixture was stirred at room temperature for one hour, then at 0-5°C for 3 hours. The resulting suspension was filtered, the filtercake washed with n-heptane (120 mL) and the resulting solids dried at 50°C under full vacuum overnight to yield the title compound as a solid.

### Example 3: Preparation of 4-(5-Dimethylamino-1-methyl-2-oxo-1,2-dihydro-imidazo[4,5-b]pyridin-3-yl)-piperidine-1-carboxylix acid ethyl ester

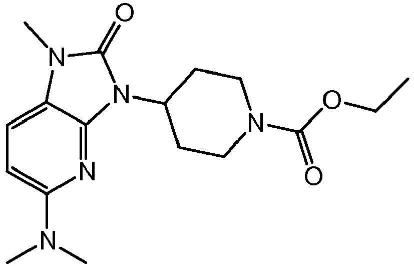

To a 3L round bottom flask was added 4-(5-dimethylamino-2-oxo-1,2-dihydro-imidazo[4,5-b]pyridin-3-yl)-piperidine-1-carboxylic acid ethyl ester (100.0 g, 0.267 mol), potassium carbonate (36.0 g, 0.255 mol), DMF (500 mL) and dimethyl carbonate (50 mL, 0.587 mol). The resulting mixture was heated to reflux for about 1 hour. The reaction mixture was then cooled to room temperature. To the resulting mixture was then added, dropwise over about 15 min, water (800 mL) and the resulting suspension cooled to 0-5°C using an ice water bath. The cold suspension was filtered and the filter cake washed with water (300 mL). The filter cake was allowed to air dry for 30 minutes, then dried in vacuo at 25°C under full vacuum overnight, to yield the title compound as a red solid.

### Example 4: Preparation of 5-Dimethylamino-1-methyl-3-piperidin-4-yl-1,3-dihydro-imidazo[4,5-b]pyridin-2-one

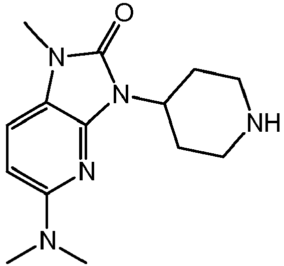

A vessel was charged with KOH (3.085 kg) and water (2.2 L) and the exothermic mixture cooled in an ice bath. To the mixture was then added ethanol (8.8 L) and the mixture again cooled with an ice bath.

To a 22 L round bottom flask (RBF) equipped with mechanical agitation, heating mantle and a thermocouple, was added the mixture prepared above and 4-(5-dimethylamino-1-methyl-2-oxo-1,2-dihydro-imidazo[4,5-b]pyridin-3-yl)-piperidine-1-carboxylic acid ethyl ester (2.20 kg) and resulting suspension was heated to reflux (84-85°C) for about 10.5 hours The reaction mixture was then cooled to room temperature overnight. The reaction mixture, which had formed a suspension, was transferred to a 50 L phase separator. Water (6.6 L) was added and the solids dissolved. The mixture was agitated for 10 minutes, then allowed to split for 30 minutes. The aqueous layer was removed, and discarded. The dark red organic layer was transferred to a 20 L rotary evaporator and the ethanol/water solvents removed under heat and vacuum to yield a suspension. The suspension was filtered using a stainless steel filter with filter paper to yield wet solid. The solid was charged along with water (4.4 L) in two 12L Morton flasks. The resulting suspensions were agitated overnight. The suspensions were filtered on two sintered glass funnels (type D, 10-20 µm), the filter cakes washed with 2 x 2.2 L and 1 x 1 L each. The resulting solids were air dry on the funnel for 30 minutes. The wet cake was then transferred to a vacuum oven for drying at 50°C and 45 Torr overnight w/nitrogen bleed, to yield the title compound as a pink/rose solid.

### Example 5: Preparation of 1-Methanesulfonyl-piperidin-4-one

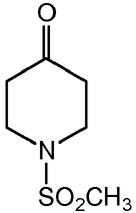

To a 20 gallon reactor vessel was added acetonitrile (20 L) and with stirring, 4-piperidone monohydrate hydrochloride (4 kg, 25.5 mol) and solid NaHCO₃ (7.5 kg, 89.25 mol). The reaction mixture was then heated to 65°C. Over about 5-6 hours, CH₃SO₂Cl (2.96 L, 4.38 kg, 38.25 mol) was metered into the reaction mixture. The reaction mixture was then stirred for 1 hour at 65°C, then cooled to room temperature. The resulting mixture was filtered and the filter cake washed with acetonitrile (4 L). The acetonitrile mother liquors were collected, returned to the reaction vessel and concentrated to near dryness. The resulting residue was cooled to room temperature. To the residue was added isopropyl acetate (8 L) to yield a suspension, which was stirred for about 30 min. Additional isopropyl acetate (12 L) was added and the mixture stirred for about 30 minutes. Two portions of heptane (12 L each) were added to the reaction mixture, with about 30 minutes stirring after each addition. The resulting mixture was filtered to yield the title compound as a solid, which was allowed to air dry.

### Example 6: Preparation of 1-Methanesulfonyl-3-(4-trifluoromethyl-benzoyl)-piperidin-4-one

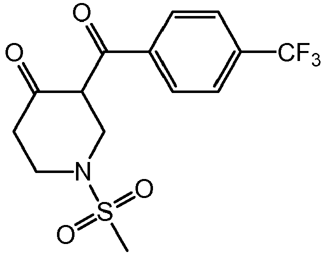

To a 5L RB 3-necked flask equipped with a mechanical stirrer, addition funnel, and a Dean-Stark trap with a condenser was added toluene (3L), 4-methylpiperidine (128.2 mL. 1.04 mol), N-methanesulfonyl piperidone (200.0 g, 0.99 mol) and p-TsOH • 5 H₂O (5% mol, 9.51 g, 0.05 mol). The resulting mixture was heated to reflux and the water was removed azeotropically by a Dean-Stark trap. After about 5-6 hours, no more H₂O was distilled off from the reaction mixture and about 20 mL of H₂O was collected. The reaction mixture was cooled to -20°C in a chilled IPA bath. To the reaction mixture was then added TEA (151.9 mL, 1.09 mol) over about 15 minutes, followed by slow addition of a solution of 4-trifluoromethylbenzoyl chloride (216.90 g, 1.04 mol) in toluene (160 mL), over about 90 minutes. The internal reaction temperature was maintained at about -20°C by adjusting the addition rate. After addition, the reaction suspension was stirred at -20°C for 2h, and then warmed slowly to room temperature over about 4 hours. The resulting brown suspension was stirred at room temperature for about 12-18 hours. The reaction mixture was then diluted with isopropyl acetate (0.5 L) and the reaction quenched with 1 N aqueous HCl (1 L). The resulting mixture was stirred at room temperature for 1 hour, then transferred to a separatory funnel and further diluted with isopropyl acetate (0.5 L) and water (1 L). The resulting mixture was stirred for 5 minutes and then left to stand for about 1 hour. The layers were separated, the organic layer containing some fine solids was washed with water (1 L, 2X), concentrated to almost dryness on a roto-evaporator to yield a thick slurry. The slurry was diluted with MTBE (1 L) and stirred overnight. The resulting solid product was collected by vacuum filtration, washed with MTBE (0.3 L), and air-dried overnight to yield the title compound as a solid.

### Example 7: Preparation of 4-Methanesulfonyl-3-(4-trifluoromethyl-phenyl)-4,5,6,7-tetrahydro-1H-pyrazolo[4,3-b]pyridine

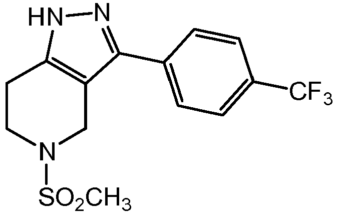

To a 22 L RB 4-necked flask equipped with a mechanical stirrer and addition funnel was charged with 95% ethanol (4.5 L) and 1,3-diketone (868.4 g, 1.87 mol) and the resulting mixture was stirred for 15 minutes. To the resulting mixture was added water (2 L) and a solution of hydrazine HCl (130 g, 1.87mol) in water (800 mL). To the resulting suspension at room temperature was added 1 N aqueous NaOH (2.06 L, 2.06 mol) slowly over 20 minutes. A slight exotherm was observed and the resulting suspension was stirred at room temperature for about 1 hour. A solid product was then collected by vacuum filtration, washed with a 1:1 mixture of 95% EtOH/H₂O (2 L). The solid product was air-dried for 3 days.

The solid was suspended in MTBE (2.7 L) and the resulting mixture was stirred under reflux for 45 minutes, then at room temperature for about 6 hours. The solid product was then collected by vacuum filtration, washed with MTBE (100 mL) and then air-dried overnight (ca 18 hours) to yield the title compound as a solid.

### Example 8: Preparation of 5-Methanesulfonyl-1(R)-oxiranylmethyl-3-(4-trifluoromethyl-phenyl)-4,5,6,7-tetrahydro-1H-pyrazolo[4,3-c]pyridine

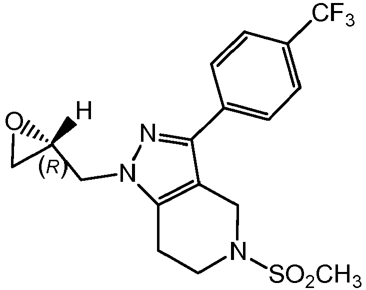

A 22 L three neck flask equipped with a mechanical stirrer, argon inlet/outlet adaptor, thermometer and stopper was charged with DMF (5.4 L), 5-methanesulfonyl-3-(4-trifluoromethyl-phenyl)-4,5,6,7-tetrahydro-1H-pyrazolo[4,3-c]pyridine (550 g, 1.56 mol) and (S)-glycidyl 3-nitrobenzenesulfonate. The resulting mixture was cooled to 8°C in an ice bath. To the reaction mixture was then added, with stirring cesium carbonate (520 g, 1.59 mol). The ice bath was removed within 5-10 minutes of the addition and the resulting mixture was stirred for about 22 hours at ambient temperature under argon. Additional cesium carbonate (132 g, 0.405 mol) was added and the resulting mixture was stirred for about 2-4 hours, then cooled in an ice bath. To the resulting mixture was then slowly added cold deionized water (10.8 L). athe ice bath was removed and the resulting mixture stirred at ambient temperature for about 1-2 hhours. The reaction mixture was then filtered, the filtercake washed with deionized water (2 L) and air dried to yield the title compound as a solid.

A sample of the solid was further recrystallized from a solution of isopropanol/isopropyl acetate/heptane according to two alternate methods as outlined below.

Recrystallization A: 5-Methanesulfonyl-1-oxiranylmethyl-3-(4-trifluoromethyl-phenyl)-4,5,6,7-tetrahydro-1H-pyrazolo[4,3-c]pyridine (54.5 g) was stirred in isopropanol (600 mL) at reflux to yield a clear solution. To the resulting mixture was then added ispropyl acetate (100-150 mL) with continued heating to complete dissolution. To the resulting mixture was added n-heptane (200 mL) and the resulting reddsh-brown mixture allowed to cool to room temperature. The resulting precipitate was filtered and washed with a cold solution of 20% n-heptane/isopropanol to yield a solid, which was air dried to yield the title compound as a tan colored solid.

Recrsytallization B: 5-Methanesulfonyl-1-oxiranylmethyl-3-(4-trifluoromethyl-phenyl)-4,5,6,7-tetrahydro-1 H-pyrazolo[4,3-c]pyridine (115 g) was slurried in isopropanol (550 mL). The resulting mixture was heated to reflux. To the mixture was then slowly added isopropyl acetate (200 mL) to yield a clear solution. The solution was allowed to cool to oom temperature, resulting in the formation of a precipitate, The precipitate was filtered, washed with cold isopropanol (about 50 mL) and then air dried to yield the title compound as a light tan solid.

### Example 9: Preparation of 5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]propyl}-piperidin-4-yl)-1-methyl-1,3-dihydro-imidazo[4,5-b]pyridin-2-one

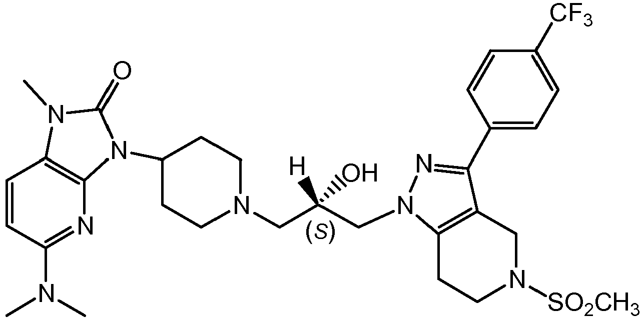

A 50 L reactor vessel was charged with ethanol (34 L), 5-dimethylamino-1-methyl-3-piperidin-4-yl-1,3-dihydro-imidazo[4,5-b]pyridin-2-one (856 g, 3.11 mol) and 5-methanesulfonyl-1(R)-oxiranylmethyl-3-(4-trifluoromethyl-phenyl)-4,5,6,7-tetrahydro-1H-pyrazolo[4,3-c]pyridine (1457 g, 3.11 mol). The resulting pink recation mixture was heated at reflux for about 6 hours, then allowed to cool to room temperature and stirred at ambient temperature for an additional 1 to 2 hours. The reaction mixture was filtered and the filter cake washed with cold (6-8°C) ethanol, then air dried to yield the title compound as a pink solid.

### Example 10: Recrystallization of 5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one

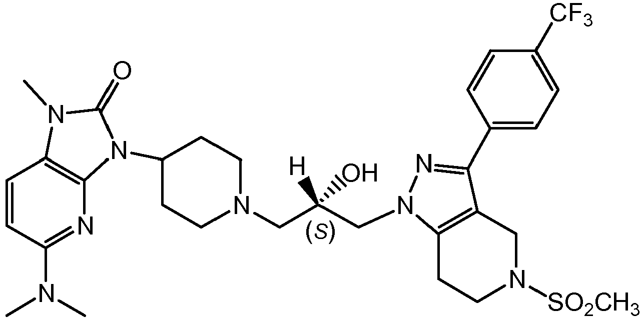

5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one (50 g, 91.3 wt%, 0.067 mol) was slurried in a mixture ethanol (95%, 600 mL) and acetone (200 mL). The resulting mixture was heated to reflux, with stirring. After about 10-15 minutes, additional acetone (in 100 mL increments) was added, followed by about 10-15 minutes of stirring, until all of the solid was observed to dissolve. A total of about 800 mL of acetone was added. The volume of the resulting mixture was reduced to approximately 1200 mL, then the heat was removed and the resulting brown solution allowed to stand at ambient temperature. A solid was observed to precipitate out of solution on standing. The resulting mixture was filtered, the filter cake washed with cold 95% ethanol (about 100 mL) and air dried to yield the title compound (in two crops) as a light pink solid.

### Example 11: Preparation of HCl Hexhydrate Salt of 5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]propyl}-piperidin-4-yl)-1-methyl-1,3-dihydro-imidazo[4,5-b]pyridin-2-one

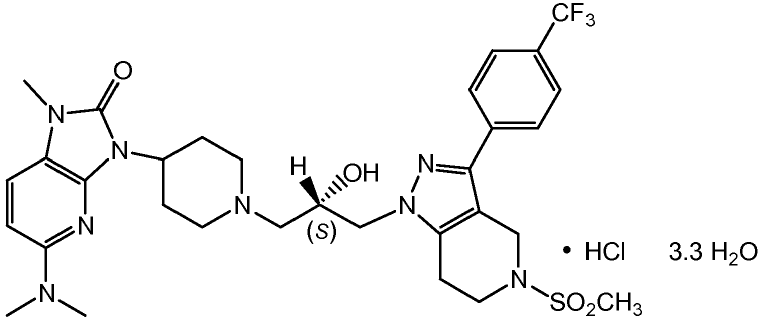

5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one (56 g, 0.0827 mol) was slurried in dristilled water (1 L) with heating. To the resulting mixture was added 1 N HCl (160 mL, 0.16 mol). On addition of the acid, most of the solid dissolved and then a pink solid prepcipitated. The resulting mixture was heated, with stirring, to reflux, until all of the solid was observed to dissolve. The resulting solution was hot-filtered through a cartridge / filter aid, then washed with hot water (200 mL). The resulting solution was allowed to cool to room temperature, with stirring, overnight. A solid was observed to precipiate out of solution. The resulting mixture was filtered, the filter cake washed with cold water (300 mL) and air dried to yield the title compound as a pink solid.

Example 12 through 25 were completed as described, with general procedures and parameters as listed below.

### General Experimental Parameters

Unless otherwise noted, the following general experimental parameters and procedures were utilized in connection with Example LJ1 through LJ14 which follow herein.

Melting points (mp) were determined on a Electrothermal Mel-Temp apparatus. Mass Spectra was obtained on a Hewlett Packard LC/MSD instrument. All HPLC runs were recorded on a Hewlett Packard 1100 Series Instrument using a Zorbax Eclipse XDB-C8, 5 µm, 4.6 x 150 mm column, λ at 220 nm & 254 nm, and flow rate at 0.75 ml/min with 0.1 % TFA. Gradient conditions:
a) 8.0 min 1% - 99% acetonitrile
b) 10.0 min 99% acetonitrile
c) 10.5 min 1% acetonitrile
d) 12.0 min 1% acetonitrile
Total run time 12 min

Chiral HPLC runs were recorded using a Chiralpak AD, 4.6 x 250 mm column, λ at 220 & 254 nm, flow rate at 1ml/min with 95/5 EtOH/hexane.

NMR spectra were measured in the indicated solvent with TMS as the internal standard on a Bruker 400 MHz instrument.

### Example 12: Preparation of 4-(6-Chloro-3-nitro-pyridin-2-ylamino)-piperidine-1-carboxylic acid ethyl ester

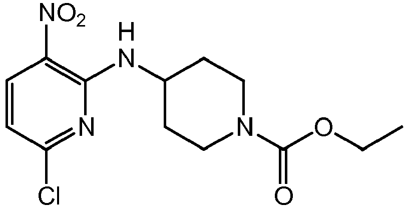

In a dried, 1-neck, 500 ml round-bottom flask with a magnetic stir bar, 2,6-dichloro-3-nitropyridine (97.0 g, 0.4625 mol) and K₂CO₃ (66.0 g, 0.4775 mol) were mixed in DMF (150 ml) then chilled to 0°C. Using an addition funnel, ethyl-4-amino-1-piperidine carboxylate (74 ml, .4227 mol) in DMF (50 ml) was slowly added to the reaction mixture (to minimize the amount of exotherm in the reaction). The reaction was allowed to stir up to room temperature for 18 h, then treated with H₂O (400 ml). The aqueous layer was washed with EtOAc (3 x 500 ml) and extracted. The combined organic layer was dried with MgSO₄, filtered and concentrated to a yellow solid. The yellow solid was purified by filtration chromatography (silica gel column: 14 cm OD, 10 cm in height and eluting with CH₂Cl₂ increasing to 95/5 CH₂Cl₂/MeOH.). The desired fractions containing the title compound were combined. Other fractions containing impurities in a mixture with the title compound were combined and filtration chromatography was repeated again to recover additional compound. The title compound was isolated as residue.

MS (electrospray), *m*/*z* for C₁₃H₁₇ClN₄O₄ (M⁺ + K) 363.0
¹H NMR (400 M*Hz*, CDCl₃) δ 8.37 - 8.35 (d, *J* = 8.59 *Hz*, 1H), 8.28 - 8.26 (br d, *J* = 7.58 *Hz,* 1 H), 6.64 - 6.62(d, *J* = 8.59 *Hz,* 1 H), 4.44 - 4.31 (m, 1H), 4.18 - 4.13 (br q, J = 7.07, 14.15 *Hz*, 4H), 3.09 - 3.03 (t, *J* = 12.13 Hz, 2H), 2.1 - 2.06 (br d, *J* = 13.14 *Hz*, 2H), 1.58 - 1.48 (m, 2H), 1.29 - 1.26 (t, *J* = 7.07 *Hz,* 3H).
HPLC retention time: 9.8 min

### Example 13: Preparation of 4-(6-Dimethylamino-3-nitro-pyridin-2-ylamino)-piperidine-1-carboxylic acid ethyl ester

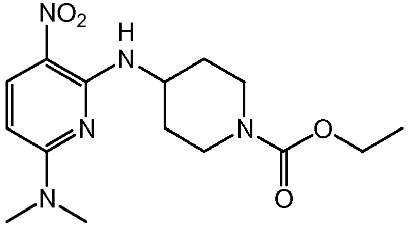

In a dried, 1-neck 1 L round-bottom flask with a magnetic stir bar, 4-(6-chloro-3-nitro-pyridin-2-ylamino)-piperidine-1-carboxylic acid ethyl ester (69.45 g, 0.2112 mol) was slurried in MeOH/CH₂Cl₂ (110 ml/20 ml). Using an addition funnel, 2 M (CH₃)₂NH in THF (325 ml, 0.6250 mol) was slowly added to the slurry mixture. The slurry reaction mixture was mildly exothermic and developed into a clear solution. After stirring overnight at room temperature for 18 h, the reaction mixture turned into a yellow slurry solution. The resulting mixture was concentrated to a yellow solid, then diluted in CH₂Cl₂ (400 ml) and washed with saturated NaHCO₃ solution (2 x 300 ml). The extracted organic layer was dried with Na₂SO₄, filtered and concentrated to yield the title compound as a yellow solid. The product was used in the next step without further purification.
MS (electrospray), *m*/*z* for C₁₅H₂₃N₅O₄ (M⁺ + Na) 360.1
¹H NMR (400 M*Hz*, CDCl₃) δ8.75 - 8.74 (d, *J* = 7.07 *Hz*, 1H), 8.17 - 8.19 (d, *J* = 9.35 *Hz,* 1 H), 5.96 - 5.98(d, *J* = 9.60 *Hz,* 1H), 4.30 - 4.21 (m, 1H), 4.17 - 4.12 (q, *J* = 7.07, 14.15 *Hz*, 2H), 4.05 - 4.07 (br d, *J* = 9.85 *Hz*, 2H), 3.18 (s, 6H), 3.10 - 3.04 (br t, *J* = 11.12 *Hz*, 2H), 2.09 - 2.06 (br d, *J* = 11.12 *Hz*, 2H), 1.61 - 1.52 (m, 2H), 1.29 - 1.25 (t, *J* = 7.33 *Hz*, 3H).
HPLC retention time: 9.19 min

### Example 14: Preparation of 4-(3-Amino-6-dimethylamino-pyridin-2-ylamino)-piperidine-1-carboxylic acid ethyl ester

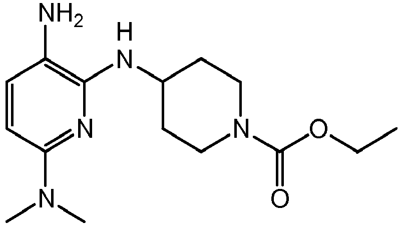

In a 2 L hydrogenation flask, 4-(6-dimethylamino-3-nitro-pyridin-2-ylamino)-piperidine-1-carboxylic acid ethyl ester (71.3 g, .2112 mol) in EtOH (500 ml) and EtOAc (150 ml) was carefully treated with 10% Pd/C catalyst (9.75 g) under N₂ (g) then hydrogenated overnight for 18 h maintaining pressure at 30-35 psi. The catalyst was filtered through a pad of CELlTE^{®} and then washed with EtOH/EtOAc (100/100 ml). The resulting dark filtrate was concentrated via rotovap to yield the title compound as a dark solid. The product was immediately used in the next step without further purification.
MS (electrospray), *m*/*z* for C₁₅H₂₅N₅O₂ (M⁺ + H) 308.1
HPLC retention time: 6.56 min

### Example 15: Preparation of 4-(5-Dimethylamino-2-oxo-1,2-dihydro-imidazo[4,5-b]pyridin-3-yl)-piperidine-1-carboxylic acid ethyl ester

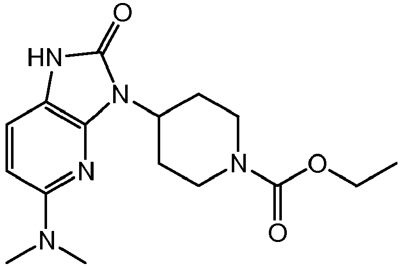

In a dried, 1-neck, 1 L round bottom flask with a magnetic stir bar, 4-(3-amino-6-dimethylamino-pyridin-2-ylamino)-piperidine-1-carboxylic acid ethyl ester (64.94 g, 0.2113) in THF (600 ml) was treated with CDI (38.0 g, 0.2343 mol) in 4 portions. A mild exotherm was observed after addition was complete. The reaction mixture was refluxed at 98°C for 24 h then stirred at room temp for another 36 h. The dark reaction mixture was concentrated via rotovap to yield a dark solid, which was then slurried in EtOAc (1 L). The solids were filtered to yield the title compound as a residue. The filtrate was washed with saturated NaHCO₃ (200 ml) and NaCl (200 ml) solution. The extracted organic layer was dried with Na₂SO₄, filtered and concentrated to yield additional compound. The product from the two isolations was combined and slurried in Et₂O (1 L) for 2 h, then filtered to yield the title compound as a solid. This material was used in the next step without further purification.
MS (electrospray), *m*/*z* for C₁₆H₂₃N₅O₃ (M⁺ - H) 332.1
¹H NMR (400 M*Hz*, CDCl₃) δ8.85 (s, 1 H), 7.16 - 7.13 (d, *J* = 8.59 *Hz*, 1H), 6.16 - 6.14 (d, *J* = 8.34 *Hz*, 1H), 4.51 - 4.43 (m, 1 H), 4.36 - 4.29 (br d, *J* = 27.8 *Hz*, 2H), 4.19 - 4.13 (q, *J* = 7.07, 14.15 *Hz*, 2H), 3.02 (s, 6H), 2.89 (br s, 2H), 2.76 - 2.66 (m, 2H), 1.80 - 1.77 (d, *J* = 11.84 *Hz*, 2H), 1.30 - 1.26 (t, *J* = 7.07 *Hz,* 3H).
HPLC retention time: 6.88 min

### Example 16: Preparation of 4-(5-Dimethylamino-1-methyl-2-oxo-1,2-dihydro-imidazo[4,5-b]pyridin-3-yl)-piperidine-1-carboxylic acid ethyl ester

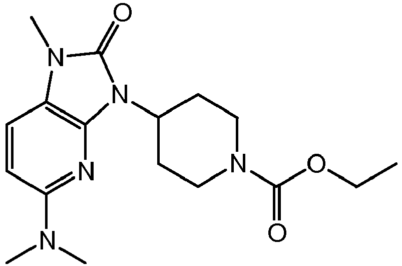

In a dried, 1-neck, 1 L round bottom flask with a magnetic stir bar, 4-(5-Dimethylamino-2-oxo-1,2-dihydro-imidazo[4,5-b]pyridin-3-yl)-piperidine-1-carboxylic acid ethyl ester (66.2 g, 0.1986 mol) in THF (600ml) was treated with KHMDS (46.0 g, 0.2191 mol) in 4 portions to form a slurry mixture. Using an addition funnel, CH₃I (25 ml, 0.4016 mol) was slowly added to the reaction mixture (to minimize the amount of exotherm in the reaction) and the resulting mixture was stirred at room temperature for 1 hour. The reaction mixture was then concentrated to an oily solid, which was then diluted into EtOAc (400 ml) and washed with saturated NaHCO₃ solution (400 ml). Thus, the aqueous layer was extracted based on volume (400 ml) and washed with EtOAc (2 x 100 ml). All the organic layers were combined, dried with Na₂SO₄, filtered and concentrated to yield an oily black solid. The solid was diluted in minimal CH₂Cl₂ then purified by filtration chromatography (silica gel column: 14 cm OD, 10 cm in height and eluting with 4/1 EtOAc/hexane). The desired fractions containing the title compound were combined to yield the title compound as a light brown solid. The solids were slurried overnight in 1/1 mixture diethyl ether/hexane (175 ml/ 175 ml), filtered and dried to yield the title compound as a residue.
MS (electrospray), *m*/*z* for C₁₇H₂₅N₅O₃ (M⁺ + Na) 370.2
¹H NMR (400 M*Hz*, CDCl₃) δ7.04 - 7.02 (d, *J* = 8.34 *Hz*, 1 H), 6.17 - 6.15 (d, *J* = 8.34 *Hz,* 1 H), 4.52 - 4.44 (m, 1 H), 4.35 - 4.28 (br d, *J* = 27.8 *Hz,* 2H), 4.18 - 4.13 (q, *J* = 7.33, 14.40 *Hz*, 2H), 3.34 (s, 3H), 3.02 (s, 6H), 2.88 (br s, 2H), 2.73 - 2.62 (m, 2H), 1.77 - 1.74 (br d, *J* = 11.62 *Hz*, 2H), 1.29 - 1.25 (t, *J* = 7.07 *Hz*, 3H).
HPLC retention time: 7.92 min

### Example 17: Preparation of 5-Dimethylamino-1-methyl-3-piperidin-4-yl-1,3-dihydro-imidazo[4,5-b]pyridin-2-one

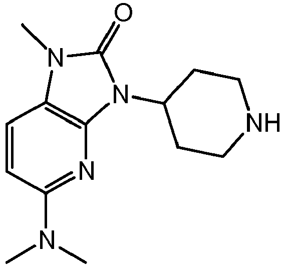

In a dried, 1-neck, 1 L round bottom flask with a magnetic stir bar, 4-(5-dimethylamino-1-methyl-2-oxo-1,2-dihydro-imidazo[4,5-b]pyridin-3-yl)-piperidine-1-carboxylic acid ethyl ester was diluted in EtOH (250 ml) / 20% NaOH (250 ml), then heated to 90°C for 36 h. The reaction mixture was cooled to room temperature and ½ volume was removed via rotovap. The resulting mixture was transferred to a separatory funnel, treated with EtOAc (400 ml) and washed with H₂O (100 ml). The extracted aqueous layer was washed with EtOAc (3 x 100 ml). The combine organic phase was dried with Na₂SO₄, filtered and concentrated to yield a solid. The solid was slurried in Et₂O (200 ml) for 2 h, then filtered to yield the title compound as a pink solid.
MS (electrospray), *m*/*z* for C₁₄H₂₁N₅O (M⁺ + H) 376.3
¹H NMR (400 M*Hz*, CDCl₃) δ7.04 - 7.02 (d, *J* = 8.59 *Hz*, 1H), 6.17 - 6.15 (d, *J* = 8.34 *Hz*, 1 H), 4.47 - 4.39 (m, 1 H), 3.34 (s, 3H), 3.24 - 3.20 (br d, *J* = 12.38 *Hz*, 2H), 3.05 (s, 6H), 2.78 - 2.72 (m, 2H), 2.65 - 2.55 (m, 2H), 1.78 - 1.75 (br s, 2H).
HPLC retention time: 5.63 min

### Example 18: Preparation of 5-Dimethylamino-3-(1-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one HCl salt

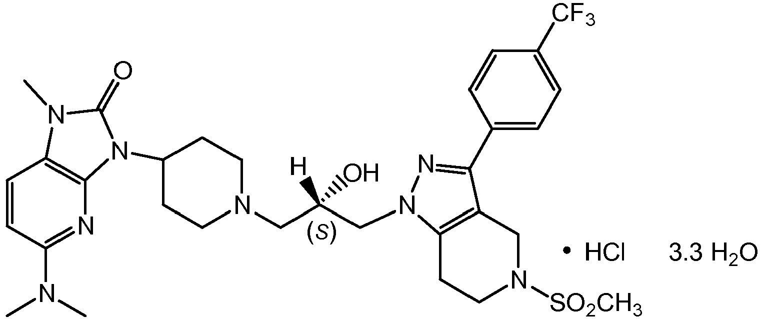

In a dried, nitrogen-flushed, round-bottom flask with a magnetic stir bar, 5-dimethylamino-1-methyl-3-piperidin-4-yl-1,3-dihydro-imidazo[4,5-b]pyridin-2-one (12.35 g, 0.0448 mol), 5-methanesulfonyl-1 (R)-oxiranylmethyl-3-(4-trifluoromethylphenyl)-4,5,6,7-tetrahydro-1H-pyrazolo[4,3-c]pyridine (12 g, 0.0299 mol) and Ti(O-*l*-Pr)₄ (13.4 mL, 0.0450 mol) in CH₂Cl₂ (200 mL) were combined. The reaction mixture was heated to 60°C for 65 h, then cooled to room temperature for 1 h. The reaction mixture was then treated with saturated NaHCO₃ (150 mL), 1 N NaOH (150 mL) and CH₂Cl₂ (50 mL), stirring overnight. The precipitated salts were filtered and washed with CH₂Cl₂ (150 mL). The organic layer in the filtrate was extracted, washed with saturated NaCl solution (150 mL), dried with Na₂SO₄, then filtered and concentrated to yield an oil. The oil was purified via flash chromatography (column: 10 cm {OD}, 15 cm of silica gel in height and eluting with 95/5 CH₂Cl₂/MeOH). The desired fractions were combined to yield an oily foam. The oily foam was treated with MeOH/Et₂O (4/1) to form a white slurry after stirring for 2 h. The white solids were filtered and washed with Et₂O to yield the title compound as its corresponding freebase. The freebase was diluted in CH₂Cl₂/CHCl₃ (125 mL/125 mL), then treated with 1.1 equiv of 2M ethereal HCl. The resulting mixture was stirred for 4 h, whereupon precipitation developed in 1.5 h. The resulting mixture was concentrated via rotovap to yield a white solid, then stirred in Et₂O (450 mL) overnight. The solids were collected by filtration and dried under house vacuum to yield the title compound as its corresponding HCl salt, as a solid.
[α]²⁰₅₈₉ - 6.9 ° (c 0.01, CH₂Cl₂)
MS (electrospray): Calculated for C₃₁H₃₉N₈F₃O₄S, 676.70; *m*/*z* Measured: 677.5 [M+1]⁺.

Title Compound Freebase: ¹H NMR (400 M*Hz*, CDCl₃): δ 7.73 7.65 (dd, *J* = 16.9, 8.1 *Hz*, 4H), 7.03-7.04 (d, *J* = 8.6Hz, 1H), 6.15 - 6.17 (d, *J* = 8.6Hz, 1H), 4.52 - 4.62 (dd, *J* = 14.7, 9.3 *Hz*, 2H), 4.32 - 4.40 (m, 1H), 4.20 - 4.24 (dd, *J* = 11.1, 2.5 *Hz*, 1 H), 4.13 (br s, 1 H), 3.99 - 4.07 (m, 2H), 3.62 - 3.77 (m, 2H), 3.34 (s, 3H), 2.68 - 3.15 (m, 6H), 3.03 (s, 6H), 2.88 (s, 3H), 2.42 - 2.53 (m, 3H), 2.17 - 2.23 (t, *J* = 10.1 *Hz*, 1 H), 1.73 - 1.76 (d, *J* = 11.4 *Hz*, 2H).

Title Compound HCl Salt: ¹H NMR (400 M*Hz*, MeOD) δ 7.66 - 7.87 (m, 4H), 7.35 (br s, 1H), 6.46 (br s, 1H), 4.65 - 4.71 (br t, *J* = 12.2 *Hz*, 1H), 4.57 (br s, 3H), 4.19 - 4.30 (m, 2H), 3.76 - 3.83 (br t, *J* = 14.4 *Hz*, 2H), 3.62 - 3.71 (m, 2H), 2.99 - 3.40 (m, 20H), 1.97 - 2.11 (m, 2H).

### Example 19: Preparation of a Mixture of 4-(6-Chloro-3-nitro-pyridin-2-ylamino)-piperidine-1-carboxylic acid ethyl ester (Reg-A1: desired compound) and 4-(6-Chloro-5-nitro-pyridin-2-ylamino)-piperidine-1-carboxylic acid ethyl ester (Reg-A2: undesired compound)

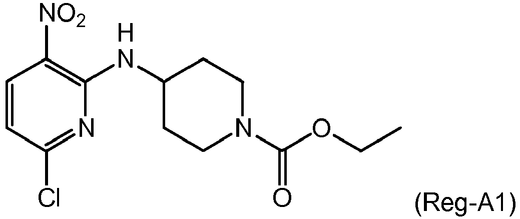

and

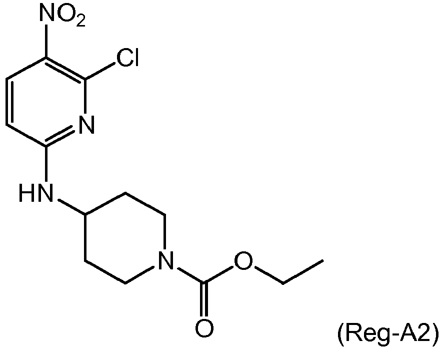

In a dried, 1-neck, 500 ml round-bottom flask with a magnetic stir bar, 2,6-dichloro-3-nitropyridine (42.0 g, 0.2187 mol) and K₂CO₃ (30.2 g, 0.2185 mol) were mixed in DMF (75 ml) then chilled to 0°C. Using an addition funnel, ethyl-4-amino-1-piperidine carboxylate (25.0 ml, 0.1457 mol) in DMF (25 ml) was slowly added to the reaction mixture (to minimize the amount of exotherm in the reaction). Additional DMF (25 ml) was added to the reaction to improve stirring. The reaction mixture was allowed to stir up to room temperature for 18 h then treated with H₂O (100 ml). The aqueous layer was washed with CH₂Cl₂ (3 x 150 ml) and extracted. The combined organic layer was dried with MgSO₄, filtered and concentrated to yield a yellow oil. The oil was purified by filtration chromatography (silica gel column: 14 cm OD, 10 cm in height and eluting with CH₂Cl₂ increasing to 95/5 CH₂Cl₂/MeOH). The desired fractions were combined and concentrated to yield an oil. The oil was stirred in Et₂O (150 ml) and placed on an ice bath for 30 min whereupon precipitation developed. The resulting yellow solids were filtered and washed with 4/1 Et₂O/hexane to yield a mixture of about 85:15 of the compounds (Reg-1) : (Reg-2) as a yellow solid. The mixture of prepared compounds was used in the next step without further purification.
MS (electrospray), *m*/*z* for C₁₃H₁₇ClN₄O₄ (M⁺ + K) 363.0
¹H NMR of (Reg-1): (400 M*Hz*, CDCl₃) δ8.37 - 8.35 (d, *J* = 8.59 *Hz*, 1H), 8.28 - 8.26 (br d, *J* = 7.58 *Hz,* 1 H), 6.64 - 6.62(d, *J* = 8.59 *Hz*, 1H), 4.44 - 4.31 (m, 1 H), 4.18 - 4.13 (br q, *J* = 7.07, 14.15 *Hz*, 4H), 3.09 - 3.03 (t, *J* = 12.13 *Hz*, 2H), 2.1 - 2.06 (br d, *J* = 13.14 *Hz*, 2H), 1.58 - 1.48 (m, 2H), 1.29 - 1.26 (t, *J* = 7.07 *Hz,* 3H).
HPLC retention time (Reg-A1): 9.81 min
HPLC retention time (Reg-A2): 9.05 min

### Example 20: Preparation of Mixture of 4-(6-Dimethylamino-3-nitro-pyridin-2-ylamino)-piperidine-1-carboxylic acid ethyl ester (Reg-B1: desired compound) and 4-(6-Dimethylamino-5-nitro-pyridin-2-ylamino)-piperidine-1-carboxylic acid ethyl ester (Reg-B2: undesired compound)

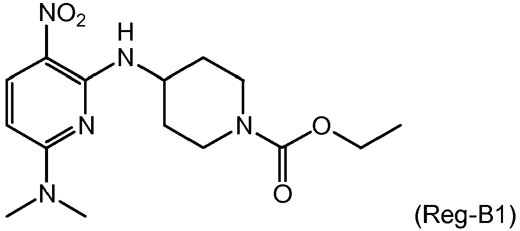

and

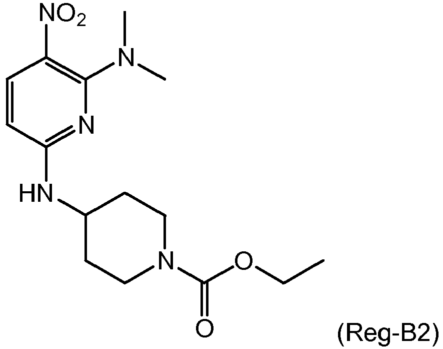

In a dried, 1-neck 1 L round-bottom flask with a magnetic stir bar, the mixture prepared as in Example 19 above (38.15 g, 0.1160 mol) was slurried in MeOH/CH₂Cl₂ (150 ml/20 ml). Using an addition funnel, 2 M (CH₃)₂NH in THF (180 ml, 0.3600 mol) was slowly added to the slurry mixture. The slurry reaction mixture was mildly exothermic and developed into a clear solution. After stirring overnight at room temperature for 18 h, the resulting mixture was concentrated to yield a yellow solid. The yellow solid was diluted in CH₂Cl₂ (250 ml) and washed with saturated NaHCO₃ solution (2 x 180 ml). The extracted organic layer was dried with Na₂SO₄, filtered and concentrated to yield a mixture of about 85: 15 of the compounds (Reg-B1): (Reg-B2). The mixture of prepared compounds was used in the next step without further purification.
MS (electrospray), *m*/*z* for C₁₅H₂₃N₅O₄ (M⁺ + Na) 360.1
¹H NMR of (Reg-B1): (400 M*Hz*, CDCl₃) δ8.75 - 8.74 (d, *J* = 7.07 *Hz*, 1 H), 8.17 - 8.19 (d, *J* = 9.35 *Hz,* 1 H), 5.96 - 5.98(d, *J* = 9.60 *Hz,* 1 H), 4.30 - 4.21 (m, 1 H), 4.17 - 4.12 (q, *J* = 7.07, 14.15 *Hz*, 2H), 4.05 - 4.07 (br d, *J* = 9.85 *Hz*, 2H), 3.18 (s, 6H), 3.10 - 3.04 (br t, *J* = 11.12 *Hz*, 2H), 2.09 - 2.06 (br d, *J* = 11.12 *Hz,* 2H), 1.61 - 1.52 (m, 2H), 1.29 - 1.25 (t, *J* = 7.33 *Hz,* 3H).
HPLC retention time of (Reg-B1): 9.17 min
HPLC retention time of (Reg-B2): 8.70 min

### Example 21: Preparation of Mixture of 4-(3-Amino-6-dimethylamino-pyridin-2-ylamino)-piperidine-1-carboxylic acid ethyl ester (Reg-C1: desired compound) and 4-(5-Amino-6-dimethylamino-pyridin-2-ylamino)-piperidine-1-carboxylic acid ethyl ester (Reg-C2): undesired compound)

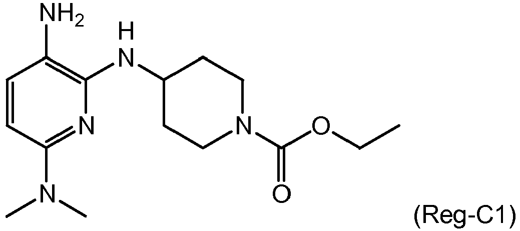

and

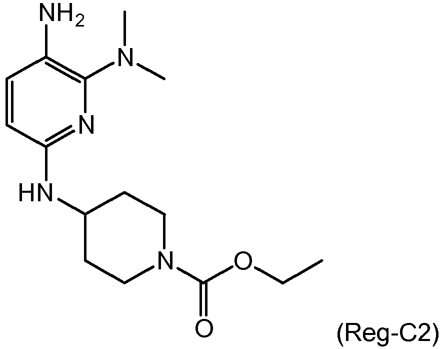

In a 2 L hydrogenation flask, the mixture prepared as in Example 20 above (39.14 g, 0.1160 mol) in EtOH (250 ml) and EtOAc (250 ml) was carefully treated with 10% Pd/C catalyst (4.0 g) under N₂ (g) then hydrogenated overnight for 18 h maintaining pressure at 30-35 psi. The catalyst was filtered through a pad of CELITE^{®} and washed with EtOAc (300 ml). The resulting dark filtrate was concentrated via rotovap to yield a mixture of about 85:15 of the compounds (Reg-C1): (Reg-C2) as a dark solid. ). The mixture of prepared compounds was used in the next step without further purification.
MS (electrospray), *m*/*z* for C₁₅H₂₅N₅O₂ (M⁺ + H) 308.1
HPLC retention time (Reg-C1): 6.68 min
HPLC retention time (Reg-C2) 6.34 min

### Example 22: Preparation of 4-(5-Dimethylamino-2-oxo-1,2-dihydro-imidazo[4,5-b]pyridin-3-yl)-piperidine-1-carboxylic acid ethyl ester

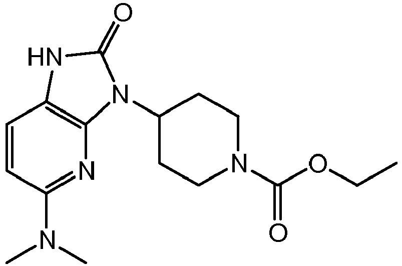

In a dried, 1-neck, 500 ml round bottom flask with a magnetic stir bar, the mixture prepared as in Example 21 above (35.62 g, 0.1159 mol) in THF (250 ml) was treated with CDI (20.0 g, 0.1233 mol) in 2 portions. A mild exotherm was observed after addition was complete. The reaction mixture was refluxed at 98°C for 24 h, then cooled to room temperature. The resulting dark reaction mixture was concentrated via rotovap to yield a dark solid, which was then slurried in EtOAc (300 ml) for 3 h. The solids were filtered to yield the title compound as a solid. The filtrate was washed with saturated NaHCO₃ (250 ml) and NaCl (250 ml) solution. The extracted organic layer was dried with Na₂SO₄, filtered and concentrated to yield additional title compound as a solid. The product materials were combined and purified by filtration chromatography (silica gel column: 14 cm OD, 10 cm in height and eluting with 9/1 EtOAc/hexane). The desired fractions containing the title compound were combined and concentrated to yield a solid. The solid was stirred in hot EtOAc (200 ml) and allowed to cool to room temperature overnight. The solids were filtered to yield the title compound as a solid.
MS (electrospray), *m*/*z* for C₁₆H₂₃N₅O₃ (M⁺ - H) 332.1
¹H NMR (400 M*Hz*, CDCl₃) δ8.85 (s, 1 H), 7.16 - 7.13 (d, *J* = 8.59 *Hz*, 1 H), 6.16 - 6.14 (d, *J* = 8.34 *Hz*, 1 H), 4.51 - 4.43 (m, 1 H), 4.36 - 4.29 (br d, *J* = 27.8 *Hz*, 2H), 4.19 - 4.13 (q, *J* = 7.07, 14.15 *Hz*, 2H), 3.02 (s, 6H), 2.89 (br s, 2H), 2.76 - 2.66 (m, 2H), 1.80 - 1.77 (d, *J* = 11.84 *Hz*, 2H), 1.30 - 1.26 (t, *J* = 7.07 *Hz,* 3H).
HPLC retention time: 6.88 min

### Example 23: Preparation of (S)-glycidol methylbenzesulfonate

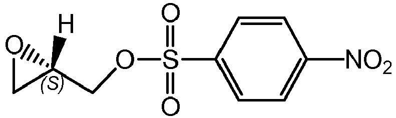

The title compound was prepared as described below, with reference to the procedure as disclosed in *J. Org. Chem.* **1989**, *54*, 1295-1304

A 1-L 3-necked round-bottomed flask was charged with (R)-glycidol (15.0g, 202.7 mmol) and anhydrous methylene chloride (225 mL). The resulting mixture was cooled to about-20°C, then triethylamine (22.5g, 223.0 mmol) was added. 4-Nitrobenzenesulfonyl chloride (47.2 g, 212.8 mmol) was added in about 5g portions over 1 h. The resulting suspension was stirred at about -5°C for 6h. The reaction mixture was then filtered through a medium porosity glass frit. The filtrate was diluted with anhydrous methylene chloride (175 mL), washed with 2% aqueous H₂SO₄ (150 mL), followed by saturated aqueous NaHCO₃ (150 mL) and then brine (150 mL). The organic layer was dried over anhydrous MgSO₄, filtered and concentrated to yield a pale yellow-brown solid. The solid was dissolved in anhydrous toluene (250 mL) and the resulting suspension warmed to about 55°C. To the resulting clear solution, hexane was added drop wise till the solution turned cloudy. The resulting solution was cooled to ambient temperature over 16h, with stirring. The resulting slurry was cooled to 0°C and maintained at this temperature with stirring, for 2h. The resulting precipitate was collected by suction filtration through a medium porosity glass frit. The filter cake was broken and the off white powder was thoroughly dried in vacuo to yield the title compound as a solid.
TLC (R_{f} = 0.68, SiO₂, ethyl acetate)
¹H NMR (400 M*Hz*, CDCl₃) δ: 2.63 (dd, 1 H), 2.85 (t, 1 H), 3.21-3.23 (m, 1 H), 4.03 (dd, 1 H), 4.48 (dd, 1 H), 8.13-8.15 (m, 2 H), 8.41-8.43 (m, 2 H).
HPLC Retention time: 8.21 min

### Example 24: Preparation of 5-Methanesulfonyl-1(R)-oxiranylmethyl-3-(4-trifluoromethyl-phenyl)-4,5,6,7-tetrahydro-1H-pyrazolo[4,3-c]pyridine

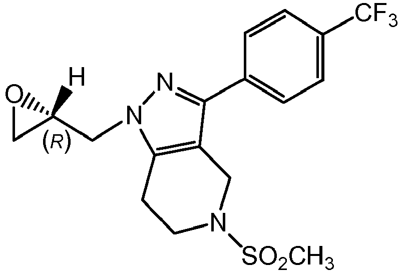

A 1-L 3-necked round-bottomed flask was charged with 5-methanesulfonyl-3-(4-trifluoromethyl-phenyl)-4,5,6,7-tetrahydro-1H-pyrazolo[4,3-c]pyridine (25.4 g, 73.5 mmol). To the resulting mixture was added anhydrous dimethoxyethane (250 mL). The reaction mixture was cooled to about -5°C in a salt / ice water bath with magnetic stirring. A pre-cooled solution (0°C) of KOt-Bu (9.0 g, 80.8 mmol) in anhydrous dimethoxyethane (50 mL) was added drop-wise over 0.5h via an addition funnel. The resulting pale yellow colored solution was maintained at about -5°C for 15 minutes. A pre-cooled solution (0°C) of the (S)-glycidol methylbenzesulfonate (20.0 g, 77.2 mmol) in anhydrous dimethoxyethane (60 mL) was added drop wise via an addition funnel over 30 minutes. The reaction mixture was then stirred at 0-5°C for about 4h. The reaction mixture was then filtered through a glass frit and concentrated on a rotary evaporator to yield a tan colored solid. The solid was dissolved in EtOAc (250 mL) and the organic layer was washed with saturated aqueous NaHCO₃ (2 X 100 mL), followed by H₂O (1 X 100 mL). The resulting mixture was filtered and concentrated to yield a tan colored solid. The solid was suspended in EtOAc (ca. 75 mL) and warmed to 50°C in a water bath. Hexane (50 mL) was added at 50°C and the resulting mixture allowed to cool to ambient temperature over 16h. Hexane (50 mL) was added drop wise and the resulting suspension was stirred at ambient temperature for about 1 h. Hexane (25 mL) was added drop wise, the suspension cooled to 0°C and maintained at this temperature for 2h with stirring. The precipitate was collected by suction filtration through a glass frit and dried in vacuo to yield the title compound as an off-white powder. Following precipitation, the filtrate was concentrated to yield an oily solid. The oily solid was subjected to silica gel column chromatography (EtOAc/Hexanes) to yield additional title compound as a solid.
MP 150-151 °C
TLC (R_{f} = 0.52, SiO₂, EtOAc)
MS (electro spray, positive mode), M⁺ 402
¹H NMR (400 M*Hz*, CDCl₃) δ: 2.49 (dd, 1 H), 2.85 (dd, 1 H), 2.92 (m, 2H), 3.36 (m, 1 H), 3.67 (m, 2H), 4.12 (dd, 1 H), 4.52 (dd, 1 H), 4.53-4.57 (2H), 7.67-7.72 (4H).
HPLC Retention time: 8.78 min

### Example 25: Preparation of 5-Dimethylamino-3-(1-{2(S)-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one

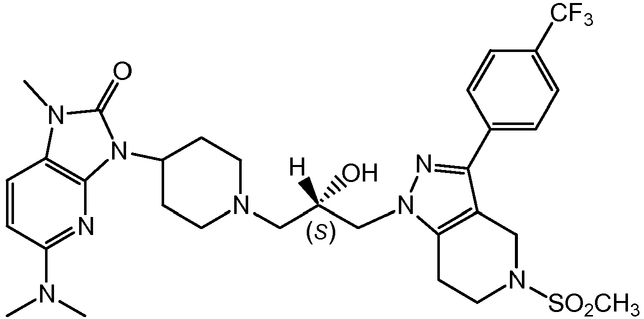

In a dried, nitrogen-flushed, round-bottom flask with a magnetic stir bar, 5-dimethylamino-1-methyl-3-piperidin-4-yl-1,3-dihydro-imidazo[4,5-b]pyridin-2-one (12.35 g, 0.0448 mol), 5-Methanesulfonyl-1-oxiranylmethyl-3-(4-trifluoromethyl-phenyl)-4,5,6,7-tetrahydro-1H-pyrazolo[4,3-c]pyridine (12 g, 0.0299 mol) and Ti(O-/-Pr)₄ (13.4 mL, 0.0450 mol) in dichloroethane (200 mL) were combined. The reaction mixture was heated to 60°C for 65 h, then cooled to room temperature for 1 h. The reaction mixture was then treated with saturated NaHCO₃ (150 mL), 1 N NaOH (150 mL) and CH₂Cl₂ (50 mL), stirring overnight. The precipitated salts were filtered and washed with CH₂Cl₂ (150 mL). The organic layer in the filtrate was extracted, washed with saturated NaCl solution (150 mL), dried with Na₂SO₄, then filtered and concentrated to yield an oil. The oil was purified via flash chromatography (column: 10 cm {OD}, 15 cm of silica gel in height and eluting with 95/5 CH₂Cl₂/MeOH). The desired fractions were combined to yield the title compound as an oily foam. The oily foam was treated with CH₃OH/Et₂O (4/1) to yield a white slurry, after stirring for 2 h. The white solids were filtered and washed with Et₂O to yield the title compound as a freebase as a solid.
Freebase mp 145°-150°C.
¹H NMR (400 M*Hz*, CDCl₃): δ 7.73 - 7.65 (dd, *J* = 16.9, 8.1 *Hz*, 4H), 7.03 - 7.04 (d, *J* = 8.6 *Hz*, 1 H), 6.15 - 6.17 (d, *J* = 8.6 *Hz*, 1 H), 4.52 - 4.62 (dd, *J* = 14.7, 9.3 *Hz,* 2H), 4.32 - 4.40 (m, 1 H), 4.20 - 4.24 (dd, *J* = 11.1, 2.5 *Hz,* 1 H), 4.13 (br s, 1 H), 3.99 - 4.07 (m, 2H), 3.62 - 3.77 (m, 2H), 3.34 (s, 3H), 2.68 - 3.15 (m, 6H), 3.03 (s, 6H), 2.88 (s, 3H), 2.42 - 2.53 (m, 3H), 2.17 - 2.23 (t, *J* = 10.1 *Hz*, 1 H), 1.73 - 1.76 (d, *J* = 11.4 *Hz*, 2H).
[α]²⁰₅₈₉ - 6.9 ° (c 0.01, CH₂Cl₂)
Hewlett Packard MS (electrospray): Calculated for C₃₁H₃₈N₈F₃O₄S, 676.70; *m*/*z* Measured: 677.5 [M+1]⁺.

The title compound as a freebase was diluted in CH₂Cl₂/CHCl₃ (125 mL/125 mL), then treated with 1.1 equiv of 2M ethereal HCl. The resulting mixture was stirred for 4 h, whereupon precipitation developed in 1.5 h. The resulting mixture was concentrated via rotovap to yield a white solid, which was then stirred in Et₂O (450 mL) overnight. The solids were collected by filtration and dried under house vacuum to yield the title compound, as its corresponding HCl salt, as a solid.
HCl salt mp 225°-230°C
¹H NMR (400 M*Hz*, MeOD) δ 7.66 - 7.87 (m, 4H), 7.35 (br s, 1 H), 6.46 (br s, 1 H), 4.65 - 4.71 (br t, *J* = 12.2 *Hz*, 1 H), 4.57 (br s, 3H), 4.19 - 4.30 (m, 2H), 3.76 - 3.83 (br t, *J* = 14.4 *Hz*, 2H), 3.62 - 3.71 (m, 2H), 2.99 - 3.40 (m, 20H), 1.97 - 2.11 (m, 2H).

Examples 26 through 30 describe procedures for the synthesis of the titled compounds. Unless otherwise noted, the identity and purity of the isolated product prepared in these examples was measured by HPLC. Unless otherwise noted, the physical properties (e.g. ¹H NMR, MS, etc.) listed at the end of Examples 26 through 30 are a listing of the physical properties measured for a representative sample of the title compound.

### Example 26: 5-Methanesulfonyl-3-(4-trifluoromethyl-phenyl)-4,5,6,7-tetrahydro-1H-pyrazolo[4,3-c]pyridine

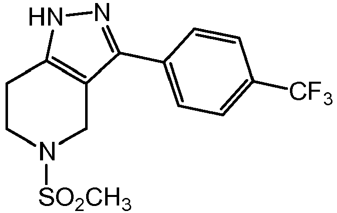

A 500 mL, 4-neck round bottom flask was charged with dichloromethane (20 g), N-methanesulfonyl-4-piperidine (15 g), 4-(trifluoromethyl)-benzoyl chloride (21.15 g) and magnesium iodide (28.26 g, 1 % H₂O), under a nitrogen atmosphere, with stirring. To the resulting mixture was then added triethylamine (25.6 g), dropwise over about 25 minutes, while maintaining the temperature of the mixture in the range of 25-35°C. The resulting mixture was stirred at 27-30°C for 50 minutes. Deionized water (60 mL) was then added and the resulting mixture stirred at 27-30°c for an additional 15 minutes. Concentrated HCl (35-37%, 17 gm) was added dropwise at 25-30°C, to a mixture pH of about 1. The resulting biphasic mixture was separated, the aqueous layer extracted with dichloromethane (3 X 135 g). The combined organic layers were washed with dichloromethane (3 X 40 mL) and dried on sodium sulfate (50 gm). The resulting mixture was distilled to remove the dichloromethane solvent, to yield a residue. To the residue was added deionized water (100 mL) and the resulting mixture was again distilled under vacuum to remove water (25 mL). Ethanol (60 g) and hydrazine monohydrochloride (8.43 g) in deionized water (10 ml) were added and the resulting mixture heated to 55°C. To the resulting mixture was added sodium hydroxide ((5.37 g) in water (40 mL) at 55-60°C. The resulting mixture was then heated to reflux (80-85°C) for 1 hour, then cooled to below 5°C, and stirred at this temperature for an additional 30 minutes. The resulting mixture was then filtered, the filtercake washed with water (3 X 10 mL) and then dried at 50-55°C for 2 hours at 700 mm to yield the title compound as a solid.

### Recrystallization

A 100 mL 4-neck round bottom flask was charged with the solid prepared as described above (18.5g) and acetonitrile (26 g) with stirring and the resulting mixture heated to reflux (80°C) for 30 minutes. The resulting mixture was filtered hot, the filtrate cooled to 0°C and held at 1-5°C, with stirring for 1 hour. The resulting mixture was filtered, the filtercake washed with cold acetonitrile (3 X 4g) and then dried at 50-55°C, 760 mm for 2 hours, to yield the title compound as a solid.
¹H NMR (300MHz, CD₃CN): δ 1.94 (t, 2H), 2.88 (s, 3H), 3.58 (t, 2H), 4.52 (s, 2H), 7.73 (d, 2H), 7.81 (d, 2H), 11.13 (br s, 1 H).
MS (electrospray), Calculated for C₁₄H₁₄ F₃N₃O₂S:, 345.3, *m*/*z* Measured: [M+1]⁺ 346.2

### Example 27: (R)-2-glycidyl-5-methanesulfonyl-3-(4-trifluoromethyl-phenyl)-4,5,6,7-tetrahydro-1H-pyrazolo[4,3-c]pyridine

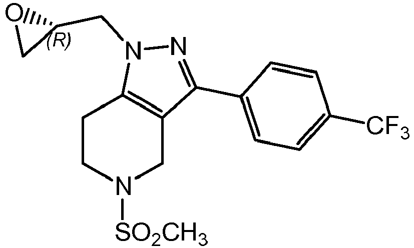

### STEP 1: 1-Chloro-3-[5-methanesulfonyl-3-(4-trifluoromethyl-phenyl)-4,5,6,7-tetrahydro-pyrazolo[4,3-c]pyridin-1-yl]-propan-2(R)-ol

A 250 mL 4-necked double-jacket glass vessel equipped with overhead mechanical stirrer, funnel, syringe pump and thermometer was charged with 5-methanesulfonyl-3-(4-trifluoromethyl-phenyl)-4,5,6,7-tetrahydro-1H-pyrazolo[4,3-c]pyridine (20 g, 58 mmol), ytterbium triflate hydrate (1.4 g, 2.32 mmol) and toluene (38.5 g) at 25°C. The resulting suspension was warmed to 60-61 °C. Over about 10 minutes, (R)-epichlorohydrin (ee<99%, 6.4 g, 6.96 mmol) was added dropwise to the stirring mixture. The resulting mixture was stirred for 3 hours, then cooled to 30°C and then diluted with 2-methyltetrahydrofuran (14.5g). To the resulting mixture was then added dropwise a mixture of 37% hydrochloric acid (2.4 g) in water (14 g). The resulting suspension was warmed to 65-67°C. The resulting bi-phasic mixture was separated, and the residual organic phase washed with water (2 X 10 mL), to yield the organic phase containing 1-chloro-3-[5-methanesulfonyl-3-(4-trifluoromethyl-phenyl)-4,5,6,7-tetrahydro-pyrazolo[4,3-c]pyridin-1-yl]-propan-2(R)-ol, which was used in the next step without further purification or isolation.

### STEP 2: (R)-2-glycidyl-5-methanesulfonyl-3-(4-trifluoromethyl-phenyl)-4,5,6,7-tetrahydro-1H-pyrazolo[4,3-c]pyridine

The organic phase isolated in STEP A above was cooled to 30°C, 30% NaOH (11.5 g, 295 mmol) was added dropwise, with stirring (which resulted in an increase in temperature to about 40°C), and the resulting mixture stirred for 12 hours at 40°C. The resulting mixture was cooled to 25°C, and then neutralized to pH=7.0 with addition of sulfuric acid (1.2) in water (14.4g). The solvent from the resulting mixture was distilled off, to yield a residue. The residue was added to water (57 g) and the resulting suspension stirred for 12 hours at 25°C, then filtered. The filtercake was pressed, washed with water (10 g) and dried for 5 hrs at 60°C, 3 mbar to yield the title compound as a beige solid.

### STEP 3: Recrystallization

A 350ml 3-neck round bottomed flask was charged with (R)-2-glycidyl-5-methanesulfonyl-3-(4-trifluoromethyl-phenyl)-4,5,6,7-tetrahydro-1H-pyrazolo[4,3-c]pyridine (24.32 g, prepared as in STEP B above) and dissolved with ethanol (143 g) at reflux. Deionized water (151 g) was then added dropwise and the resulting solution cooled to 20°C over 3 h, then stirred for 12hrs at 20°C. The resulting suspension was filtered; the precipitate was collected on a glass filter funnel (D3, 90mm), and pressed well. The filtercake was washed with deionized water (10.0 g), then dried at 5-10 mbar and 60°C for 5hrs to yield the title compound as an off-white solid.
HPLC-purity of the isolated material was measured at 96%.
¹H NMR (400 M*Hz*, CDCl₃) δ: 2.49 (dd, 1 H), 2.85 (dd, 1 H), 2.92 (m, 2H), 3.36 (m, 1 H), 3.67 (m, 2H), 4.12 (dd, 1 H), 4.52 (dd, 1 H), 4.53-4.57 (2H), 7.67-7.72 (4H).
MS (electro spray, positive mode), M⁺ 402

### Example 28: 1-Chloro-3-[5-methanesulfonyl-3-(4-trifluoromethyl-phenyl)-4,5,6,7-tetrahydro-pyrazolo[4,3-c]pyridin-1-yl]propan-2-(R)-ol

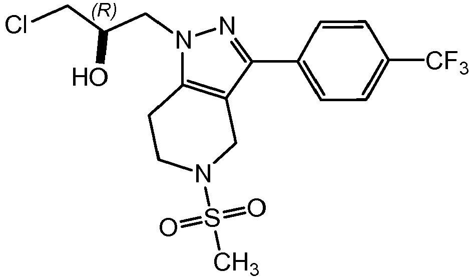

A 1 L double-jacket glass vessel was charged with 5-methanesulfonyl-3-(4-trifluoromethyl-phenyl)-4,5,6,7-tetrahydro-1H-pyrazolo[4,3-c]pyridine (132.55 g, 380 mmol), ytterbium triflate hydrate (9.43 g, 15 mmol) and toluene (347 g) at 25°C. To the resulting mixture was then added, dropwise over about 20 minutes, (R)-epichlorohydrin (ee<99%, 43.05 g, 456 mmol) in toluene (52 g). The resulting mixture heated to 60°C and stirred at this temperature for about 4.5 hours. Approximate 59 g (70 mL) of the mixture solvent was then distilled off at about 75°C to yield a slurry. The slurry was diluted with 2-methyl-THF (125 g). To the resulting mixture was then added, dropwise, a mixture of 37% HCl (37.46 g) in water (150 g). The resulting suspension was heated to about 65-67°C to yield a biphasic mixture. The lower acidic aqueous phase was separated, and the residual organic phase washed three times with water (180 g). Approximately 352 g (410 mL) of solvent was distilled off from the organic phase at about 75°C. To the resulting slurry was then added isopropanol (495 g) to a final volume of about 810 mL and the resulting mixture heated to about 73°C. The resulting mixture was then allowed to cool to room temperature over about 7 hours, then stirred for about 48 hours, over which time a precipitate was observed to form. The precipitate was collected by filtration and washed with isopropanol (118g), dried for 2 hours at 70°C, 50-2 mbar then further dried for 3 hours at 85°C, 2 mbar to yield the title compound as a solid.
¹H NMR (300MHz, DMSO-d₆): δ 2.93 (br t, 2H), 3.00 (s, 3H), 3.58 (t, 2H), 3.72 (dd, 2H), 4.07 - 4.25 (m, 3H), 4.48 (s, 2H), 5.60 (d, 1 H), 7.79 (d, 2H), 7.84 (d, 2H).
MS (electro spray, positive mode), *m*/*z* for C₁₇H₁₉ClF₃N₃O₃S (M⁺): 438

### Example 29: 5-Dimethylamino-3-(1-{2-(S)-hydroxy-3-[5-methanesulfonyl-3-(4-trifluoromethyl-phenyl)-4,5,6,7-tetrahydro-pyrazolo[4,3-c]pyridin-1-yl]-propyl}-piperidin-4-yl)-1-methyl-1,3-dihydro-imidazo[4,5-b]pyridin-2-one

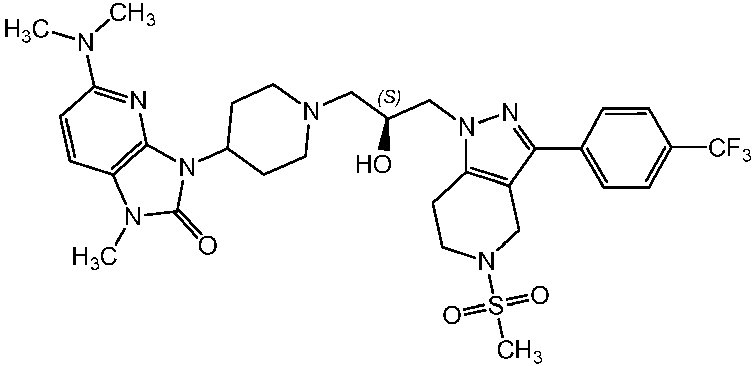

A 1 L double-jacket glass vessel was charged with 1-Chloro-3-[5-methanesulfonyl-3-(4-trifluoromethyl-phenyl)-4,5,6,7-tetrahydro-pyrazolo[4,3-c]pyridin-1-yl]-propan-2-(R)-ol (66.71 g, 150 mol; prepared, for example, as described in Example 29 above), 5-dimethylamino-1-methyl-3-piperidin-4-yl-1,3-dihydro-imidazo[4,5-b]pyridin-2-one (43.75 g, 0.153 mol), potassium carbonate (20.763 g, 0.150 mol) and warm (25°C) isopropanol (786 g); and the resulting mixture heated to about 65°C and stirred for about 5.5 hours, over which time a precipitate was observed to form. The resulting mixture was then allowed to cool to room temperature over about 8 hours, then stirred at 25°C for 2 days. The resulting mixture was then filtered using a glass filter funnel and the solid collected. The filter cake was washed with isopropanol (236 g) and pressed well. The resulting white / off-white wet solid was mixed with water (900 g) and the resulting mixture heated to 65°C for about 1 hour, with stirring. The resulting warm suspension was filtered over a glass filter funnel, the vessel washed with warm (50-60°C) water (1100 g) and the wash water filtered over the collected material. The combined collected solid was dried at 85°C, 2-50 mbar for about 18 hours to yield the title compound as a solid.
¹H NMR (400 M*Hz*, MeOD) δ 7.66 - 7.87 (m, 4H), 7.35 (br s, 1 H), 6.46 (br s, 1 H), 4.65 - 4.71 (br t, *J* = 12.2 *Hz*, 1 H), 4.57 (br s, 3H), 4.19 - 4.30 (m, 2H), 3.76 - 3.83 (br t, *J* = 14.4 *Hz*, 2H), 3.62 - 3.71 (m, 2H), 2.99 - 3.40 (m, 20H), 1.97 - 2.11 (m, 2H).

### Example 30: 5-Methanesulfonyl-3-(4-trifluoromethyl-phenyl)-4,5,6,7-tetrahydro-1H-pyrazolo[4,3-c]pyridine

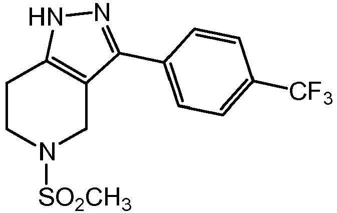

A 1 L double-jacket reaction glass vessel was flushed with argon atmosphere, and then, filled with *N*-Methanesulfonylpiperidin-4-one (106.33 g, 0.6 mol) and hydrazine monohydrochloride (41.11 g, 0.6 mol). The resulting mixture was suspended at ET = 25°C with methanol (240.0 g). During the homogenization of the mixture, while stirring, a light endothermic reaction was observed. After about 20 minutes of stirring at 25°C, the internal temperature of the reaction mixture had increased to about 47-48°C. To the resulting mixture was then added, 4-trifluoromethylbenzaldehyde (103.47 g, 0.6 mol), dropwise by means of syringe pump, while vigorously stirring the reaction mixture at about 50°C. When the addition was complete, the resulting opalescent orange-beige solution was stirred for 1.5h. The resulting mixture was then cooled to about 10°C, over which time, the mixture was observed to become a suspension, which suspension increased substantially in density upon cooling. To the resulting mixture was then added bromine (119.9 g, 0.75 mol), dropwise, while maintaining stirring and an internal temperature of no greater than about 20°C. The resulting mixture was then allowed to warm to about room temperature and stirred for 1 hour. The resulting yellow-orange suspension was neutralized by dropwise addition, at about 20°C, of a 30% methanolic solution of sodium methoxide (377.3 g, 0.21 mol). The resulted beige mixture was stirred for 1 h at about 20°C, then slowly cooled to about - 8°C and stirred at this temperature for about 10 hours, over which time a precipitate was observed to form. The precipitate was collected on a glass filter funnel (D3, 90 mm) and pressed to yield the title compound as a beige solid.

### Recrystallization

The collected wet, beige solid (165.87 g, 0.24 mol) was dissolved in a 0.5 L double jacket reaction glass vessel in acetone (2158.4 g) and deionized water (133.0 g) and the resulting mixture heated with stirring to 70°C. After about 30 min all of the solid material was observed to dissolve and a biphasic mixture was formed. The biphasic mixture was stirred continuously rpm and cooled to 20°C over about 4 hours, then maintained at this temperature, with stirring for 11 hours; over which time a precipitate was observed to form. The precipitate was collected on a glass filter funnel (D3, 90mm) and pressed to yield the title compound. The precipitate was washed with deionized water (120.0 g) and acetone (55.4 g), dried at about 5-10mbar and 50°C for 3.5 h, then at 80°C for 6.5 h and finally at 100°C for 5 h to yield the title compound as a white solid.
¹H NMR (300MHz, CD₃CN): δ 1.94 (t, 2H), 2.88 (s, 3H), 3.58 (t, 2H), 4.52 (s, 2H), 7.73 (d, 2H), 7.81 (d, 2H), 11.13 (br s, 1 H).
MS (electrospray), Calculated for C₁₄H₁₄ F₃N₃O₂S: 345.3, *m*/*z* Measured: [M+1]⁺ 346.2

### Example 31: Oral Formulation - Prophetic Example

As a specific embodiment of an oral composition, 100 mg of the compound prepared as in Example 7 is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A process for the preparation of a compound of formula (I) 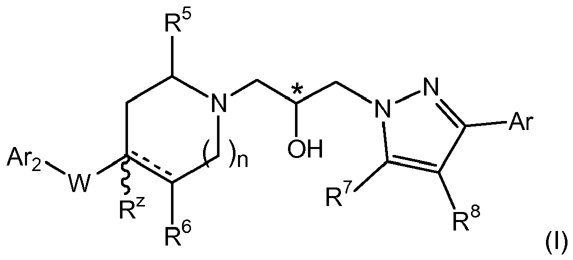 wherein
Ar₂ is a monocyclic or bicyclic ring system, unsaturated, saturated or aromatic, optionally fused, optionally including between 1 and 5 heteroatom ring moieties independently selected from the group consisting of O, S, N, SO₂ and C=O; wherein said Ar₂ ring system is optionally substituted with between 1 and 4 substituents;
W represents O, S, NR²⁷, C=O, (C=O)NH, NH(C=O),CHR²⁸, or a covalent bond;
R²⁷ is hydrogen, C₁₋₅alkyl, C₃₋₅alkenyl, phenyl, naphthyl, benzyl, phenethyl, C₁₋₅heterocyclyl, C₂₋₈acyl, aroyl, R²⁹OC=O, R³⁰R³¹NC=O, R²⁹SO, R²⁹S, R²⁹SO₂ or R³⁰R³¹ NSO₂; or alternatively, R²⁷ and part of Ar₂ can be taken together to form an optionally substituted 5- to 6-membered heterocyclic ring with optionally 1 to 3 additional heteroatom moieties in the ring selected from O, NR⁹, NR¹⁰, N, SO₂, C=O and S; which ring may be saturated, unsaturated or aromatic; wherein R⁹ and R¹⁰ are independently selected from the group consisting of H, C₁₋₃alkyl, and -CH₂CO₂(C₁₋₄alkyl);
R²⁸ is hydrogen, C₁₋₅alkyl, C₃₋₅alkenyl, hydroxy, phenyl, benzyl, C₁₅heterocyclyl, R²⁹O, R³⁰R³¹NC=O, R²⁹S, R²⁹SO, R²⁹SO₂ or R³⁰R³¹NSO₂;
R²⁹ is C₁₋₅alkyl, C₃₋₅akenyl, phenyl, benzyl or C₁₋₅heterocyclyl;
R³⁰ and R³¹ are each independently selected from the group consisting of hydrogen, C₁₋₅alkyl, C₃₋₅alkenyl, phenyl, benzyl, phenethyl, naphthyl, and C₁₅heteroaryl; alternatively R³⁰ and R³¹ can be taken together to form an optionally substituted 4- to 7-membered ring carbocyclic or heterocyclic ring, which ring may be saturated, unsaturated or aromatic;
R^{z} is H or OH and the dashed line is absent; or R^{z} is absent where the dashed line is an sp² bond;
R⁵ and R⁶ are each independently selected from the group consisting of hydrogen and C₁₋₅alkyl;
n is an integer selected from 0, 1 or 2;
R⁷ and R⁸ are each independently hydrogen, C₁₋₅alkyl, C₂₋₅alkenyl, C₁₅alkoxy, C₁₋₅alkylthio, halogen, or a 4-7 membered carbocyclyl or heterocyclyl;
alternatively, R⁷ and R⁸ can be taken together to form an optionally substituted 5- to 7-membered carbocyclyc or heterocyclic ring, which ring may be unsaturated or aromatic, and may be optionally substituted with between one and three substituents independently selected from the group consisting of halo, cyano, amino, hydroxy, nitro, R⁴, R⁴O-, R⁴S-, R⁴O(C₁₋₅alkylene)-, R⁴O(C=O)-, R⁴(C=O)-, R⁴(C=S)-, R⁴(C=O)O-, R⁴O(C=O)(C=O)-, R⁴SO₂, NHR⁴⁴(C=NH)-, NHR⁴⁴SO₂-, an NHR⁴⁴(C=O)-;
R⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
R⁴⁴ is H, C₁₋₅alkyl, C₂₋₅alkenyl, C₁₋₅heterocyclyl, (C₁₋₅heterocyclyl)C₁₆alkylene, phenyl, benzyl, phenethyl, NH₂, mono- or di(C₁₋₆alkyl)N-, (C₁₆alkoxy)carbonyl- or R⁴²OR⁴³-; wherein R⁴² is H, C₁₋₅alkyl, C₂₋₅alkenyl, phenyl, benzyl, phenethyl, C₁₋₅heterocyclyl, or (C₁₋₅heterocyclyl)C₁₋₆alkylene- and R⁴³ is C₁₋₅alkylene, phenylene, or divalent C₁₋₅heterocyclyl;
Ar represents a monocyclic or bicyclic aryl or heteroaryl ring, optionally substituted with between 1 and 3 substituents independently selected from the group consisting of halogen, C₁₋₅alkoxy, C₁₋₅alkyl, C₂₋₅alkenyl, cyano, azido, nitro, R²²R²³N, R²⁴SO₂, R²⁴SO, R²⁴OC=O, R²²R²³NC=O, C₁₋₅haloalkyl, C₁₅haloalkoxy, C₁₋₅haloalkylthio and C₁₋₅alkylthio;
R²² is hydrogen, C₁₋₅alkyl, C₃₋₅alkenyl, phenyl, phenethyl, benzyl, C₁₅heterocyclyl, C₂₋₈acyl, aroyl, R³⁸OC=O, R²⁵R²⁶NC=O, R³⁸SO, R³⁸SO₂, R³⁸S, or R²⁵R²⁶NSO₂;
R²³ is hydrogen, C₁₋₅alkyl, C₃₋₅alkenyl, phenyl, benzyl or C1-5heterocyclyl; alternatively, R²² and R²³ can be taken together to form an optionally substituted 4- to 7-membered heterocyclic ring, which ring may be saturated, unsaturated or aromatic;
R²⁴ is C₁₋₅alkyl, C₃₋₅alkenyl, phenyl, benzyl, or C₁₋₅heterocyclyl;
R²⁵ and R²⁶ independently are hydrogen, C₁₋₅alkyl, C₃₋₅alkenyl, phenyl, benzyl or C₁₋₅heterocyclyl; or alternatively, R²⁵ and R²⁶ can be taken together to form an optionally substituted 4- to 7-membered heterocyclic ring, which ring may be saturated, unsaturated or aromatic;
R³⁸ is H, C₁₋₅alkyl, C₃₋₅alkenyl, phenyl, benzyl, phenethyl or C₁₅heterocyclyl;
wherein each of the above hydrocarbyl or heterocarbyl groups, unless otherwise indicated, and in addition to any specified substituents, is optionally and independently substituted with between 1 and 3 substituents selected from the group consisting of methyl, halomethyl, hydroxymethyl, halo, hydroxy, amino, nitro, cyano, C₁₋₅alkyl, C₁₋₅alkoxy, -COOH, C₂₋₆acyl, [di(C₁₄alkyl)amino]C₂₋₅alkylene, [di(C₁₋₄alkyl)amino]C₂₋₅alkyl-NH-CO-, and C₁₅haloalkoxy;
or a pharmaceutically acceptable salt, amide or ester thereof; or a stereoisomeric form thereof; comprising 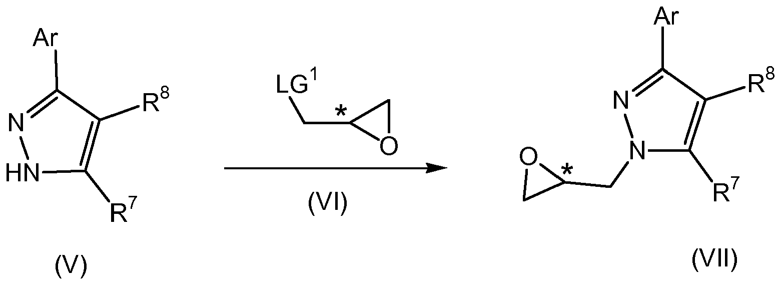 reacting a compound of formula (V) with a compound of formula (VI), wherein LG¹ is a leaving group selected from 4-nitrophenyl-sulfonyl, 3-nitrophenyl-sulfonyl, mesyl, tosyl, trifluoromethane sulfonyl, 3-fluorobenzensulfonyl, 3-(trifluoromethyl)-benzene sulfonyl, 4-chloro-benzenesulfonyl and 3-chloro-benzenesulfonyl; in the presence of an organic or inorganic base; to yield the corresponding compound of formula (VII); 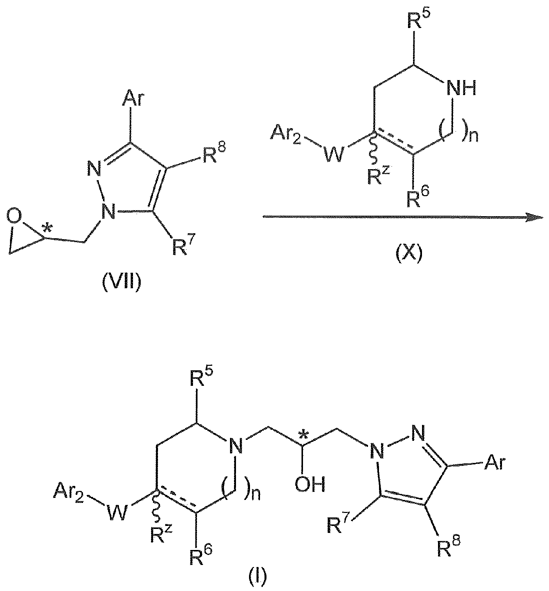 reacting the compound of formula (VII) with a compound of formula (X); in an organic solvent; to yield the corresponding compound of formula (I).

2. A process for the preparation of a compound of formula (I-S) 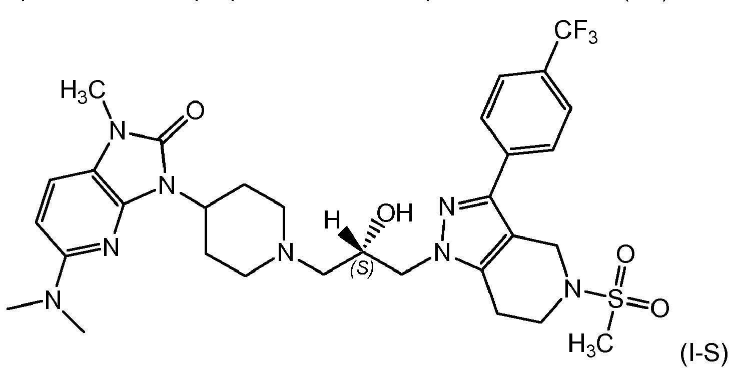 or a pharmaceutically acceptable salt, amide or ester thereof; or a stereoisomeric form thereof; comprising 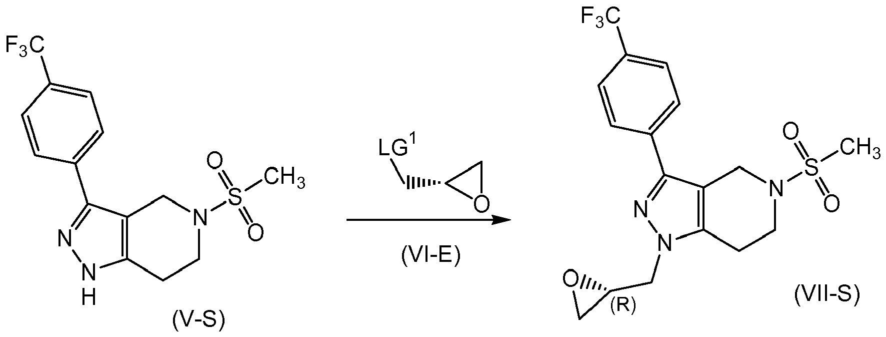 reacting a compound of formula (V-S) with a compound of formula (VIE), wherein LG¹ is a leaving group selected from 4-nitrophenyl-sulfonyl, 3-nitrophenyl-sulfonyl, mesyl, tosyl, trifluoromethane sulfonyl, 3-fluoro-benzensulfonyl, 3-(trifluoromethyl)-benzene sulfonyl, 4-chloro-benzenesulfonyl and 3-chloro-benzenesulfonyl; in the presence of an organic or inorganic base; to yield the corresponding compound of formula (VII-S); 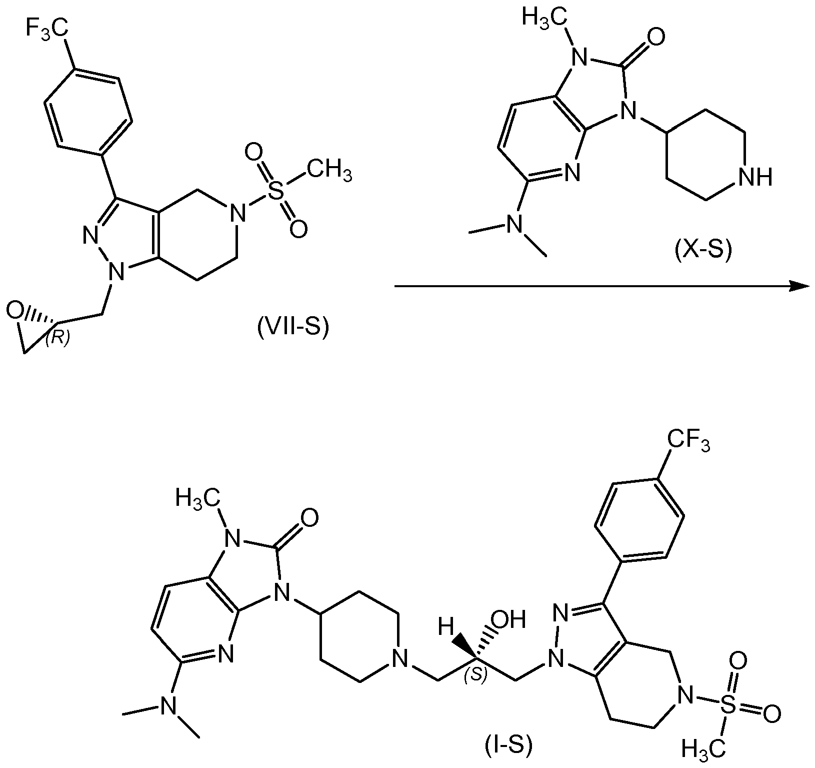 reacting the compound of formula (VII-S) with a compound of formula (X-S); in an organic solvent; to yield the corresponding compound of formula (IS).

3. The process according to claim 2, wherein LG¹ is 3-nitrophenyl-sulfonyl.

4. The process according to claim 2, wherein the compound of formula (VIE) is present in an amount in the range of from about 0.5 to about 3.0 molar equivalents, or wherein the compound of formula (VI-E) is present in about 1.0 to about 1.5 molar equivalents.

5. The process according to claim 2, wherein the organic or inorganic base is selected from the group consisting of TEA, DIPEA, pyridine, NaH, KOt-Bu, NaOt-Bu, Cs₂CO₃, K₂CO₃, Na₂CO₃ and CsF, or wherein the organic or inorganic base is Cs₂CO₃.

6. The process according to claim 2, wherein when the compound of formula (V-S) is reacted with the compound of formula (VI-E) in an organic solvent, the organic solvent is DMF.

7. The process according to claim 2, wherein the compound of formula (V-S) is reacted with the compound of formula (VI-E) at a temperature in the range of from about 0°C to about 5°C, followed by heating to a temperature in the range of from about room temperature to about 60°C.

8. The process according to claim 2, wherein the compound of formula (X-S) is present in an amount in the range of from about 0.5 to about 3.0 molar equivalents, or wherein the compound of formula (X-S) is present in an amount of about 1.1 molar equivalents.

9. The process according to claim 2, wherein when the compound of formula (VII-S) is reacted with the compound of formula (X-S) in an organic solvent, the organic solvent is selected from the group consisting of DCE, ethanol, isopropyl alcohol and DMF, or wherein the organic solvent is ethanol.

10. The process according to claim 2, wherein the compound of formula (VII-S) is reacted with the compound of formula (X-S) at about solvent reflux temperature,

11. The process according to claim 2, wherein the compound of formula (VII-S) is reacted with the compound of formula (X-S) in the presence of a Lewis acid selected from the group consisting of titanium propoxide, Yb(OTf)₃ and Al(OTf)₃.

12. The process according to claim 11, wherein the Lewis acid is present in a catalytic amount.

13. A crystalline, hexahydrate mono-HCl salt of a compound of formula (I-S) 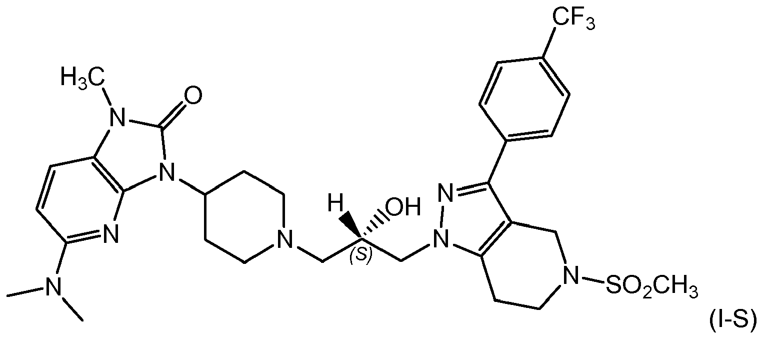 comprising the following X-ray diffraction peaks:
| **Angle (°2θ)** | **d-spacing (Å)** |
|---|---|
| 4.33 | 20.42 |
| 7.58 | 11.67 |
| 8.39 | 10.54 |
| 8.80 | 10.04 |
| 9.16 | 9.66 |
| 12.71 | 6.96 |
| 13.30 | 6.66 |
| 13.69 | 6.47 |
| 14.69 | 6.03 |
| 15.55 | 5.70 |
| 15.96 | 5.55 |
| 18.44 | 4.81 |
| 18.69 | 4.75 |
| 19.07 | 4.65 |

14. A process for the preparation of a crystalline, mono-HCl, hexahydrate salt of claim 13 comprising reacting a compound of formula (I-S) with HCl acid in water.

15. A process for the preparation of a compound of formula (X-S) 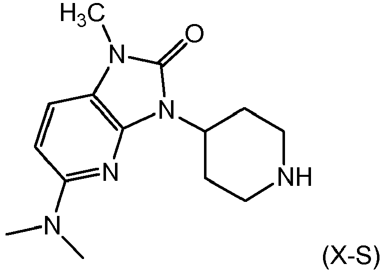 comprising 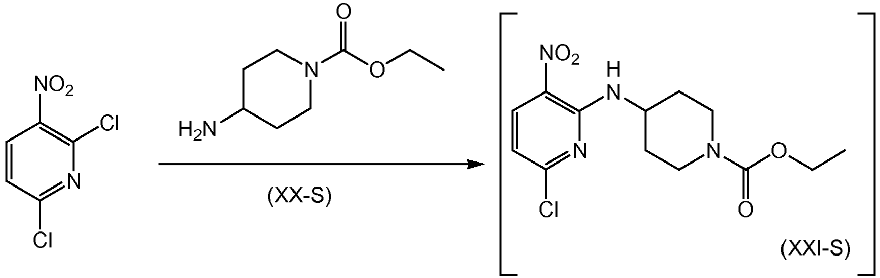 reacting 2,6-dichloro-3-nitro-pyridine with a compound of formula (XX-S), wherein PG⁵ is a nitrogen protecting group; in the presence of an organic or inorganic base; in an organic solvent; to yield the corresponding compound of formula (XXI-S); 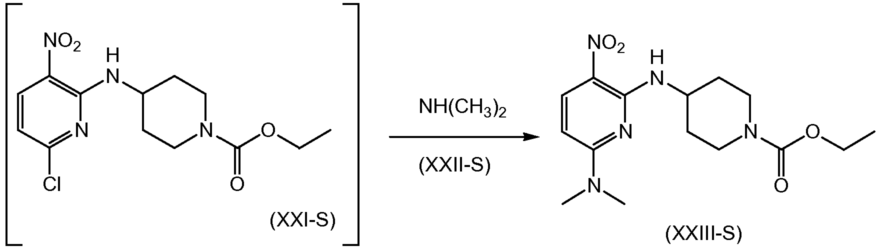 reacting the compound of formula (XXI-S) with a compound of formula (XXII-S); in an organic solvent; to yield the corresponding compound of formula (XXIII-S); 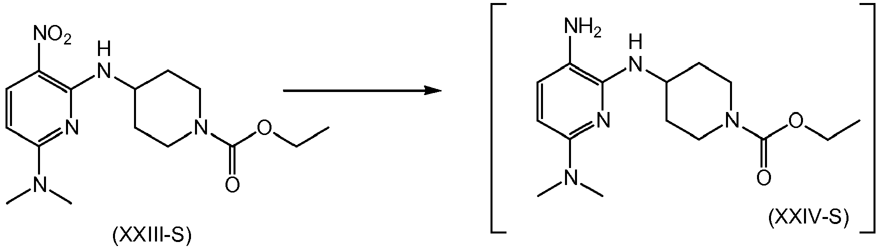 reacting the compound of formula (XXIII-S) with a reducing agent; in the presence of a catalyst; in an organic solvent; to yield the corresponding compound of formula (XXIV-S); 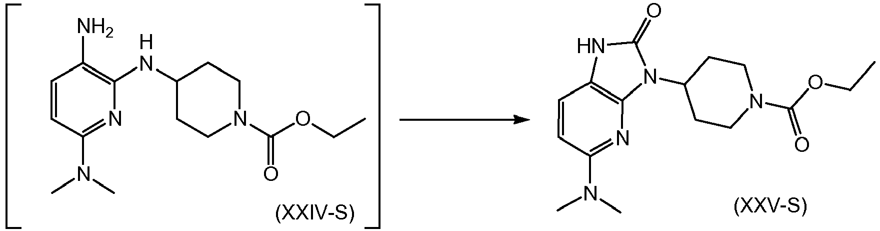 reacting the compound of formula (XXIV-S) with a reagent selected from the group consisting of CDI, N-methylcarbodiimide, phosgene and triphosgene; in an organic solvent; to yield the corresponding compound of formula (XXV-S); 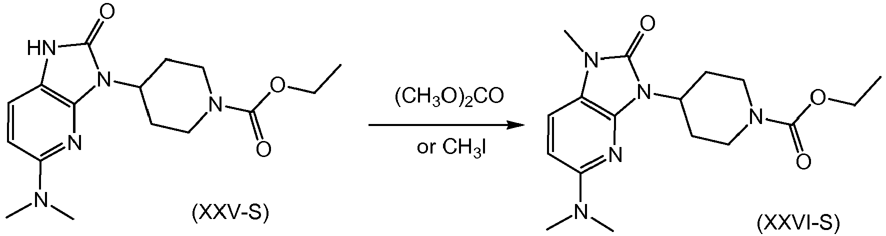 reacting the compound of formula (XXV-S) with carbonic acid dimethyl ester or CH₃I; in the presence of an inorganic base; in an organic solvent; to yield the corresponding compound of formula (XXVI-S); 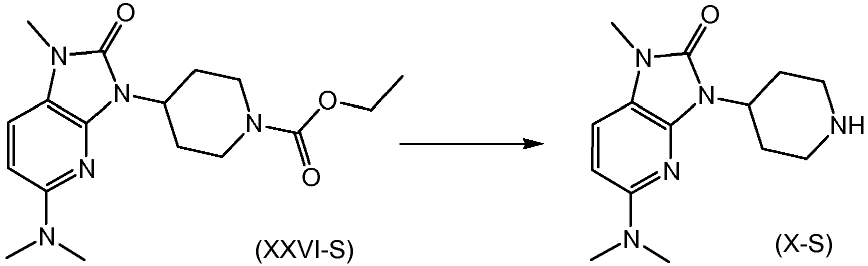 de-protecting the compound of formula (XXVI-S); to yield the corresponding compound of formula (X-S).

## Patentansprüche

1. Verfahren zur Herstellung einer Verbindung der Formel (I) 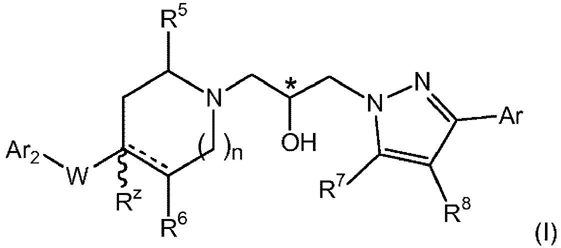 wobei
Ar₂ für ein monocyclisches oder bicyclisches Ringsystem steht, das ungesättigt, gesättigt oder aromatisch und gegebenenfalls anelliert ist und gegebenenfalls zwischen 1 und 5 Heteroatomringgruppierungen, die unabhängig aus der Gruppe bestehend aus 0, S, N, SO₂ und C=O ausgewählt sind, enthält; wobei das Ringsystem Ar₂ gegebenenfalls durch zwischen 1 und 4 Substituenten substituiert ist;
W für 0, S, NR²⁷, C=O, (C=O)NH, NH(C=O), CHR²⁸ oder eine kovalente Bindung steht;
R²⁷ für Wasserstoff, C₁₋₅-Alkyl, C₃₋₅-Alkenyl, Phenyl, Naphthyl, Benzyl, Phenethyl, C₁₋₅-Heterocyclyl, C₂₋₈-Acyl, Aroyl, R²⁹OC=O, R³⁰R³¹NC=O, R²⁹ SO, R²⁹S, R²⁹SO₂ oder R³⁰R³¹NSO₂ steht; oder alternativ dazu R²⁷ und ein Teil von Ar₂ zusammengenommen einen gegebenenfalls substituierten 5-bis 6-gliedrigen heterocyclischen Ring mit gegebenenfalls 1 bis 3 zusätzlichen Heteroatomgruppierungen im Ring, die aus 0, NR⁹, NR¹⁰, N, SO₂, C=O und S ausgewählt sind, bilden können; wobei der Ring gesättigt, ungesättigt oder aromatisch sein kann; wobei R⁹ und R¹⁰ unabhängig aus der Gruppe bestehend aus H, C₁₋₃-Alkyl und -CH₂CO₂ (C₁₋₄-Alkyl) ausgewählt sind;
R²⁸ für Wasserstoff, C₁₋₅-Alkyl, C₃₋₅-Alkenyl, Hydroxy, Phenyl, Benzyl, C₁₋₅-Heterocyclyl, R²⁹O, R³⁰R³¹NC=O, R²⁹S, R²⁹SO, R²⁹SO₂ oder R³⁰R³¹NSO₂ steht; R²⁹ für C₁₋₅-Alkyl, C₃₋₅-Alkenyl, Phenyl, Benzyl oder C₁₋₅-Heterocyclyl steht;
R³⁰ und R³¹ jeweils unabhängig aus der Gruppe bestehend aus Wasserstoff, C₁₋₅-Alkyl, C₃₋₅-Alkenyl, Phenyl, Benzyl, Phenethyl, Naphthyl und C₁₋₅-Heteroaryl ausgewählt sind; alternativ dazu R³⁰ und R³¹ zusammengenommen einen gegebenenfalls substituierten 4- bis 7-gliedrigen carbocyclischen oder heterocyclischen Ring bilden können, wobei der Ring gesättigt, ungesättigt oder aromatisch sein kann;
R^{z} für H oder OH steht und die gestrichelte Linie fehlt oder R^{z} fehlt, wo die gestrichelte Linie für eine sp²-Bindung steht;
R⁵ und R⁶ jeweils unabhängig aus der Gruppe bestehend aus Wasserstoff und C₁₋₅-Alkyl ausgewählt sind;
n für eine ganze Zahl steht, die aus 0, 1 oder 2 ausgewählt ist;
R⁷ und R⁸ jeweils unabhängig für Wasserstoff, C₁₋₅-Alkyl, C₂₋₅-Alkenyl, C₁₋₅-Alkoxy, C₁₋₅-Alkylthio, Halogen oder ein 4- bis 7-gliedriges Carbocyclyl oder Heterocyclyl stehen;
alternativ dazu R⁷ und R⁸ zusammengenommen einen gegebenenfalls substituierten 5- bis 7-gliedrigen carbocyclischen oder heterocyclischen Ring bilden können, wobei der Ring ungesättigt oder aromatisch sein kann und gegebenenfalls durch zwischen einem und drei Substituenten, die unabhängig aus der Gruppe bestehend aus Halogen, Cyano, Amino, Hydroxy, Nitro, R⁴, R⁴O-, R⁴S-, R⁴O(C₁₋₅-Alkylen)-, R⁴O(C=O)-, R⁴(C=O)-, R⁴(C=S) -, R⁴(C=O)O-, R⁴O(C=O) (C=O) -, R⁴SO₂, NHR⁴⁴(C=NH) -, NHR⁴⁴SO₂- und NHR⁴⁴(C=O) - ausgewählt sind, substituiert sein kann;
R⁴ für H, C₁₋₅-Alkyl, C₂₋₅-Alkenyl, C₁₋₅-Heterocyclyl, (C₁₋₅-Heterocyclyl)-C₁₋₆-alkylen, Phenyl, Benzyl, Phenethyl, NH₂, Mono- oder Di(C₁₋₆-alkyl)N-, (C₁₋₆-Alkoxy)carbonyl- oder R⁴²OR⁴³- steht; wobei R⁴² für H, C₁₋₅-Alkyl, C₂₋₅-Alkenyl, Phenyl, Benzyl, Phenethyl, C₁₋₅-Heterocyclyl oder (C₁₋₅-Heterocyclyl)-C₁₋₆-alkylen- steht und R⁴³ für C₁₋₅-Alkylen, Phenylen oder zweiwertiges C₁₋₅-Heterocyclyl steht; R⁴⁴ für H, C₁₋₅-Alkyl, C₂₋₅-Alkenyl, C₁₋₅-Heterocyclyl, (C₁₋₅-Heterocyclyl)-C₁₋₆-alkylen, Phenyl, Benzyl, Phenethyl, NH₂, Mono- oder Di(C₁₋₆-alkyl)N-, (C₁₋₆-Alkoxy)carbonyl- oder R⁴²OR⁴³- steht; wobei R⁴² für H, C₁₋₅-Alkyl, C₂₋₅-Alkenyl, Phenyl, Benzyl, Phenethyl, C₁₋₅-Heterocyclyl oder (C₁₋₅-Heterocyclyl)-C₁₋₆-alkylen- steht und R⁴³ für C₁₋₅-Alkylen, Phenylen oder zweiwertiges C₁₋₅-Heterocyclyl steht; Ar für einen monocyclischen oder bicyclischen Aryl- oder Heteroarylring stehen, der gegebenenfalls durch zwischen 1 und 3 Substituenten, die unabhängig aus der Gruppe bestehend aus Halogen, C₁₋₅-Alkoxy, C₁₋₅-Alkyl, C₂₋₅-Alkenyl, Cyano, Azido, Nitro, R²²R²³N, R²⁴SO₂, R²⁴SO, R²⁴OC=O, R²²R²³NC=O, C₁₋₅-Halogenalkyl, C₁₋₅-Halogenalkoxy, C₁₋₅-Halogenalkylthio und C₁₋₅-Alkylthio ausgewählt sind, substituiert ist;
R²² für Wasserstoff, C₁₋₅-Alkyl, C₃₋₅-Alkenyl, Phenyl, Phenethyl, Benzyl, C₁₋₅-Heterocyclyl, C₂₋₈-Acyl, Aroyl, R³⁸OC=O, R²⁵R²⁶NC=O, R³⁸SO, R³⁸SO₂, R³⁸S oder R²⁵R²⁶NSO₂ steht;
R²³ für Wasserstoff, C₁₋₅-Alkyl, C₃₋₅-Alkenyl, Phenyl, Benzyl oder C₁₋₅-Heterocyclyl steht; alternativ dazu R²² und R²³ zusammengenommen einen gegebenenfalls substituierten 4- bis 7-gliedrigen heterocyclischen Ring bilden können, wobei der Ring gesättigt, ungesättigt oder aromatisch sein kann;
R²⁴ für C₁₋₅-Alkyl, C₃₋₅-Alkenyl, Phenyl, Benzyl oder C₁₋₅-Heterocyclyl steht;
R²⁵ und R²⁶ jeweils unabhängig aus Wasserstoff, C₁₋₅-Alkyl, C₃₋₅-Alkenyl, Phenyl, Benzyl oder C₁₋₅-Heterocyclyl ausgewählt sind; oder alternativ dazu R²⁵ und R²⁶ zusammengenommen einen gegebenenfalls substituierten 4- bis 7-gliedrigen heterocyclischen Ring bilden können, wobei der Ring gesättigt, ungesättigt oder aromatisch sein kann;
R³⁸ für H, C₁₋₅-Alkyl, C₃₋₅-Alkenyl, Phenyl, Benzyl, Phenethyl oder C₁₋₅-Heterocyclyl steht;
wobei jede der obigen Hydrocarbyl- oder Heterocarbylgruppen, sofern nicht anders angegeben und zusätzlich zu angegebenen Substituenten, gegebenenfalls und unabhängig durch zwischen 1 und 3 Substituenten aus der Gruppe bestehend aus Methyl, Halogenmethyl, Hydroxymethyl, Halogen, Hydroxy, Amino, Nitro, Cyano, C₁₋₅-Alkyl, C₁₋₅-Alkoxy, -COOH, C₂₋₆-Acyl, [Di (C₁₋₄-alkyl) amino] C₂₋₅-alkylen, [Di-(C₁₋₄-alkyl)amino]C₂₋₅-alkyl-NH-CO- und C₁₋₅-Halogen-alkoxy substituiert ist;
oder eines pharmazeutisch unbedenklichen Salzes, Amids oder Esters davon oder einer stereoisomeren Form davon; bei dem man 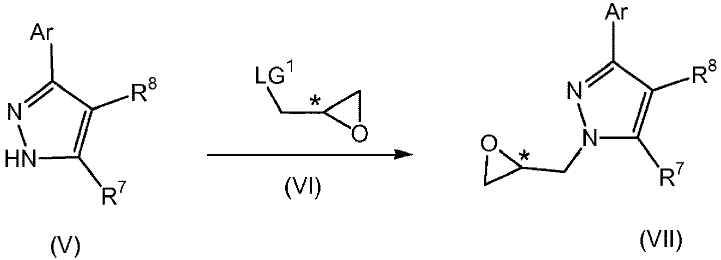 eine Verbindung der Formel (V) in Gegenwart einer organischen oder anorganischen Base mit einer Verbindung der Formel (VI), worin LG¹ für eine Abgangsgruppe, die aus 4-Nitrophenylsulfonyl, 3-Nitrophenylsulfonyl, Mesyl, Tosyl, Trifluormethansulfonyl, 3-Fluorbenzolsulfonyl, 3-(Trifluormethyl)benzolsulfonyl, 4-Chlorbenzolsulfonyl und 3-Chlorbenzolsulfonyl ausgewählt ist, steht, zu der entsprechenden Verbindung der Formel (VII) umsetzt; 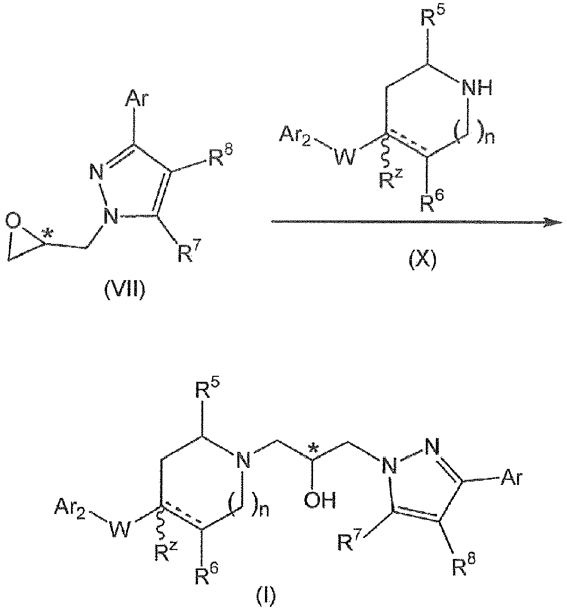 die Verbindung der Formel (VII) in einem organischen Lösungsmittel mit einer Verbindung der Formel (X) zu der entsprechenden Verbindung der Formel (I) umsetzt.

2. Verfahren zur Herstellung einer Verbindung der Formel (I-S) 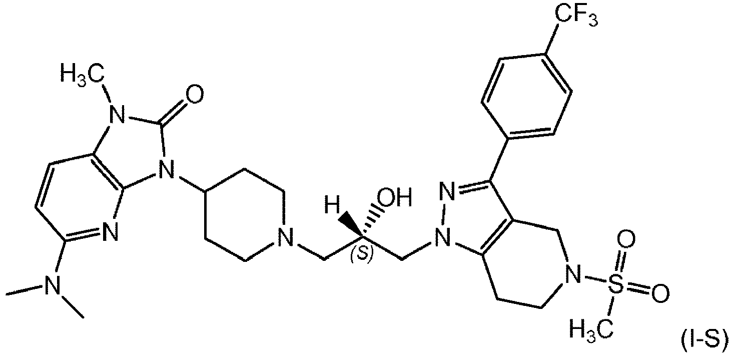 oder eines pharmazeutisch unbedenklichen Salzes, Amids oder Esters davon oder einer stereoisomeren Form davon; bei dem man 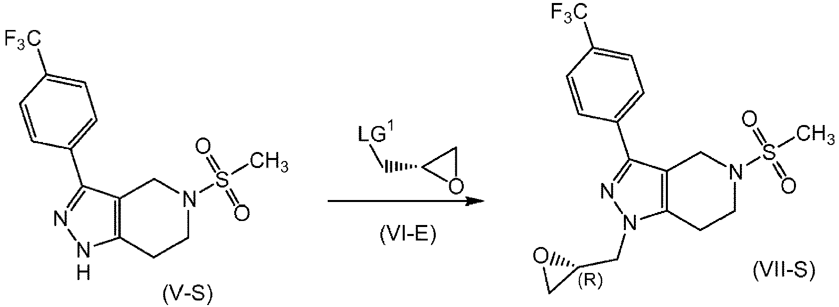 eine Verbindung der Formel (V-S) in Gegenwart einer organischen oder anorganischen Base mit einer Verbindung der Formel (VI-E), worin LG¹ für eine Abgangsgruppe, die aus 4-Nitrophenylsulfonyl, 3-Nitrophenylsulfonyl, Mesyl, Tosyl, Trifluormethansulfonyl, 3-Fluorbenzolsulfonyl, 3-(Tri-fluormethyl)benzolsulfonyl, 4-Chlorbenzolsulfonyl und 3-Chlorbenzolsulfonyl ausgewählt ist, steht, zu der entsprechenden Verbindung der Formel (VII-S) umsetzt; 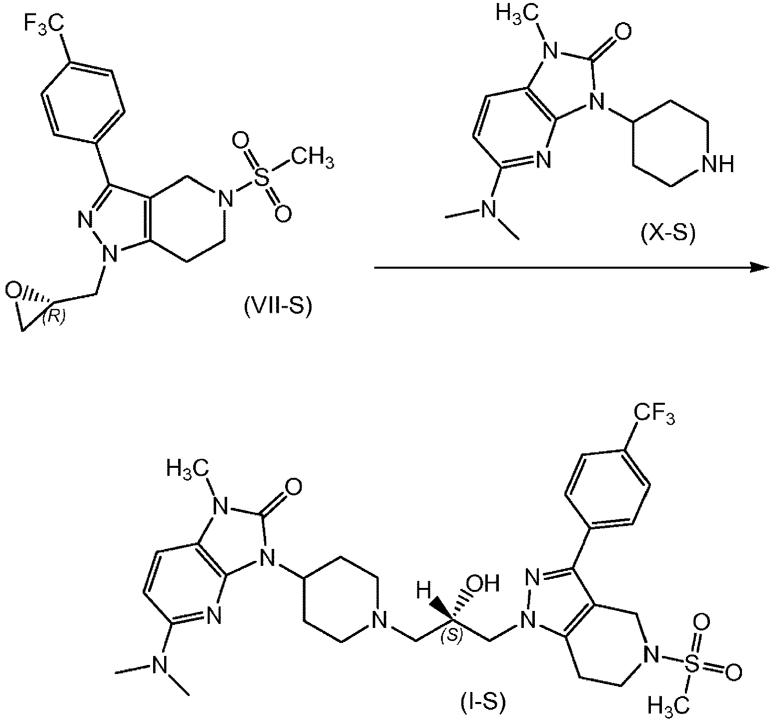 die Verbindung der Formel (VII-S) in einem organischen Lösungsmittel mit einer Verbindung der Formel (X-S) zu der entsprechenden Verbindung der Formel (I-S) umsetzt.

3. Verfahren nach Anspruch 2, bei dem LG¹ für 3-Nitrophenylsulfonyl steht.

4. Verfahren nach Anspruch 2, bei dem die Verbindung der Formel (VI-E) in einer Menge im Bereich von etwa 0,5 bis etwa 3,0 Moläquivalenten vorliegt oder bei dem die Verbindung der Formel (VI-E) in einer Menge im Bereich von etwa 1,0 bis etwa 1,5 Moläquivalenten vorliegt.

5. Verfahren nach Anspruch 2, bei dem man die organische oder anorganische Base aus der Gruppe bestehend aus TEA, DIPEA, Pyridin, NaH, KOt-Bu, NaOt-Bu, Cs₂CO₃, K₂CO₃, Na₂CO₃ und CsF auswählt oder bei dem es sich bei der organischen oder anorganischen Base um Cs₂CO₃ handelt.

6. Verfahren nach Anspruch 2, bei dem es sich dann, wenn man die Verbindung der Formel (V-S) in einem organischen Lösungsmittel mit der Verbindung der Formel (VI-E) umsetzt, bei dem organischen Lösungsmittel und DMF handelt.

7. Verfahren nach Anspruch 2, bei dem man die Verbindung der Formel (V-S) bei einer Temperatur im Bereich von etwa 0°C bis etwa 5°C umsetzt und dann auf eine Temperatur im Bereich von etwa Raumtemperatur bis etwa 60°C erhitzt.

8. Verfahren nach Anspruch 2, bei dem die Verbindung der Formel (X-S) in einer Menge im Bereich von etwa 0,5 bis etwa 3,0 Moläquivalenten vorliegt oder bei dem die Verbindung der Formel (X-S) in einer Menge von etwa 1,1 Moläquivalenten vorliegt.

9. Verfahren nach Anspruch 2, bei dem man dann, wenn man die Verbindung der Formel (VII-S) in einem organischen Lösungsmittel mit der Verbindung der Formel (X-S) umsetzt, das organische Lösungsmittel aus der Gruppe bestehend aus DCE, Ethanol, Isopropylalkohol und DMF auswählt oder bei dem es sich bei dem organischen Lösungsmittel um Ethanol handelt.

10. Verfahren nach Anspruch 2, dem man die Verbindung der Formel (VII-S) bei etwa der Rückflusstemperatur des Lösungsmittels mit der Verbindung der Formel (X-S) umsetzt.

11. Verfahren nach Anspruch 2, bei dem man die Verbindung der Formel (VII-S) in Gegenwart einer Lewis-Säure aus der Gruppe bestehend aus Titanpropoxid, Yb(OTf)₃ und Al(OTf)₃ mit der Verbindung der Formel (X-S) umsetzt.

12. Verfahren nach Anspruch 11, bei dem die Lewis-Säure in einer katalytisch wirksamen Menge vorliegt.

13. Kristallines Hexahydrat-Mono-HCl-Salz einer Verbindung der Formel (I-S) 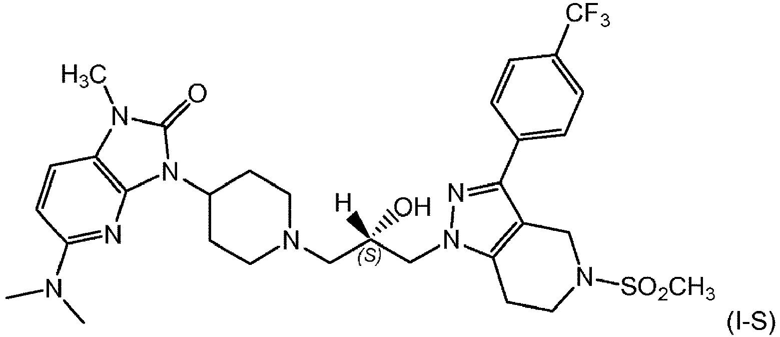 mit den folgenden Röntgenbeugungspeaks:
| **Winkel (°2θ)** | **d-Abstand (Å)** |
|---|---|
| 4,33 | 20,42 |
| 7,58 | 11,67 |
| 8,39 | 10,54 |
| 8,80 | 10,04 |
| 9,16 | 9,66 |
| 12,71 | 6,96 |
| 13,30 | 6,66 |
| 13,69 | 6,47 |
| 14,69 | 6,03 |
| 15,55 | 5,70 |
| 15,96 | 5,55 |
| 18,44 | 4,81 |
| 18,69 | 4,75 |
| 19,07 | 4,65 |

14. Verfahren zur Herstellung eines kristallinen Mono-HCl-Hexahydrat-Salzes nach Anspruch 13, bei dem man eine Verbindung der Formel (I-S) mit HCl-Säure in Wasser umsetzt.

15. Verfahren zur Herstellung einer Verbindung der Formel (X-S) 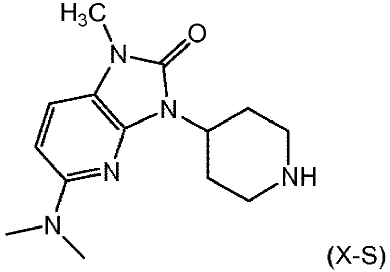 bei dem man 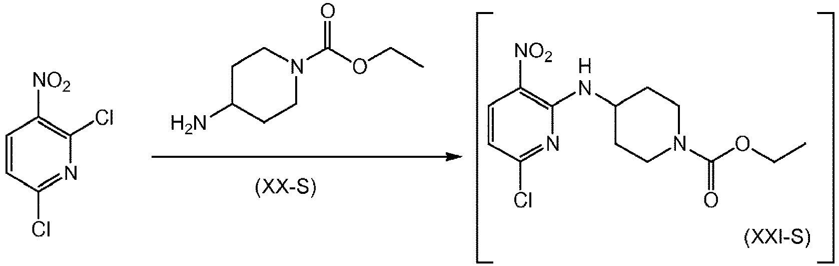 2,6-Dichlor-3-nitropyridin in Gegenwart einer organischen oder anorganischen Base in einem organischen Lösungsmittel mit einer Verbindung der Formel (XX-S), worin PG⁵ für eine Stickstoff-Schutzgruppe steht, zu der entsprechenden Verbindung der Formel (XXI-S) umsetzt; 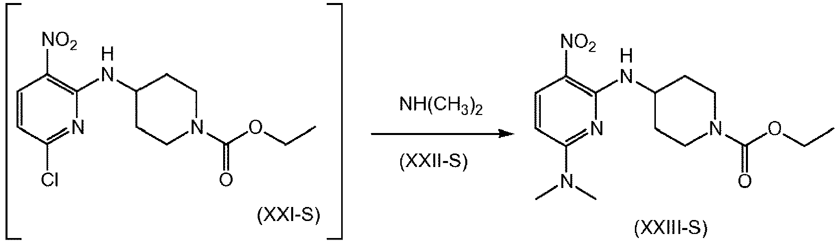 die Verbindung der Formel (XXI-S) in einem organischen Lösungsmittel mit einer Verbindung der Formel (XXII-S) zu der entsprechenden Verbindung der Formel (XXIII-S) umsetzt; 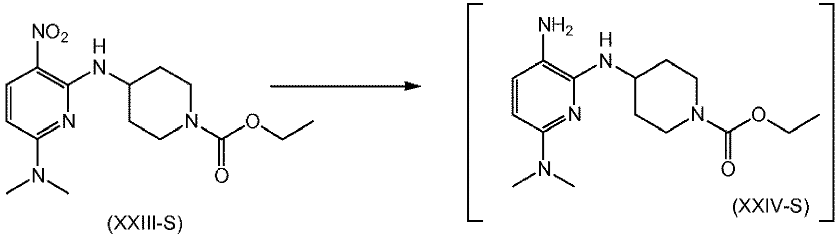 die Verbindung der Formel (XXIII-S) in Gegenwart eines Katalysators in einem organischen Lösungsmittel mit einem Reduktionsmittel zu der entsprechenden Verbindung der Formel (XXIV-S) umsetzt; 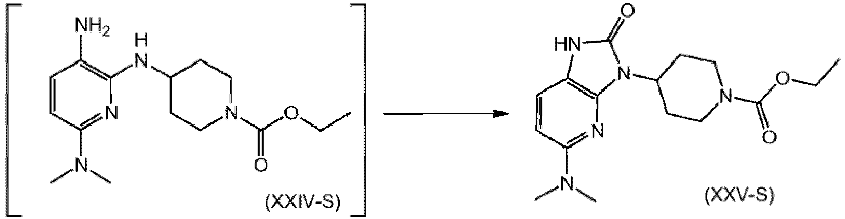 die Verbindung der Formel (XXIV-S) in einem organischen Lösungsmittel mit einem Reagens aus der Gruppe bestehend aus CDI, N-Methylcarbodiimid, Phosgen und Triphosgen zu der entsprechenden Verbindung der Formel (XXV-S) umsetzt; 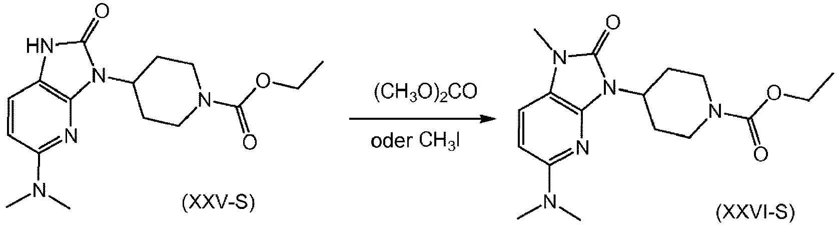 die Verbindung der Formel (XXV-S) in Gegenwart einer anorganischen Base in einem organischen Lösungsmittel mit Kohlensäuredimethylester oder CH₃I zu der entsprechenden Verbindung der Formel (XXVI-S) umsetzt; 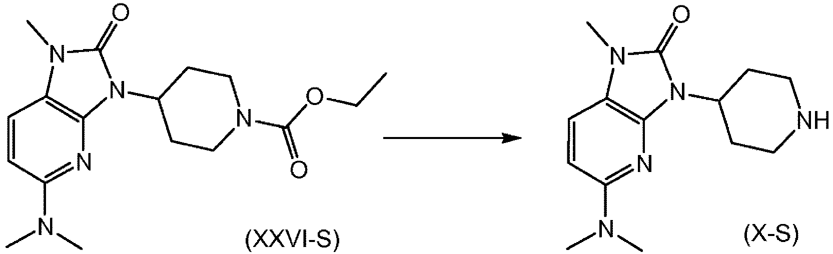 die Verbindung der Formel (XXVI-S) entschützt, wobei man die entsprechende Verbindung der Formel (X-S) erhält.

## Revendications

1. Procédé de synthèse d'un composé de formule (I) 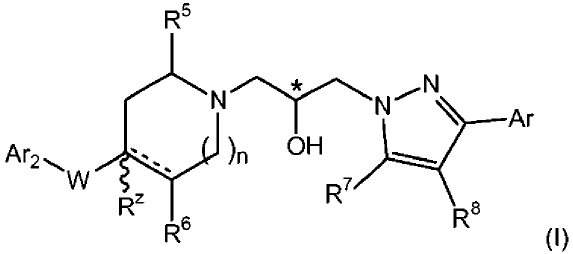 où
Ar₂ représente un système cyclique monocyclique ou bicyclique, insaturé, saturé ou aromatique, éventuellement fusionné, incluant éventuellement entre 1 et 5 entités de cycle hétéroatomiques indépendamment choisies dans le groupe constitué par 0, S, N, SO₂ et C=O; ledit système cyclique Ar₂ étant éventuellement substitué par entre 1 et 4 substituants ;
W représente 0, S, NR²⁷, C=O, (C=O)NH, NH(C=O), CHR²⁸ ou une liaison covalente ;
R²⁷ représente un atome d'hydrogène ou un groupement alkyle en C₁₋₅, alcényle en C₃₋₅, phényle, naphtyle, benzyle, phénéthyle, hétérocyclyle en C₁₋₅, acyle en C₂₈, aroyle, R²⁹OC=O, R³⁰R³¹NC=O, R²⁹SO, R²⁹S, R²⁹SO₂ ou R³⁰R³¹NSO₂ ; ou, de façon alternative, R²⁷ et une partie de Ar₂ peuvent former ensemble un cycle hétérocyclique éventuellement substitué comportant 5 à 6 chaînons ainsi qu'éventuellement 1 à 3 entités hétéroatomiques supplémentaires dans le cycle choisies parmi 0, NR⁹, NR¹⁰, N, SO₂, C=O et S ; ledit cycle pouvant être saturé, insaturé ou aromatique ; R⁹ et R¹⁰ étant indépendamment choisis dans le groupe constitué par H et les groupements alkyle en C₁₋₃ et -CH₂CO₂-(alkyle en C₁₋₄) ;
R²⁸ représente un atome d'hydrogène ou un groupement alkyle en C₁₋₅, alcényle en C₃₋₅, hydroxy, phényle, benzyle, hétérocyclyle en C₁₋₅, R²⁹O, R³⁰R³¹NC=O, R²⁹S, R²⁹SO, R²⁹SO₂ ou R³⁰R³¹ NSO2 ;
R²⁹ représente un groupement alkyle en C₁₋₅, alcényle en C₃₋₅, phényle, benzyle ou hétérocyclyle en C₁₋₅ ;
chacun des radicaux R³⁰ et R³¹ est indépendamment choisi dans le groupe constitué par l'atome d'hydrogène et les groupements alkyle en C₁₋₅, alcényle en C₃₋₅, phényle, benzyle, phénéthyle, naphtyle et hétéroaryle en C₁₋₅ ; de façon alternative, R³⁰ et R³¹ peuvent former ensemble un cycle carbocyclique ou hétérocyclique éventuellement substitué comportant entre 4 et 7 chaînons, ledit cycle pouvant être saturé, insaturé ou aromatique ;
R^{z} représente H ou OH et le segment en pointillé est absent ; ou R^{z} est absent lorsque le segment en pointillé représente une liaison sp² ;
chacun des radicaux R⁵ et R⁶ est indépendamment choisi dans le groupe constitué par l'atome d'hydrogène et les groupements alkyle en C₁₋₅ ;
n représente un entier sélectionné parmi 0, 1 ou 2 ;
chacun des radicaux R⁷ et R⁸ représente indépendamment un atome d'hydrogène ou un groupement alkyle en C₁₋₅, alcényle en C₂₋₅, alkoxy en C₁₋₅, alkylthio en C₁₋₅, halogéno ou un carbocyclyle ou hétérocyclyle comportant entre 4 et 7 chaînons ;
de façon alternative, R⁷ et R⁸ peuvent former ensemble un cycle carbocyclique ou hétérocyclique éventuellement substitué comportant entre 5 et 7 chaînons, ledit cycle pouvant être insaturé ou aromatique, et étant éventuellement substitué par entre un et trois substituants indépendamment choisis dans le groupe constitué par les groupements halogéno, cyano, amino, hydroxy, nitro, R⁴, R⁴O-, R⁴S-, R⁴O (alkylène en C₁₋₅)-, R⁴O (C=O) -, R⁴ (C=O) -, R⁴ (C=S) -, R⁴ (C=O) 0-, R⁴O (C=O) (C=O) -, R⁴SO₂, NHR⁴⁴(C=NH) -, NHR⁴⁴SO₂- et NHR⁴⁴(C=O) - ;
R⁴ représente H ou un groupement alkyle en C₁₋₅, alcényle en C₂₋₅, hétérocyclyle en C₁₋₅, (hétérocyclyle en C₁₋₅)-(alkylène en C₁₋₆), phényle, benzyle, phénéthyle, NH₂, mono- ou di- (alkyle en C₁₋₆)-N-, (alkoxy en C₁₋₆) - carbonyl- ou R⁴²OR⁴³-; où R⁴² représente H ou un groupement alkyle en C₁₋₅, alcényle en C₂₋₅, phényle, benzyle, phénéthyle, hétérocyclyle en C₁₋₅, ou (hétérocyclyle en C₁₋₅) - (alkylène en C₁₋₆) - et R⁴³ représente un groupement alkylène en C₁₋₅, phénylène ou hétérocyclyle divalent en C₁₋₅ ;
R⁴⁴ représente H ou un groupement alkyle en C₁₋₅, alcényle en C₂₋₅, hétérocyclyle en C₁₋₅, (hétérocyclyle en C₁₋₅) - (alkylène en C₁₋₆), phényle, benzyle, phénéthyle, NH₂, mono- ou di- (alkyle en C₁₋₆)-N-, (alkoxy en C₁₋₆)-carbonyl- ou R⁴²OR⁴³- ; où R⁴² représente H ou un groupement alkyle en C₁₋₅, alcényle en C₂₋₅, phényle, benzyle, phénéthyle, hétérocyclyle en C₁₋₅, ou (hétérocyclyle en C₁₋₅) - (alkylène en C₁₋₆)- et R⁴³ représente un groupement alkylène en C₁₋₅, phénylène ou hétérocyclyle divalent en C₁₋₅ ;
Ar représente un cycle aryle ou hétéroaryle monocyclique ou bicyclique, éventuellement substitué par entre 1 et 3 substituants indépendamment choisis dans le groupe constitué par les atomes d'halogène et les groupements alkoxy en C₁₋₅, alkyle en C₁₋₅, alcényle en C₂₋₅, cyano, azido, nitro, R²²R²³N, R²⁴SO_{2,} R²⁴SO, R²⁴OC=O, R²²R²³NC=O, halogénoalkyle en C₁₋₅, halogénoalkoxy en C₁₋₅, halogénoalkylthio en C₁₋₅ et alkylthio en C₁₋₅ ;
R²² représente un atome d'hydrogène ou un groupement alkyle en C₁₋₅, alcényle en C₃₋₅, phényle, phénéthyle, benzyle, hétérocyclyle en C₁₋₅, acyle en C₂₋₈, aroyle, R³⁸OC=O, R²⁵R²⁶NC=O, R³⁸SO, R³⁸SO₂, R³⁸S ou R²⁵R²⁶NSO₂ ;
R²³ représente un atome d'hydrogène ou un groupement alkyle en C₁₋₅, alcényle en C₃₋₅, phényle, benzyle ou hétérocyclyle en C₁₋₅ ; de façon alternative, R²² et R²³ peuvent former ensemble un cycle hétérocyclique éventuellement substitué comportant entre 4 et 7 chaînons, ledit cycle pouvant être saturé, insaturé ou aromatique ;
R²⁴ représente un groupement alkyle en C₁₋₅, alcényle en C₃₋₅, phényle, benzyle ou hétérocyclyle en C₁₋₅ ;
chacun des radicaux R²⁵ et R²⁶ représente indépendamment un atome d'hydrogène ou un groupement alkyle en C₁₋₅, alcényle en C₃₋₅, phényle, benzyle ou hétérocyclyle en C₁₋₅ ; ou, de façon alternative, R²⁵ et R²⁶ peuvent former ensemble un cycle hétérocyclique éventuellement substitué comportant entre 4 et 7 chaînons, ledit cycle pouvant être saturé, insaturé ou aromatique ;
R³⁸ représente H ou un groupement alkyle en C₁₋₅, alcényle en C₃₋₅, phényle, benzyle, phénéthyle ou hétérocyclyle en C₁₋₅ ;
chacun des groupements hydrocarbyle ou hétérocarbyle ci-avant, à moins que le contraire ne soit précisé, et en plus de tout substituant indiqué, étant éventuellement et indépendamment substitué par entre 1 et 3 substituants choisis dans le groupe constitué par les groupements méthyle, halogénométhyle, hydroxyméthyle, halogéno, hydroxy, amino, nitro, cyano, alkyle en C₁₋₅, alkoxy en C₁₋₅, -COOH, acyle en C₂₋₆, [di-(alkyle en C₁₋₄)-amino]-(alkylène en C₂₋₅), [di-(alkyle en C₁₋₄)-amino]-(alkyle en C₂₋₅)-NH-CO- et halogénoalkoxy en C₁₋₅ ;
ou l'un de ses sels, amides ou esters de qualité pharmaceutique ; ou l'une de ses formes stéréoisomères ; comprenant 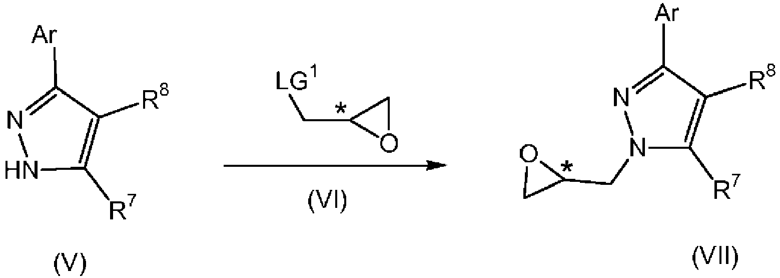 la réaction d'un composé de formule (V) avec un composé de formule (VI), où LG¹ représente un groupement partant choisi parmi les groupements 4-nitrophényl-sulfonyle, 3-nitrophényl-sulfonyle, mésyle, tosyle, trifluorométhanesulfonyle, 3-fluoro-benzensulfonyle, 3-(trifluorométhyl)-benzènesulfonyle, 4-chloro-benzènesulfonyle et 3-chloro-benzènesulfonyle ; en présence d'une base organique ou inorganique ; pour obtenir le composé correspondant de formule (VII) ; 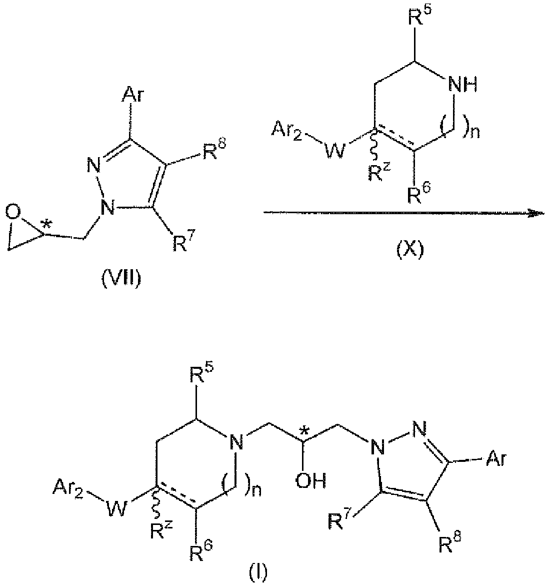 la réaction du composé de formule (VII) avec un composé de formule (X) ; dans un solvant organique ; pour obtenir le composé correspondant de formule (I).

2. Procédé de synthèse d'un composé de formule (I-S) 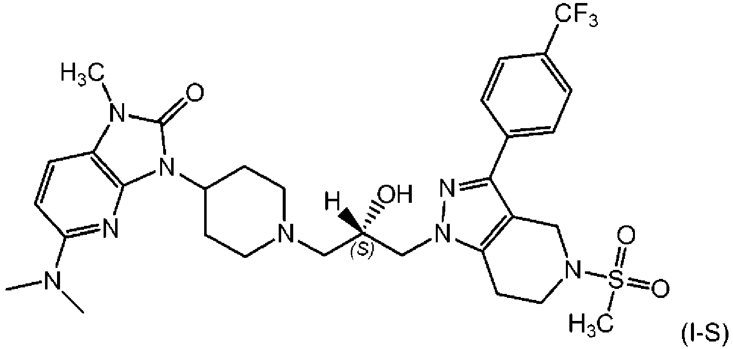 ou de l'un de ses sels, amides ou esters de qualité pharmaceutique ; ou de l'une de ses formes stéréoisomères ; comprenant 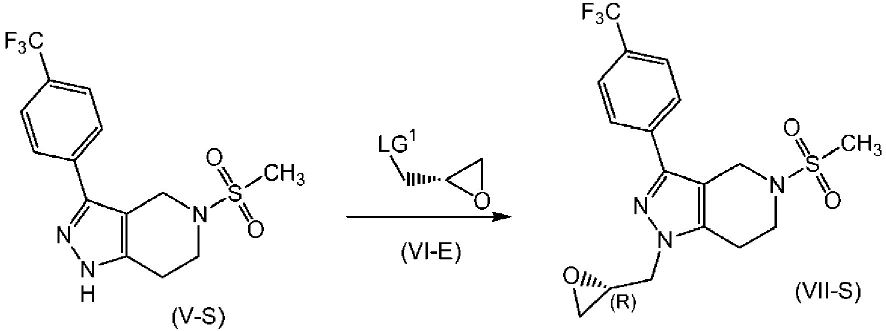 la réaction d'un composé de formule (V-S) avec un composé de formule (VI-E), où LG¹ représente un groupement partant choisi parmi les groupements 4-nitrophényl-sulfonyle, 3-nitrophényl-sulfonyle, mésyle, tosyle, trifluorométhanesulfonyle, 3-fluoro-benzensulfonyle, 3-(trifluorométhyl)-benzènesulfonyle, 4-chloro-benzènesulfonyle et 3-chloro-benzènesulfonyle ; en présence d'une base organique ou inorganique ; pour obtenir le composé correspondant de formule (VII-S) ; 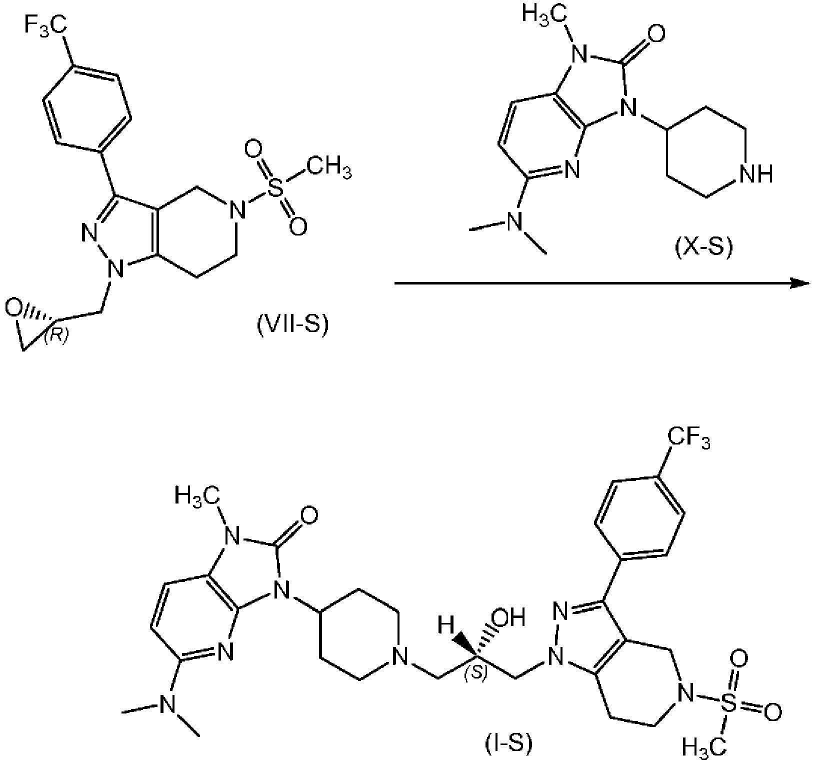 la réaction du composé de formule (VII-S) avec un composé de formule (X-S) ; dans un solvant organique ; pour obtenir le composé correspondant de formule (I-S).

3. Procédé conforme à la revendication 2, où LG¹ représente un groupement 3-nitrophényl-sulfonyle.

4. Procédé conforme à la revendication 2, où le composé de formule (VI-E) est présent à une teneur incluse dans l'intervalle compris entre environ 0,5 et environ 3,0 équivalents molaires, ou où le composé de formule (VI-E) est présent à une teneur comprise entre 1,0 et environ 1,5 équivalent molaire.

5. Procédé conforme à la revendication 2, où la base organique ou inorganique est choisie dans le groupe constitué par les suivantes : TEA, DIPEA, pyridine, NaH, KOt-Bu, NaOt-Bu, Cs₂CO₃, K₂CO₃, Na₂CO₃ et CsF, ou où la base organique ou inorganique est Cs₂CO₃.

6. Procédé conforme à la revendication 2, où lorsque le composé de formule (V-S) réagit avec le composé de formule (VI-E) dans un solvant organique, le solvant organique est le DMF.

7. Procédé conforme à la revendication 2, où le composé de formule (V-S) réagit avec le composé de formule (VI-E) à une température comprise entre environ 0 °C et environ 5 °C, avant d'être chauffé à une température incluse dans l'intervalle compris entre environ la température ambiante et environ 60 °C.

8. Procédé conforme à la revendication 2, où le composé de formule (X-S) est présent à une teneur incluse dans l'intervalle compris entre environ 0,5 et environ 3,0 équivalents molaires, ou où le composé de formule (X-S) est présent à une teneur d'environ 1,1 équivalent molaire.

9. Procédé conforme à la revendication 2, où le composé de formule (VII-S) réagit avec le composé de formule (X-S) dans un solvant organique, le solvant organique étant choisi dans le groupe constitué par le DCE, l'éthanol, l'isopropanol et le DMF, ou où le solvant organique est l'éthanol.

10. Procédé conforme à la revendication 2, où le composé de formule (VII-S) réagit avec le composé de formule (X-S) à une température environ égale à la température de reflux du solvant.

11. Procédé conforme à la revendication 2, où le composé de formule (VII-S) réagit avec le composé de formule (X-S) en présence d'un acide de Lewis choisi dans le groupe constitué par le propoxyde de titane, Yb(OTf)₃ et Al(OTf)₃.

12. Procédé conforme à la revendication 11, où l'acide de Lewis est présent dans une quantité catalytique.

13. Sel cristallin mono-HCl hexahydraté d'un composé de formule (I-S) 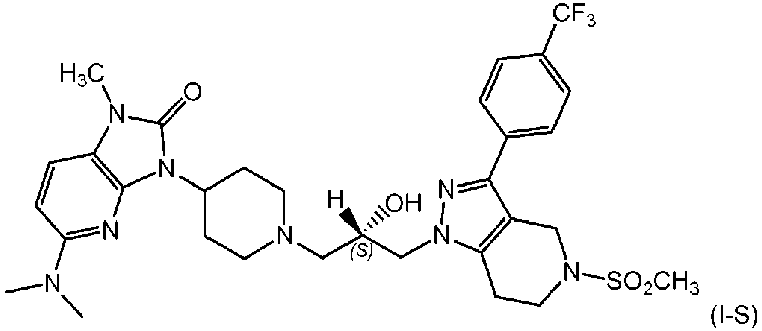 comprenant les pics de diffraction X suivants :
| **Angle (°2θ)** | **distance d (Å)** |
|---|---|
| 4,33 | 20,42 |
| 7,58 | 11,67 |
| 8,39 | 10,54 |
| 8,80 | 10,04 |
| 9,16 | 9,66 |
| 12,71 | 6,96 |
| 13,30 | 6,66 |
| 13,69 | 6,47 |
| 14,69 | 6,03 |
| 15,55 | 5,70 |
| 15,96 | 5,55 |
| 18,44 | 4,81 |
| 18,69 | 4,75 |
| 19,07 | 4,65 |

14. Procédé de synthèse d'un sel cristallin mono-HCl hexahydraté conforme à la revendication 13 comprenant la réaction d'un composé de formule (I-S) avec l'acide HCl dans l'eau.

15. Procédé de synthèse d'un composé de formule (X-S) 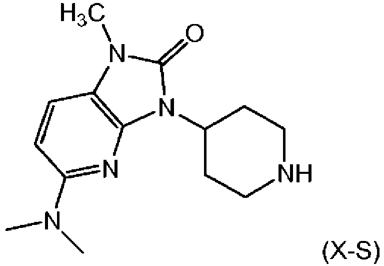 comprenant 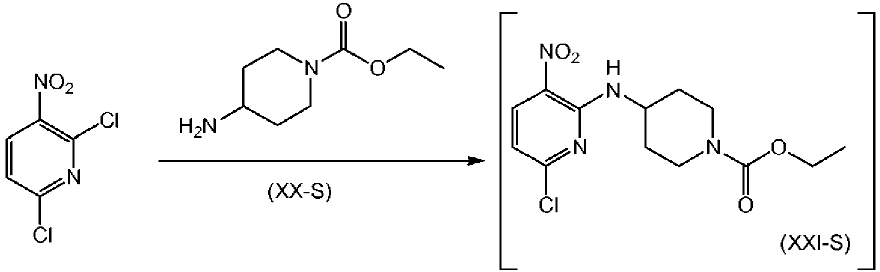 la réaction de la 2,6-dichloro-3-nitro-pyridine avec un composé de formule (XX-S), où PG⁵ représente un groupement protecteur d'azote ; en présence d'une base organique ou inorganique ; dans un solvant organique ; pour obtenir le composé correspondant de formule (XXI-S); 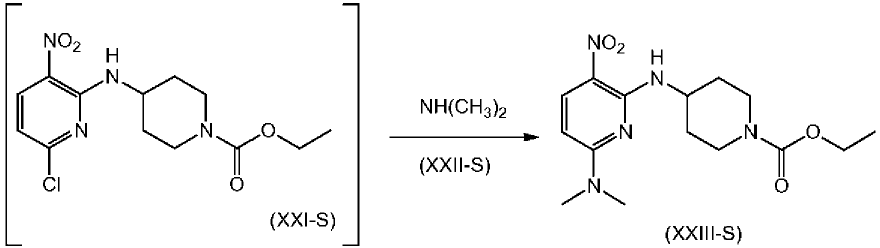 la réaction du composé de formule (XXI-S) avec un composé de formule (XXII-S) ; dans un solvant organique ; pour obtenir le composé correspondant de formule (XXIII-S) ; 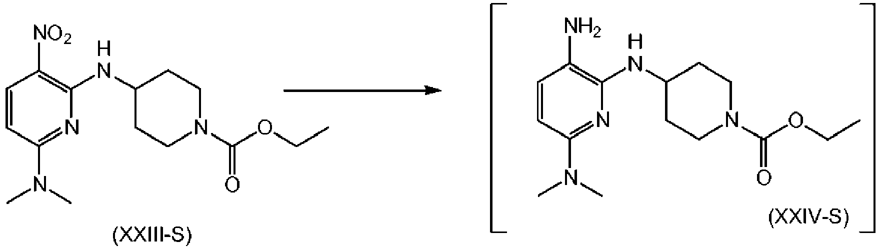 la réaction du composé de formule (XXIII-S) avec un agent réducteur ; en présence d'un catalyseur ; dans un solvant organique ; pour obtenir le composé correspondant de formule (XXIV-S) ; 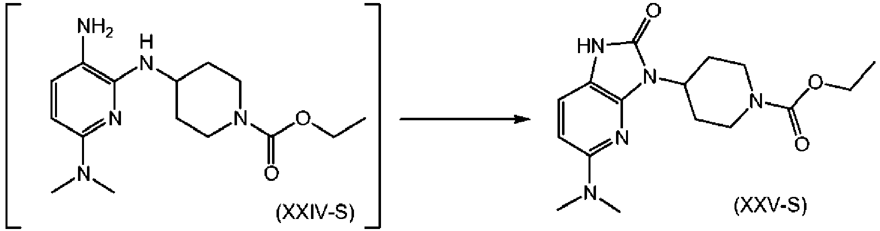 la réaction du composé de formule (XXIV-S) avec un réactif choisi dans le groupe constitué par CDI, le N-méthylcarbodiimide, le phosgène et le triphosgène ; dans un solvant organique ; pour obtenir le composé correspondant de formule (XXV-S) ; 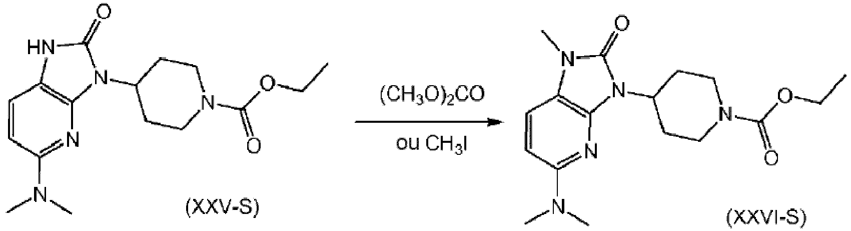 la réaction du composé de formule (XXV-S) avec un ester diméthylique d'acide carbonique ou CH₃I ; en présence d'une base inorganique ; dans un solvant organique ; pour obtenir le composé correspondant de formule (XXVI-S) ; 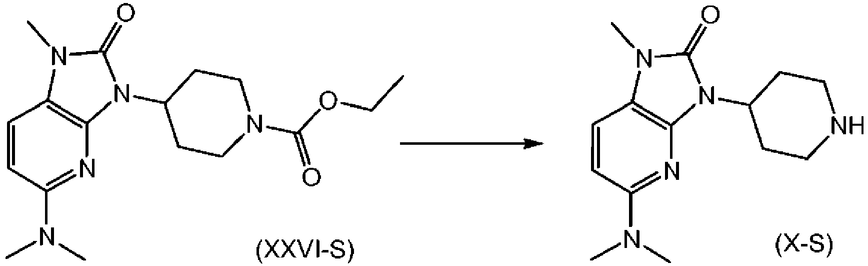 la déprotection du composé de formule (XXVI-S) ; pour obtenir le composé correspondant de formule (X-S).
